# EUROPÄISCHE PATENTSCHRIFT

(11) **EP 1 017 684 B1**
(45) Veröffentlichungstag und Bekanntmachung des Hinweises auf die Patenterteilung: **20.11.2002**
(21) Anmeldenummer: 98946346.8
(22) Anmeldetag: 17.08.1998
(51) Int. Cl.: C07D 257/02, C07B 59/00, A61K 49/00, A61K 51/04

(54) **KONTRASTMITTEL FÜR DAS INFARKT- UND NEKROSEIMAGING**
CONTRASTING AGENT FOR INFARCT AND NECROSIS IMAGING
AGENTS DE CONTRASTE POUR IMAGERIE DE NECROSES ET D'INFARCTUS

(30) Priorität: 26.09.1997 DE 19744003
(43) Veröffentlichungstag der Anmeldung: 12.07.2000
(73) Patentinhaber: SCHERING AKTIENGESELLSCHAFT, 13342 Berlin (DE)
(72) Erfinder: PLATZEK, Johannes, D-12621 Berlin (DE); NIEDBALLA, Ulrich, D-14195 Berlin (DE); RADÜCHEL, Bernd, D-13465 Berlin (DE); EBERT, Wolfgang, D-15831 Mahlow (DE); WEINMANN, Hanns-Joachim, D-14129 Berlin (DE)
(86) Internationale Anmeldenummer: EP9805184
(87) Internationale Veröffentlichungsnummer: WO99016757

(56) Entgegenhaltungen:
- EP-A- 0 255 471
- EP-A- 0 430 863
- EP-A- 0 485 045
- WO-A-95/07270
- WO-A-95/09848
- DE-A- 4 425 857
- DE-A- 19 549 286

## Beschreibung

Die Erfindung betrifft den in den Patentansprüchen gekennzeichneten Gegenstand, nämlich neue Verbindungen, die als Kontrastmittel insbesondere für das Infarkt- und Nekroseimaging geeignet sind, Verfahren zu deren Herstellung und pharmazeutische Mittel enthaltend diese Verbindungen.

Detektion, Lokalisierung und Überwachung von Nekrosen oder Infarkten ist ein wichtiger Bereich in der Medizin. So ist der Myokardinfarkt nicht ein stationärer Vorgang, sondern ein dynamischer Prozeß, der sich über einen längeren Zeitraum (Wochen bis Monate) erstreckt. Die Erkrankung verläuft in etwa drei Phasen, die nicht scharf voneinander getrennt, sondern überlappend sind. Die erste Phase, die Entwicklung des Myokardinfarktes, umfaßt die 24 Stunden nach dem Infarkt, in denen die Zerstörung wie eine Stoßwelle (Wellenfrontphänomen) vom Subendokard zum Myokard fortschreitet. Die zweite Phase, der bereits bestehende Infarkt, umfaßt die Stabilisierung des Bereiches, in dem Faserbildung (Fibrose) als Heilprozeß erfolgt. Die dritte Phase, der ausgeheilte Infarkt, beginnt, nachdem alles zerstörte Gewebe durch fibröses Narbengewebe ersetzt ist. Während dieser Periode findet eine umfangreiche Restrukturierung statt.
Bis heute ist kein präzises und zuverlässiges Verfahren bekannt, das die aktuelle Phase eines Myokardinfarktes am lebenden Patienten diaanostizierbar macht. Für die Beurteilung eines Myokardinfarktes ist es von entscheidender Bedeutung, zu wissen, wie groß der Anteil des bei dem Infarkt verlorenen Gewebes ist und an welcher Stelle der Verlust erfolgte, denn von dieser Kenntnis hängt die Art der Therapie ab.
Infarkte erfolgen nicht nur im Myokard, sondern auch in anderen Geweben, besonders im Hirn.
Während der Infarkt in gewissem Umfang heilbar ist, können bei einer Nekrose, dem lokal begrenzten Gewebetod, nur die schädlichen Folgen für den Restorganismus verhindert oder wenigstens gemildert werden. Nekrosen können auf vielfache Weise entstehen: durch Verletzungen, Chemikalien, Sauerstoffdefizit oder duch Strahlung. Wie beim Infarkt ist die Kenntnis von Umfang und Art einer Nekrose wichtig für das weitere ärztliche Vorgehen.
Schon früh erfolgten daher Versuche, die Lokalisation von Infarkten und Nekrosen durch Einsatz von Kontrastmitteln bei nichtinvasiven Verfahren wie Szintigraphie oder Kernspintomographie zu verbessern. In der Literatur nehmen die Versuche, Porphyrine für das Nekroseimaging einzusetzen, einen großen Raum ein. Die erzielten Resultate ergeben jedoch ein widersprüchliches Bild. So beschreiben Winkelman und Hoyes in Nature, 200, 903 (1967), daß sich Mangan-5,10,15,20-Tetrakis(4-sulfonatophenyl)-porphyrin (TPPS) selektiv im nekrotischen Teil eines Tumors anreichert.
Lyon et al. (Magn. Res. Med. 4, 24 (1987)) dagegen beobachteten, daß sich Mangan-TPPS im Körper verteilt, und zwar in Niere, Leber, Tumor und nur zu einem geringen Teil in den Muskeln. Interessant ist dabei, daß die Konzentration im Tumor erst am 4. Tag ihr Maximum erreicht und das auch nur, nachdem die Autoren die Dosis von 0.12 mmol/kg auf 0.2 mmol/kg gesteigert hatten. Die Autoren sprechen daher auch von einer nichtspezifischen Aufnahme des TPPS in den Tumor. Bockhurst et al. wiederum berichten in Acta Neurochir 60, 347 (1994, Suppl.), daß MnTPPS selektiv an Tumorzellen bindet.
Foster et al. (J. Nucl. Med. 26, 756 (1985)) ihrerseits fanden, daß sich ¹¹¹ In-5,10,15,20-Tetrakis-(4-N-methyl-pyridinium)-Porphyrin (TMPyP) nicht im nekrotischen Teil, sondern in den lebenden Randschichten anreichert. Daraus zu folgern, daß eine Porphyrin-Gewebe-Wechselwirkung besteht, ist naheliegend, aber nicht zwingend.
In Circulation Vol. 90, No. 4, Teil 2, Seite 1468, Abstract No. 2512 (1994) berichten Ni et al., daß sie mit einem Mangan-Tetraphenyl-Porphyrin (Mn-TPP) und einem Gadolinium-Mesoporphyrin (Gd-MP) Infarktbereiche darstellen können. In der internationalen Patentanmeldung WO 95/31219 wurden beide Substanzen zum Infarktund Nekroseimaging eingesetzt. Die Autoren Marchal und Ni schreiben (siehe Beispiel 3), daß für die Verbindung Gd-MP der Metallgehalt der Infarkt-Niere ähnlich hoch war wie der des nichtinfarzierten Organs, daß er jedoch für das Myokard beim infarzierten Gewebe (Beispiel I) neunmal so groß war. Erstaunlich war, daß das Verhältnis der Signalintensitäten beim MRI für infarziertes im Vergleich zum gesunden Gewebe in beiden Fällen mit 2.10 bzw. 2.19 vergleichbar hoch war.
Porphyrine neigen dazu, sich in der Haut abzulagern, was zu einer Photosensibilisierung führt. Die Sensibilisierung kann Tage, ja sogar Wochen andauern. Dies ist ein unerwünschter Nebeneffekt bei der Verwendung von Porphyrinen als Diagnostika. Außerdem ist der therapeutische Index für die Porphyrine nur sehr klein, da z.B. für MnTPPS eine Wirkung erst bei einer Dosis von 0.2 mmol/kg einsetzt, die LD₅₀ aber bereits bei 0.5 mmol/kg liegt.

Bisher gibt es also keine Verbindungen, die befriedigend als Kontrastmittel beim Infarktund Nekroseimaging eingesetzt werden können. Aufgabe der vorliegenden Erfindung war es daher, neue Verbindungen zu synthetisieren, die die Anforderungen an ein Diagnostikum für diesen speziellen Anwendungszweck erfüllen und die Nachteile der bisher bekannten Verbindungen überwinden.
Diese Aufgabe wird mit den Verbindungen der allgemeinen Formel I gelöst: 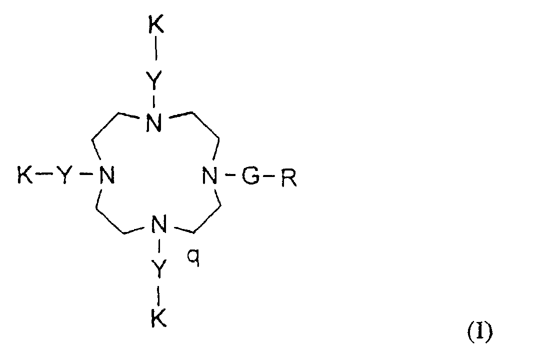 worin
q eine Zahl 1, 2 oder 3 bedeutet,
G eine direkte Bindung, eine SO₂- oder eine CO-Gruppe ist,
K für einen Metallkomplex oder dessen Salze organischer und/oder anorganischer Basen oder Aminosäuren oder Aminosäureamide steht,
R eine unverzweigte oder verzweigte C₄-C₃₀-Kohlenwasserstoffkette ist, die gegebenenfalls substituiert ist durch 1 - 2 Aminogruppen, 1 - 5 OH-Gruppen, 1 - 5 OR¹-Gruppen mit R¹ in der Bedeutung einer C₁-C₆-Alkylgruppe, 1 NH-K-Gruppe, 1 - 3 Carboxygruppen, 1 - 2 aliphatische oder 1 - 3 aromatische Ringe, und die gegebenenfalls 1 - 6 Amidgruppen, 1 - 2 SO₂-Gruppen, 1 - 2 Schwefelatome, 1 - 6 Sauerstoffatome, 1 - 2 aliphatische oder 1 - 3 aromatische Ringe enthält, wobei die aromatischen Ringe gegebenenfalls substituiert sind mit 1 - 2 Chloratomen, 1 - 2 Säuregruppen, 1 - 2 OR¹-Gruppen oder 1 - 2 C₁-C₆-Alkylgruppen,
Y eine direkte Bindung oder eine Kette der allgemeinen Formel II oder III ist: 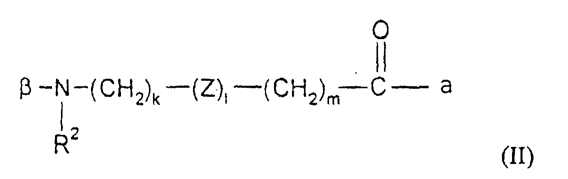 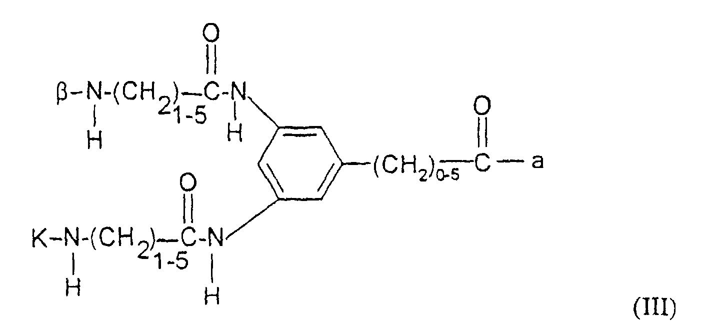 worin
R² ein Wasserstoffatom, eine Phenylgruppe, eine Benzylgruppe oder eine C₁-C₇-Alkylgruppe ist, die gegebenenfalls substituiert ist mit einer Carboxy-, einer Methoxy- oder einer Hydroxygruppe,
Z eine Polyglycolethergruppe mit bis zu 5 Glycoleinheiten oder ein Molekülteil der allgemeinen Formel IV ist

―CH(R³)- (IV),

worin R³ ein C₁-C₁₀-Alkylrest, eine Carbonsäure mit 1 - 7 Kohlenstoffatomen, eine Phenylgruppe, eine Benzylgruppe oder eine -(CH₂)₁₋₅-NH-K-Gruppe ist, α die Bindung an das Stickstoffatom der Gerüstkette, β die Bindung zum Metallkomplex K darstellt,
und in der die Variablen k und m für natürliche Zahlen zwischen 0 und 10 und 1 für 0 oder 1 stehen.

Die neuen Verbindungen haben einige wesentliche Vorteile gegenüber den bekannten Verbindungen. So weisen sie eine hohe Relaxivity im NMR-Experiment auf, was zur Folge hat, daß die Verbindungen für kernspintomographische Untersuchungen niedrig dosiert werden können. Sie sind besonders geeignet für das Nekrose-Imaging, was durch die nachfolgenden pharmakologischen Beispiele gezeigt wird. Die erfindungsgemäßen Verbindungen sind sehr gut vertraglich und werden vollständig ausgeschieden. Im Gegensatz zu den Porphyrinen lagern sie sich nicht in der Haut ab und erzeugen keine Photosensibilisierung. Für die Anwendung der erfindungsgemäßen Verbindungen in der Röntgendiagnostik ist es notwendig, höhere Dosen zu applizieren. Aufgrund der guten Verträglichkeit der Verbindungen stellt dies aber kein Hindernis für die Verwendung in der Röntgendiagnostik dar.

Von den neuen erfindungsgemäßen Verbindungen der allgemeinen Formel I sind diejenigen mit q in der Bedeutung der Ziffer 1 bevorzugt.

In der allgemeinen Formel II ist R² in der Bedeutung eines Wasserstoffatoms bevorzugt.

In der allgemeinen Formel IV ist R³ in der Bedeutung einer Carbonsäure mit 1 bis 4 Kohlenstoffatomen oder einer -(CH₂)₁₋₅-NH-K-Gruppe bevorzugt.

Als Molekülteil Y seien die folgenden Strukturen beispielhaft genannt:

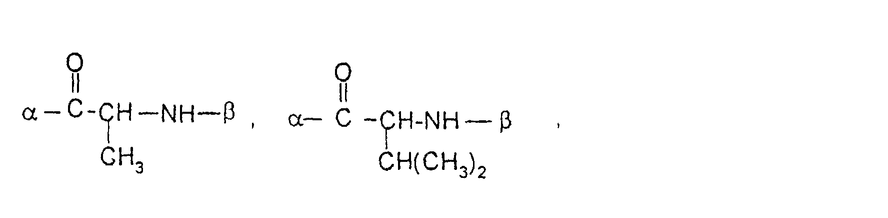

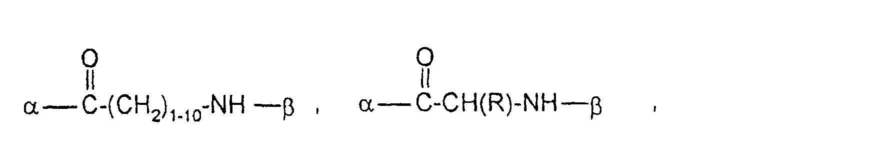

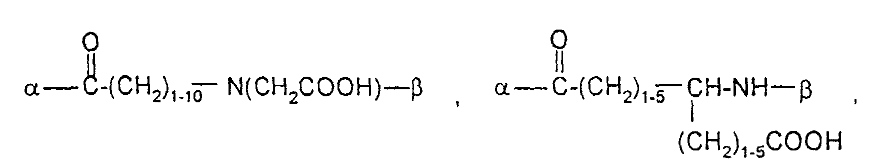

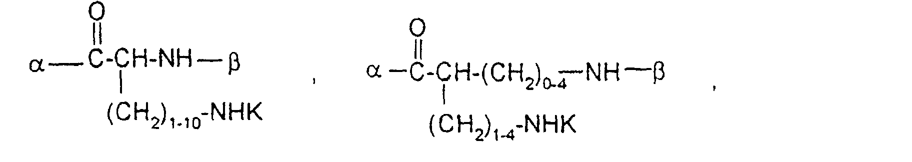

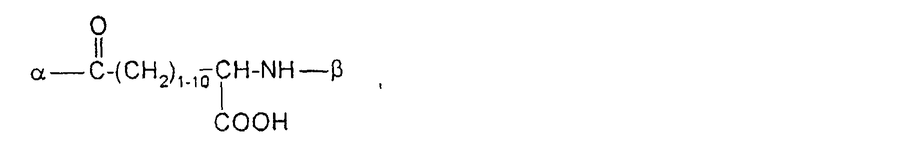

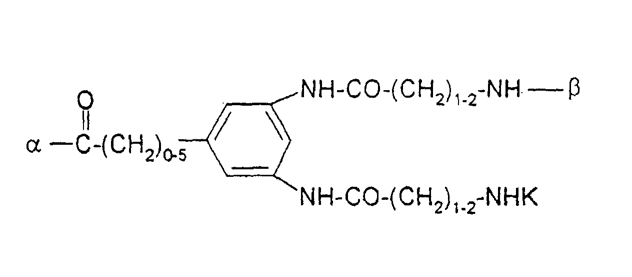

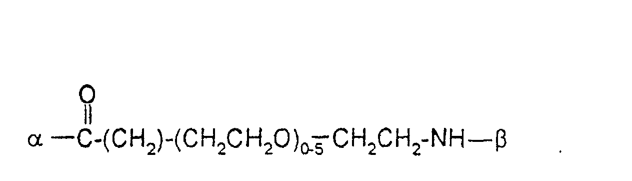

Bevorzugte Molekülteile Y sind die direkte Bindung sowie die folgenden Strukturen:

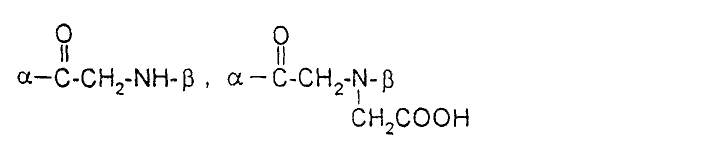

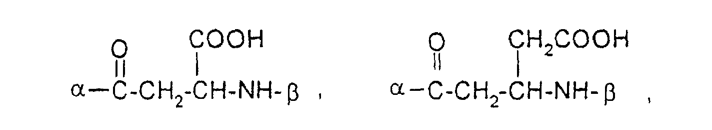

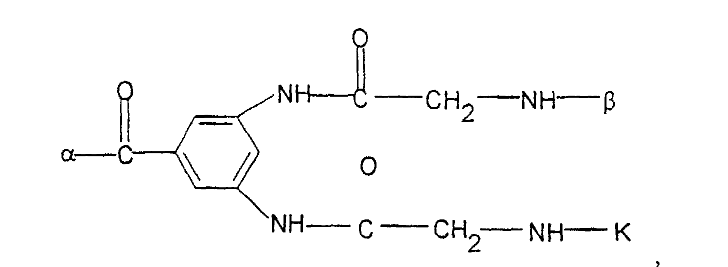

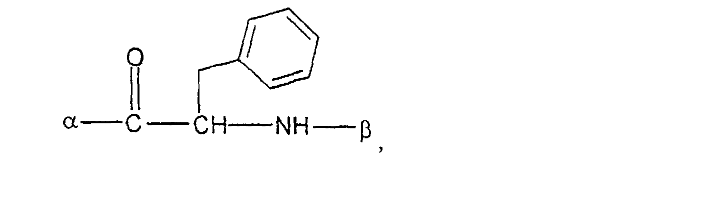

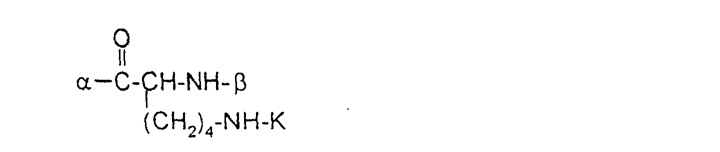

Die Kompiexbiidner bzw. Metallkomplexe haben die folgenden Strukturen: 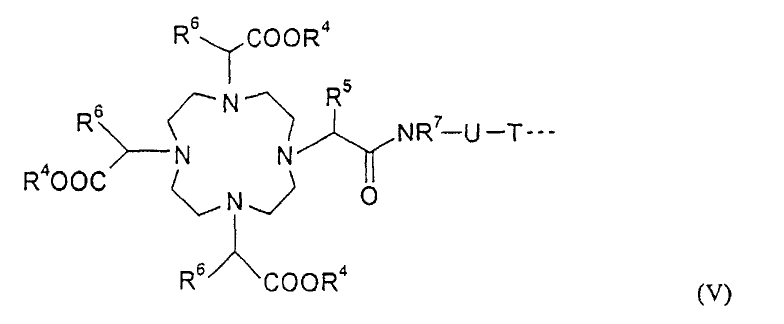 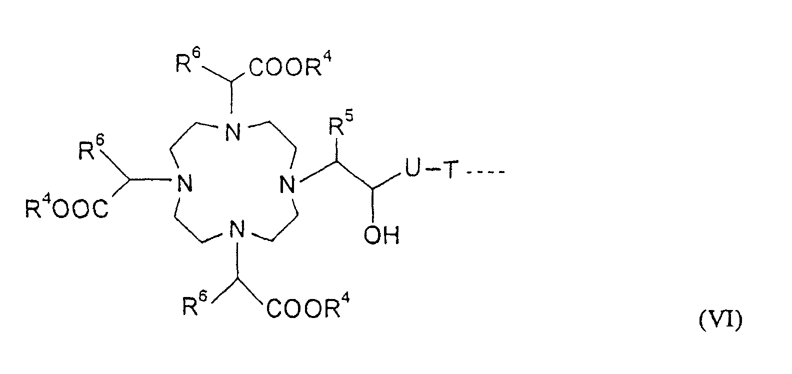 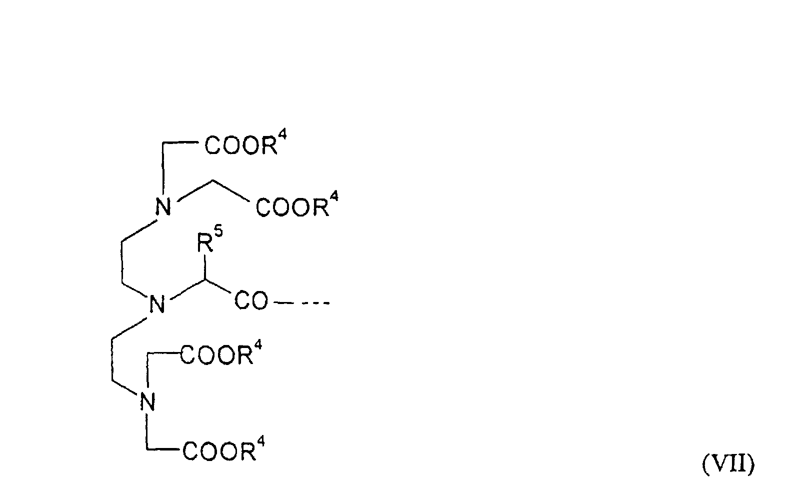 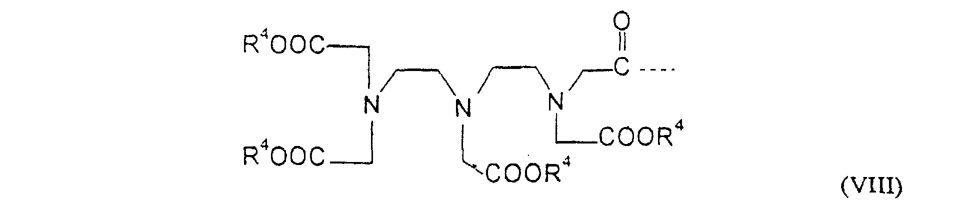 wobei
- R⁴ unabhängig voneinander ein Wasserstoffatom oder ein Metallionenäquivalent der
   Elemente der Ordnungszahlen 20 - 32, 37 - 39, 42 - 44, 49 oder 57 - 83 ist, mit der Maßgabe, daß mindestens zwei der Substituenten R⁴ für ein Metallionenäquivalent der genannten Elemente stehen,
- R⁵ ein Wasserstoffatom oder eine geradkettige, verzweigte, gesättigte oder ungesättigte C₁-C₃₀-Alkylkette ist, die gegebenenfalls substituiert ist durch 1 -5 Hydroxy-, 1 - 3 Carboxy- oder 1 Phenylgruppe(n) und/oder gegebenenfalls durch 1 - 10 Sauerstoffatome, 1 Phenylen- oder 1 Phenylenoxygruppe unterbrochen ist,
- R⁶ ein Wasserstoffatom, ein geradkettiger oder verzweigter C₁-C₇-Alkylrest, ein Phenyl- oder Benzylrest ist,
- R⁷ ein Wasserstoffatom, eine Methyl- oder Ethylgruppe ist, die gegebenenfalls substituiert ist durch eine Hydroxy- oder Carboxygruppe,
- U eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1 Thioharnstoff-, 1 - 2 Carboxyalkylimino-, 1 - 2 Estergruppen, 1 - 10 Sauerstoff-, 1 - 5 Schwefel- und/oder 1 - 5 Stickstoffatome enthaltende und/oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppen substituierte, geradkettige, verzweigte, gesättigte oder ungesättigte C₁-C₂₀-Kohlenwasserstoffkette ist, wobei die gegebenenfalls enthaltenen Phenylengruppen durch 1 - 2 Carboxy-, 1 - 2 Sulfon- oder 1 - 2 Hydroxygruppen substituiert sein können,
- T für eine -CO-β, -NHCO-β oder -NHCS-β-Gruppe steht, wobei β die Bindungsstelle an Y angibt.

Der Substituent R⁵ ist bevorzugt ein Wasserstoffatom, eine C₁ - C₇-Kette oder eine Phenyl- oder Benzylgruppe, die gegebenenfalls substituiert ist durch eine Hydroxymethyloder 1 - 2 OH-Gruppen.

Als besonders bevorzugte Substituenten R⁵ seien genannt:
das Wasserstoffatom, die Methyl-, Ethyl-, Propyl-, Isopropyl-, Benzyl-, Phenylgruppe, -CH₂CH₂OH,
-CH₂OH, -CH₂-COOH, -COOH, -CH₂CHOHCH₂OH, -CH₂O-CH₂CH₂OCH₃, -CH₂OCH₃, -CH₂-O-C₆H₄-COOH.

Als bevorzugte Substituenten R⁶ seien genannt:
das Wasserstoffatom, die Methyl-, Ethyl-, Propyl-, Isopropyl-, Benzyl- und Phenylgruppe, wobei das Wasserstoffatom besonders bevorzugt ist.

Als bevorzugte Substituenten R⁷ seien genannt:
das Wasserstoffatom, die Methyl-, Ethyl-, -CH₂CH₂OH-, -CH₂-COOH-gruppe, wobei das Wasserstoffatom besonders bevorzugt ist.

Die für U stehende C ₁-C₂₀-Kohlenwasserstoffkette, die bevorzugt eine C₁-C₁₀-Kohlenwasserstoffkette ist, enthält vorzugsweise die folgenden Gruppen:
-CH₂NHCO-, -NHCOCH₂O-, -NHCOCH₂OC₆H₄-, -N(CH₂CO₂H)-, -CH₂OCH₂-,
-NHCOCH₂C₆H₄-, -NHCSNHC₆H₄-, -CH₂OC₆H₄-, -CH₂CH₂Ound/oder ist durch die Gruppen -COOH, -CH₂COOH substituiert.

Als Beispiele für U seien folgende Gruppen angeführt:
-CH₂-, -CH₂CH₂-, -CH₂CH₂CH₂-, -C₆H₄-, -C₆H₁₀-, -CH₂C₆H₄-,
-CH₂NHCOCH₂CH(CH₂CO₂H)-C₆H₄-,
-CH₂NHCOCH₂OCH₂-,
-CH₂NHCOCH₂C₆H₄-, 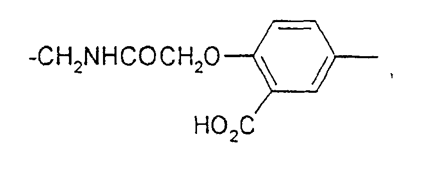
-CH₂NHCSNH-C₆H₄-CH(CH₂COOH)CH₂-,
-CH₂OC₆H₄-N(CH₂COOH)CH₂-,
-CH₂NHCOCH₂O(CH₂CH₂O)₄-C₆H₄-,
-CH₂O-C₆H₄-,
-CH₂CH₂-O-CH₂CH₂-, -CH₂CH₂-O-CH₂CH₂-O-CH₂CH₂-,

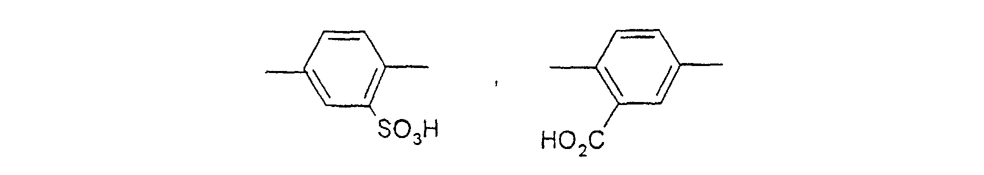

Besonders bevorzugt sind dabei die folgenden Gruppen:
-CH₂-, -CH₂CH₂-, -CH₂CH₂CH₂-, -C₆H₄-, -C₆H₁₀-, -CH₂C₆H₄-,
-CH₂NHCOCH₂CH(CH₂CO₂H)-C₆H₄-,
-CH₂NHCOCH₂OCH₂-,
-CH₂NHCOCH₂C₆H₄-,
-CH₂NHCSNH-C₆H₄-CH(CH₂COOH)CH₂-,
-CH₂OC₆H₄-N(CH₂COOH)CH₂-,
-CH₂NHCOCH₂O(CH₂CH₂O)₄-C₆H₄-,
-CH₂O-C₆H₄-,
-CH₂CH₂-O-CH₂CH₂-, -CH₂CH₂-O-CH₂CH₂-O-CH₂CH₂- .

Als Beispiele für K seien die folgenden Strukturen aufgeführt: 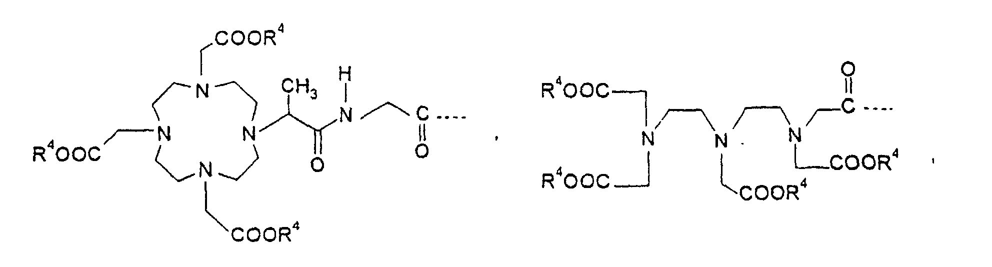 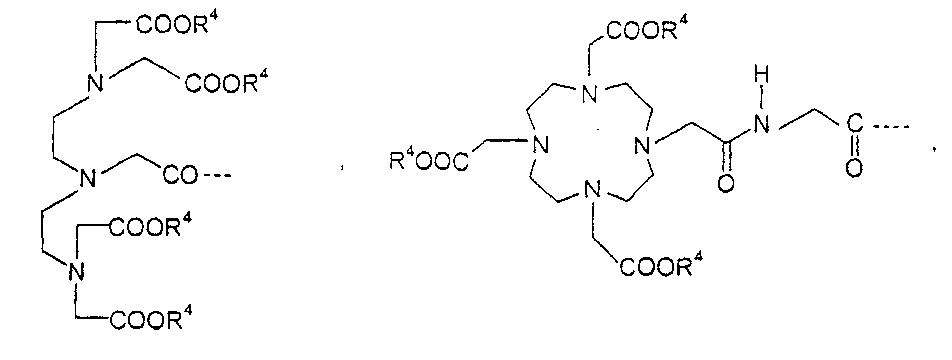 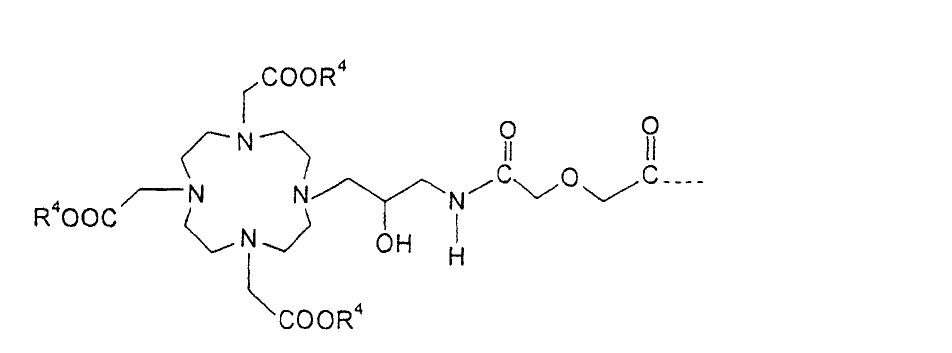 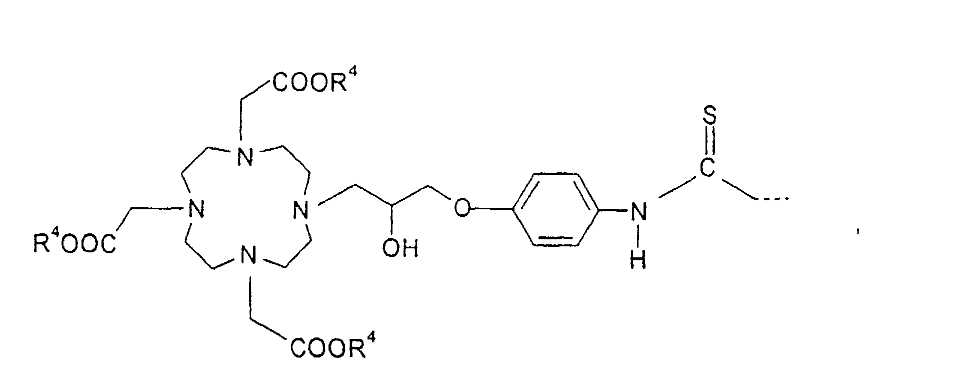 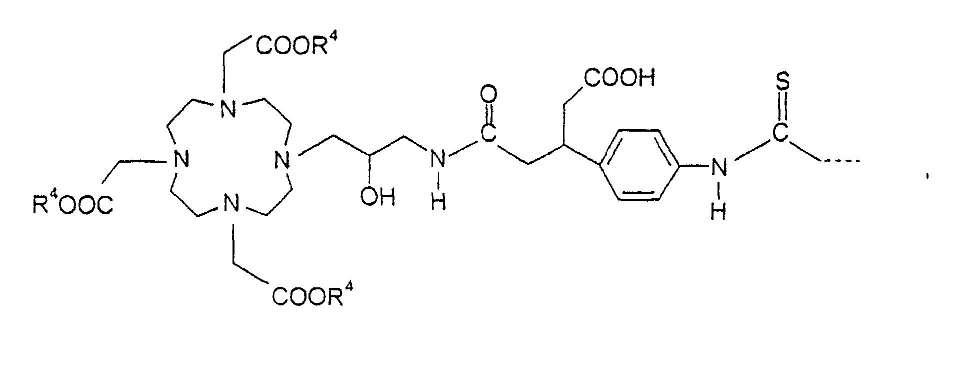 wobei die fünf erstgenannten bevorzugt sind.

Als Molekülteil G ist die direkte Bindung oder die CO-Gruppe bevorzugt.

Das Molekülteil R kann eine Gruppe der allgemeinen Formel IX sein: 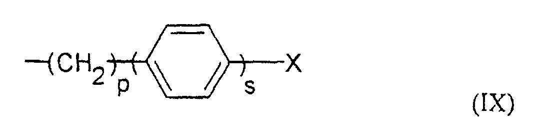 worin p für die Zahlen 0 bis 25 und s für 0, 1 oder 2 stehen und p und s nicht gleichzeitig Null sind,
und worin X ein Wasserstoffatom, eine Methylgruppe, eine Carboxylgruppe, eine OH-Gruppe, eine OCH₃-Gruppe, eine CONH₂-Gruppe, ein Chloratom, eine C₁-C₁₀-Alkylkette, eine O-CₙH₂ₙ₊₁-Gruppe, eine O-(CH₂)ₙ-COOH-Gruppe, eine -O-(CH₂CH₂)ᵣ-CₙH₂ₙ₊₁-Gruppe oder eine NH-CO-CₙH₂ₙ₊₁-Gruppe ist, mit n= 1 - 15 und r = 1 - 5.

Weiterhin kann R eine der folgenden Gruppen sein: 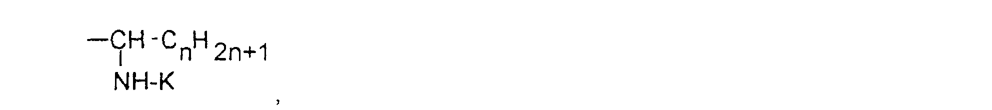 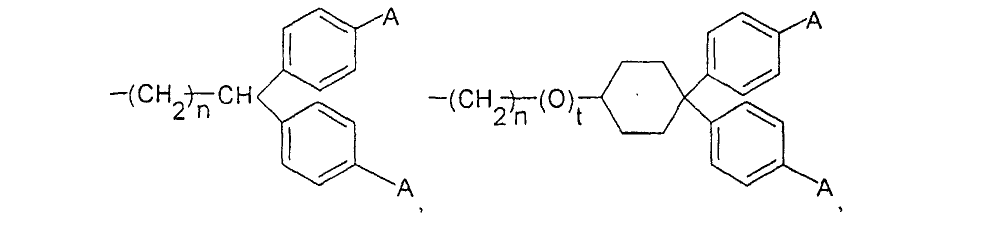 worin n= 1 - 15, t = 0 oder 1 ist und A ein Wasserstoffatom, ein Chloratom oder eine OCH₃-Gruppe bedeutet.

Bevorzugt sind Verbindungen, in denen G und R zusammen eine der folgenden Strukturen aufweisen:
-CO-C₁₅H₃₁, -CO-C₁₄H₂₉, -CO-C₁₃H₂₇, -CO-C₁₀H₂₀-NH-CO-C₆H₁₃, -CO-C₆H₁₃,
-CO-CH₂-O-CH₂CH₂-O-C₁₀H₂₁, -CO-CH₂-O-C₁₃H₂₇, -C₁₅H₃₁, -C₁₄H₂₉, -SO₂-C₁₃H₂₇, -SO₂-C₁₄H₂₉, -SO₂-C₁₅H₃₁,

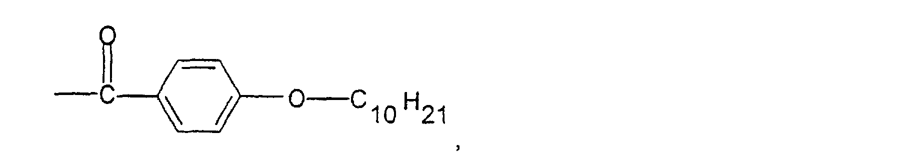

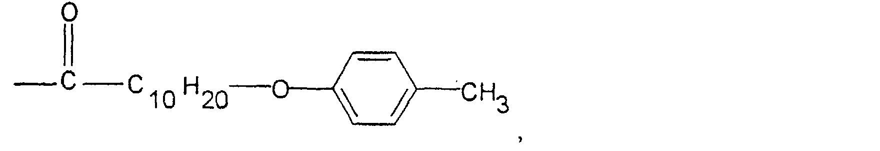

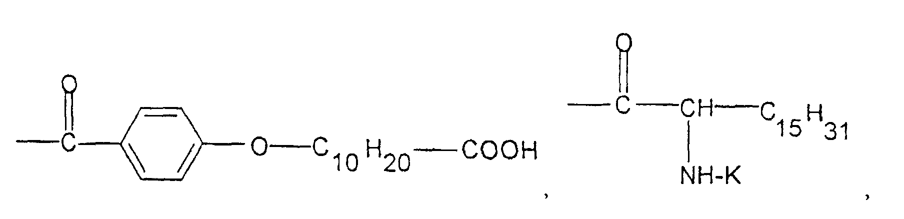

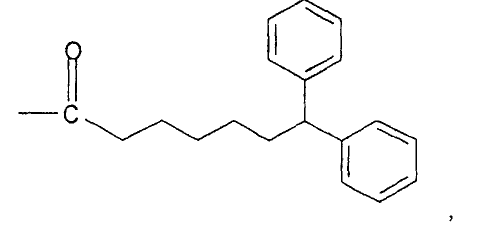

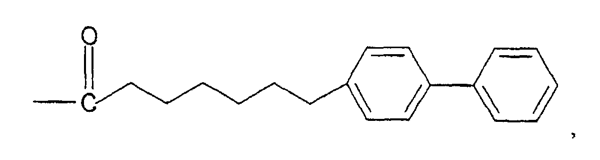

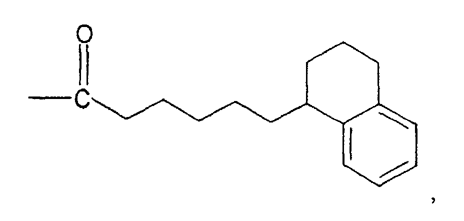

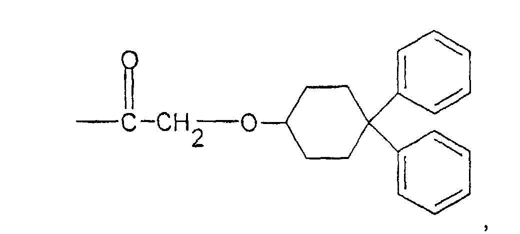

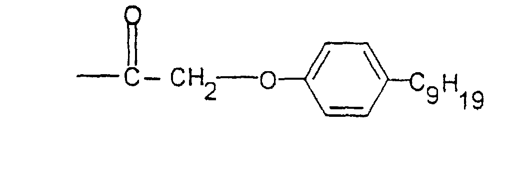

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21 - 29, 42. 44 und 58 - 70. Geeignete Ionen sind beispielsweise das Chrom(III)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)-, Mangan(II)- und Eisen(III)-ion.
Für die Verwendung der erfindungsgemäßen Verbindungen in der Nuklearmedizin muß das Metallion radioaktiv sein. Geeignet sind zum Beispiel Radioisotope der Elemente Kupfer, Kobalt, Gallium, Germanium, Yttrium, Strontium, Technetium, Indium, Ytterbium, Gadolinium, Samarium, Iridium, Rhenium und Bismut; bevorzugt sind Technetium, Gallium, Indium und Rhenium.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21 - 29, 39, 42, 44, 57 - 83, bevorzugt 25, 26 sowie 57 - 83 enthalten, geeignet sind; dies sind beispielsweise das Lanthan(III)-ion und die oben genannten Ionen der Lanthanidenreihe.

Besonders bevorzugt sind dabei Mangan(II)-, Eisen(II)-, Eisen(III)-, Praesodym(III)-, Neodym(III)-, Samarium(III)-, Gadolinium(III)-, Ytterbium(III)- oder Bismut(III)-ionen und insbesondere Dysprosium(III)-ionen.

Die restlichen aciden Wasserstoffatome, das heißt diejenigen, die nicht durch das Zentralion substituiert worden sind, können gegebenenfalls ganz oder teilweise durch Kationen von anorganischen und/oder organischen Basen, Aminosäuren oder Aminosäureamiden ersetzt sein.

Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die Herstellung der erfindungsgemäßen Verbindungen der allgemeinen Formel I erfolgt dadurch, daß man Verbindungen der allgemeinen Formel I' 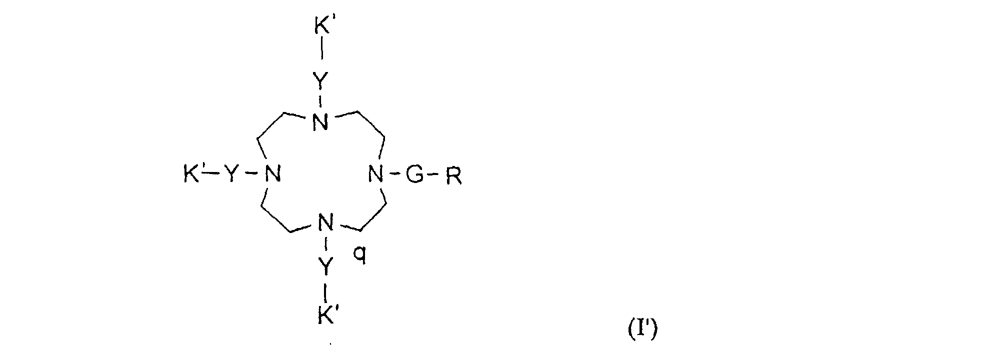 worin K' für K steht mit R⁴ in der Bedeutung eines Wasserstoffatoms oder einer Carboxyschutzgruppe,
nach Abspaltung der gegebenenfalls vorhandenen Schutzgruppen in an sich bekannter Weise mit mindestens einem Metalloxid oder Metallsalz eines Elementes der Ordnungszahlen 20 - 32, 37 - 39, 42 - 44, 49 oder 57 - 83 umsetzt und gegebenenfalls anschließend in den so erhaltenen Komplexverbindungen noch vorhandene acide Wasserstoffatome ganz oder teilweise durch Kationen von anorganischen und/oder organischen Basen, Aminosäuren oder Aminosäureamiden substituiert.

Steht K für einen Tetraazamakrocyclus, kann die Synthese der erfindungsgemäßen Verbindungen auch in der Weise erfolgen, daß man eine Verbindung der allgemeinen Formel I" 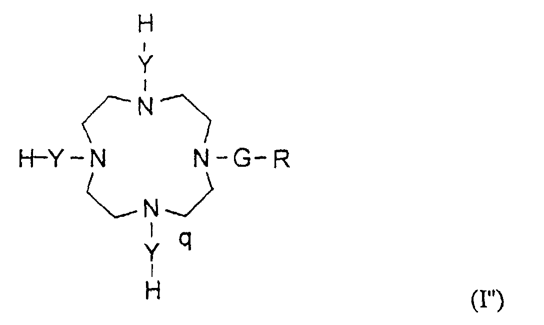 mit einem Komplex V' oder VI' umsetzt, 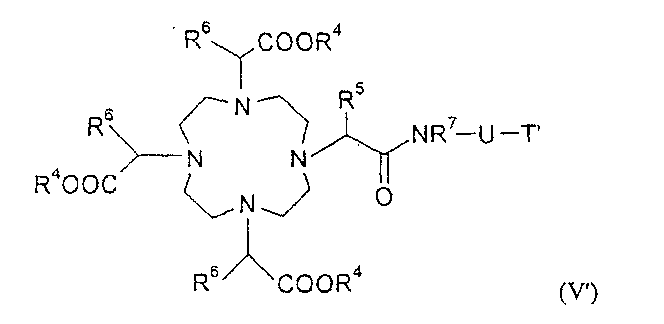 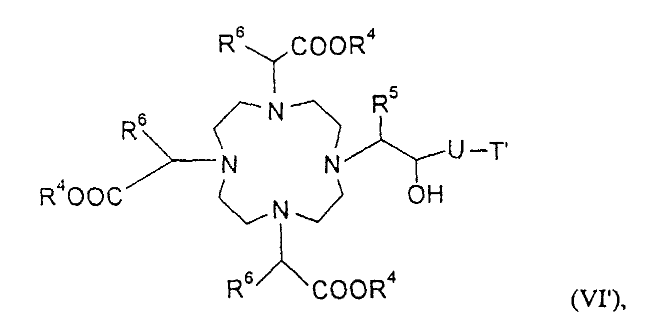 wobei T' für eine -C*O-, -COOH, -N=C=O- oder -N=C=S-Gruppe und -C*O für eine aktivierte Carboxylgruppe steht und die Reste R⁴ bis R⁷ wie oben definiert sind,
mit der Maßgabe, daß gewünschtenfalls weitere Carboxylgruppen in Form ihrer Salze mit anorganischen und/oder organischen Basen, Aminosäuren oder Aminosäureamiden vorliegen.

Als Beispiele für eine aktivierte Carbonylgruppe C*O seien Anhydrid, p-Nitrophenylester, N-Hydroxysuccinimidester, Pentafluorphenylester und Säurechlorid genannt.

Falls R⁴ für eine Säureschutzgruppe steht, kommen niedere Alkyl-, Aryl- und Aralkylgruppen, beispielsweise die Methyl-, Ethyl-, Propyl-, Butyl-, Phenyl-, Benzyl-, Diphenylmethyl-, Triphenylmethyl-, bis-(p-Nitrophenyl)-methylgruppe, sowie Trialkylsilylgruppen in Frage.

Die gegebenenfalls gewünschte Abspaltung der Schutzgruppen erfolgt nach den dem Fachmann bekannten Verfahren (s. z.B. E.Wünsch, Methoden der Org. Chemie, Houben-Weyl, Bd XV/1, 4. Auflage 1974, S. 315), beispielsweise durch Hydrolyse, Hydrogenolyse, alkalische Verseifung der Ester in wäßrig-alkoholischer Lösung bei Temperaturen von 0 °C bis 50 °C, saure Verseifung mit Mineralsäuren oder im Fall von tert-Butylestern mit Hilfe von Trifluoressigsäure.

Die Einführung der gewünschten Metallionen erfolgt in der Weise, wie sie z.B. in der Deutschen Offenlegungsschrift 34 01 052 offenbart worden ist, indem man das Metalloxid oder ein Metallsalz (beispielsweise das Nitrat. Acetat. Carbonat, Chlorid oder Sulfat) des Elements der Ordnungszahlen 20 - 32, 37-39, 42-44, 57 - 83 in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome der Säuregruppen durch Kationen von anorganischen und/oder organischen Basen, Aminosäuren oder Aminosäureamiden substituiert.

Die Einführung der gewünschten Metallionen kann sowohl auf der Stufe der allgemeinen Formel I' oder vor der Kopplung der Strukturteile K, d.h. auf der Stufe der Herstellung der Komplexe V' oder VI' erfolgen.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren, wie zum Beispiel Glycinamid.

Zur Herstellung der neutralen Komplexverbindungen kann man beispielsweise den sauren Komplexsalzen in wäßriger Lösung oder Suspension so viel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Die Reinigung der so erhaltenen Komplexe erfolgt, gegebenenfalls nach Einstellung des pH-Wertes durch Zusatz einer Säure oder Base auf pH 6 bis 8, bevorzugt ca. 7, vorzugsweise durch Ultrafiltration mit Membranen geeigneter Porengröße (z.B. Amicon ®YM1, Amicon®YM3), Gelfiltration an z.B. geeigneten Sephadex®-Gelen oder durch HPLC an Kieselgel oder reverse-phase Material.

Im Falle von neutralen Komplexverbindungen ist es häufig von Vorteil, die oligomeren Komplexe über einen Anionenaustauscher, beispielsweise IRA 67 (OH⁻-Form) und gegebenenfalls zusätzlich über einen Kationenaustauscher, beispielsweise IRC 50 (H⁺-Form) zur Abtrennung ionischer Komponenten zu geben.

Die Synthese der Verbindungen der allgemeinen Formel I', d.h. die Verknüpfung von Komplexbildnern an Verbindungen der Formel I", erfolgt ebenso wie die Kopplung der Metallkomplexe der allgemeinen Formel K' an die Verbindungen der allgemeinen Formel I" analog literaturbekannter Methoden, wie sie z.B. beschrieben sind in US-5,135,737, H. Takalo et al, Bioconjugate Chem. 1994, 5, 278; EP 0430863; EP 0331616; WO 96/01655; EP 0271 180; US-5,364,613; WO 95/17451 und WO 96/02669. Sie wird in Lösungsmitteln wie z.B. Wasser, Methylenchlorid, Acetonitril, Chloroform, DMSO, Pyridin, Ethanol/Wasser, Ethanol/Acetonitril, Dioxan, DMF, THF, niedere Alkohole, Tetramethylharnstoff, N-Methylpyrrolidon, Polyethylenglykole, 1,2-Dimethoxyethan, Dimethylacetamid, Formamid, 1,2-Dichlorethan oder - falls möglich - deren Mischungen mit Wasser bei Temperaturen von -10 °C bis 100 °C, bevorzugt 0 bis 50 °C, besonders bevorzugt bei Raumtemperaturen innerhalb von 5 Minuten bis 72 Stunden, bevorzugt 1 bis 24 Stunden, besonders bevorzugt 1 bis 14 Stunden, gegebenenfalls unter Zusatz einer organischen oder anorganischen Base, wie z.B. aromatischen oder aliphatischen Aminen, Alkali- oder Erdalkali-hydroxiden, -carbonaten oder -hydrogencarbonaten und quartären Ammoniumhydroxiden durchgeführt. Beispielhaft genannt seien Triethylamin, Diisopropyl-N-ethylamin (Hünig-Base), N-Methylmorpholin, Tributylamin, Tetramethylethylendiamin, Pyridin, Lutedin, 2,4,6-Trimethylpyridin, 4-Dimethylaminopyridin, N-Methylimidazol, Tetramethylguanidin, DBU, Lithium-, Natrium-, Kalium, Calcium-, Magnesium-, Bariumhydroxid, -carbonat, -hydrogencarbonat. Die Umsetzung kann auch in den dem Fachmann bekannten Pufferlösungen, vorzugsweise bei pH 8 bis 11, besonders bevorzugt bei pH 8,5 bis 9 erfolgen. Die pH-Wert-Einhaltung erfolgt bevorzugt unter Verwendung eines pH-Staten.

Erfolgt die Kopplung mit einem Metallkomplex so wird bevorzugt Dimethylsulfoxid als Lösungsmittel verwendet. Es hat sich hierbei als vorteilhaft erwiesen, Salze wie z. B. Lithiumchlorid, Natriumbromid. Lithiumbromid und Lithiumjodid als löslichkeitsverbessernde Zusätze zu verwenden.

Wird die Kopplung mit einer in situ aktivierten Carboxylgruppe durchgeführt, so können dem Fachmann bekannte Kupplungs-Reagenzien wie z. B. DCCI, EEDQ, Staab-Reagenz, BOP, PyBOP, TBTU, TDBTU, HBTU (s. z.B. Fournic-Zaluski et al., J. Med. Chem. 1996, 39, 2596; Houben-Weyl, Band XV/2, Teil II, 1974; Y.M. Angell et al, Tetrahedron Letters 1994, 35, 5981; L.A. Carpino et at, J. Chem. Soc. Commun. 1994, 201; H-O. Kim et al, Tetrahedron Letters 1995, 36, 6013; D. Papaioannou et al, Tetrahedron Letters, 1995, 36, 5187, G. Stemple et al, Bioorg. Med. Letters 1996, 6, 55; verwendet werden.

Die als Ausgangssubstanzen verwendeten aktivierten Komplexe bzw. Komplexbildner V', VI', VII', VIII' und VIII'a 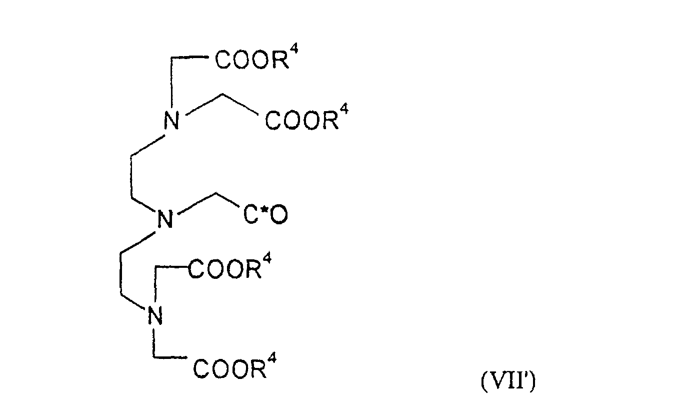 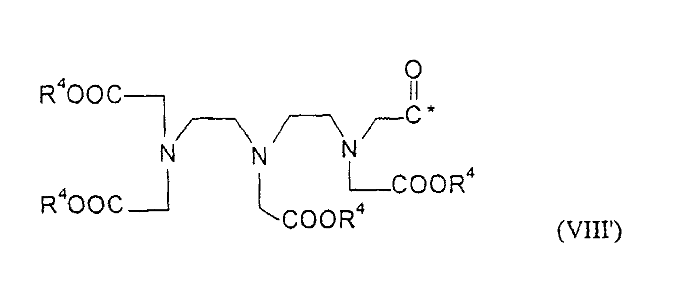 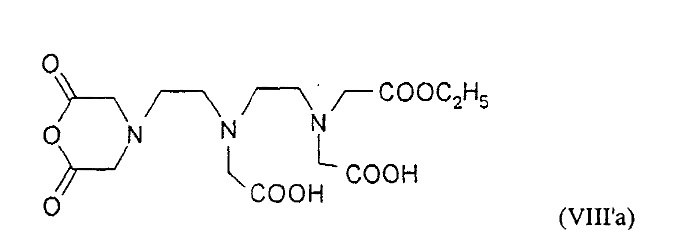 sind literaturbekannt oder analog literaturbekannten Methoden erhältlich:
VIII' und VIII'a s. z.B. EP 263 059,
VII' s. z.B. DE 19507822, DE 19580858, DE 19507819,
V' und VI' s. z.B. US-5,053,503, WO 96/02669, WO 96/01655, EP 0430863, EP 255471, US-5,277,895, EP 0232751, US-4,885,363.

Die Ausgangssubstanzen der allgemeinen Formel I" werden aus Verbindungen der allgemeinen Formel I A 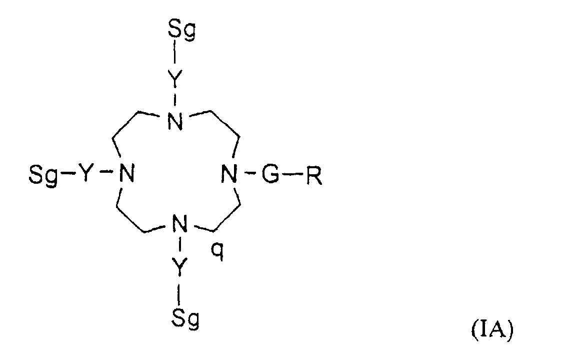 worin Sg für eine Aminoschutzgruppe steht, erhalten.

Als Aminoschutzgruppen seien die dem Fachmann geläufigen Benzyloxycarbonyl-, tertiär-Butoxycarbonyl-, Trifluoracetyl-, Fluorenylmethoxycarbonyl-, Benzyl-, Formyl-, 4-Methoxybenzyl-, 2,2,2-Trichlorethoxycarbonyl-, Phthaloyl-, 1,2-Oxazolin-, Tosyl-, Dithiasuccinoyl-, Allyloxycabonyl-, Sulfat-, Pent-4-encarbonyl-, 2-Chloracetoxymethyl (bzw.-ethyl) benzoyl-, Tetrachlorphthaloyl-, Alkyloxycarbonylgruppen genannt [Th. W. Greene, P.G.M. Wuts, Protective Groups in Organic Syntheses, 2nd ed, John Wiley and Sons (1991), S. 309 - 385; E. Meinjohanns et al, J. Chem. Soc. Pekin Trans 1, 1995, 405; U. Ellensik et al, Carbohydrate Research 280, 1996, 251; R. Madsen et al, J. Org. Chem. 60, 1995, 7920; R.R. Schmidt, Tetrahedron Letters 1995, 5343].

Die Abspaltung der Schutzgruppen erfolgt nach den dem Fachmann bekannten Verfahren (s. z.B. E. Wünsch, Methoden der Org. Chemie, Houben-Weyl, Bd XV/1, 4. Auflage 1974, S. 315), beispielsweise durch Hydrolyse, Hydrogenolyse, alkalische Verseifung der Ester mit Alkali in wäßrig-alkoholischer Lösung bei Temperaturen von 0 °C bis 50 °C, saure Verseifung mit Mineralsäuren oder im Fall von Boc-Gruppen mit Hilfe von Trifluoressigsäure.

Die Herstellung der geschützten Makrocyclen der allgemeinen Formel I A kann durch Acylierung von Verbindungen der allgemeinen Formel I B 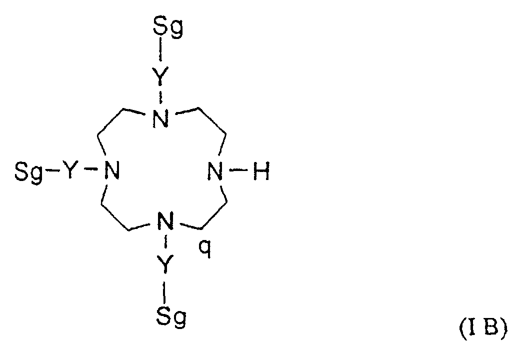 mit einem den gewünschten Substituenten einführenden Substrat der allgemeinen Formel IC

Nu-G-R (I C)

worin Nu für ein Nucelofug steht, erfolgen.

Als Nucleofug dienen vorteilhafterweise die Reste:

F, Cl, Br, I, ―OTs , ―OMs , OH ,

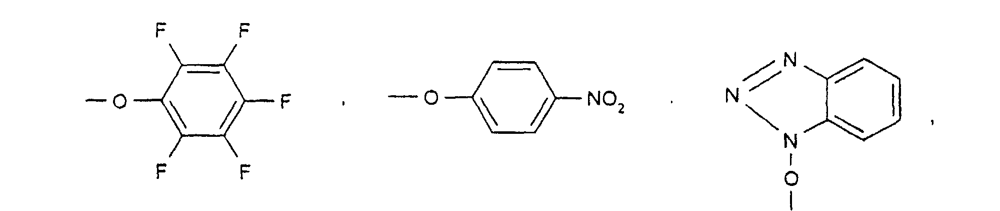

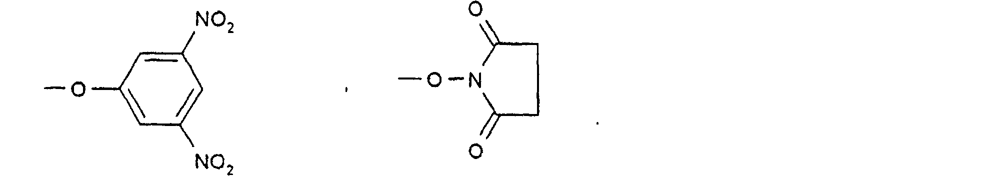

Die Umsetzung wird im Gemisch von Wasser und organischen Lösungsmitteln wie Isopropanol, Ethanol, Methanol, Butanol, Dioxan, Tetrahydrofuran, Dimethylformamid, Dimethylacetamid, Formamid oder Dichlormethan durchgeführt. Bevorzugt sind ternäre Gemische aus Wasser, Isopropanol und Dichlormethan.

Die Umsetzung wird in einem Temperaturintervall zwischen -10 °C - 100 °C, vorzugsweise zwischen 0 °C - 30 °C durchgeführt.

Als Säurefänger dienen anorganische und organische Basen wie Triethylamin, Pyridin, N-Methylmorpholin, Diisopropylethylamin, Dimethylaminopyridin, Alkali- und Erdalkalihydroxyde, ihre Carbonate oder Hydrogencarbonate wie Lithiumhydroxid, Natriumhydroxid, Kaliumhydroxid, Natriumcarbonat, Natriumhydrogencarbonat, Kaliumhydrogencarbonat.

Steht Nu für eine OH-Gruppe, so können dem Fachmann bekannte Kupplungs-Reagenzien wie z.B. DCCI, EEDQ, Staab-Reagenz, BOP, PyBOP, TBTU, TDBTU, HBTU (s. z.B. Fournic-Zaluski et al., J. Med. Chem. 1996, 39, 2596; Houben-Weyl, Band XV/2, Teil II, 1974, Y.M. Angell et al, Tetrahedron Letters 1994, 35, 5981; L.A. Carpino et at, J. Chem. Soc. Commun. 1994, 201; H-O. Kim et al, Tetrahedron Letters 1995, 36, 6013; D. Papaioannou et al, Tetrahedron Letters, 1995, 36, 5187, G. Stemple et al, Bioorg. Med. Letters 1996, 6, 55; verwendet werden.

Die Herstellung der als Edukte für die oben genannte Acylierungsreaktion benötigten Verbindungen IB ist in der DE-OS 19549286 beschrieben.

Die Synthese der Verbindungen der allgemeinen Formel IC erfolgt nach den dem Fachmann bekannten Methoden und ist in den Beispielen hinreichend beschrieben.

Eine weitere Möglichkeit der Synthese von Makrocyclen der allgemeinen Formel IA besteht darin, daß man Verbindungen der allgemeinen Formel IE 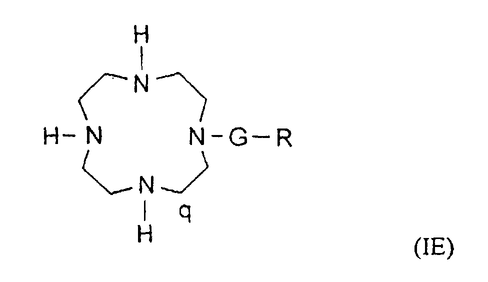 mit literaturbekannten Verbindungen der allgemeinen Formel ID

Nu-Y-Sg (ID)

in einer dem Fachmann bekannten Weise, wie bei der Reaktion von IB mit IC beschrieben, umsetzt.

Die oben genannte Verbindung der allgemeinen Formel IE erhält man durch Abspaltung von Aminoschutzgruppen aus Verbindungen der allgemeinen Formel IF, 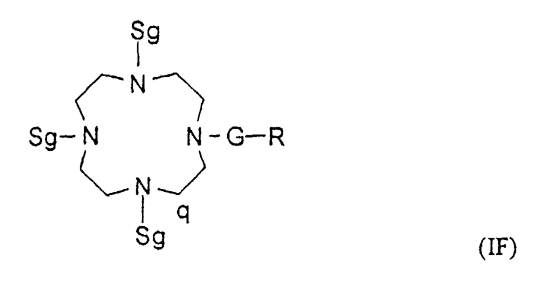 die zugänglich sind aus den literaturbekannten Verbindungen der allgemeinen Formel IG.

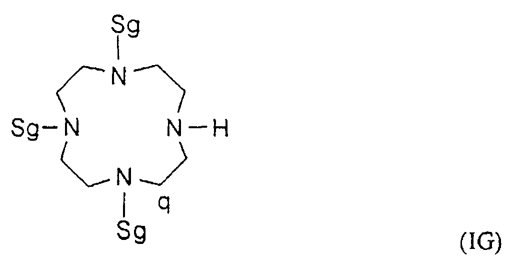

Diese sind durch Acylierung mit Substraten der allgemeinen Formel IC analog der oben beschriebenen Umsetzung von IB mit IC erhältlich.

Die Herstellung der erfindungsgemäßen pharmazeutischen Mittel erfolgt in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern oder schwachen Komplexen (wie zum Beispiel Diethylentriaminpentaessigsäure oder die zu den erfindungsgemäßen Metallkomplexen korrespondierenden Ca-Oligomer-Komplexe) oder - falls erforderlich - Elektrolyte wie zum Beispiel Natriumchlorid oder - falls erforderlich - Antioxidantien wie zum Beispiel Ascorbinsäure.

Sind für die enterale bzw. parenterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [zum Beispiel Methylcellulose, Lactose, Mannit] und/oder Tensid(en) [zum Beispiel Lecithine, Tween®, Myrj® ] und/oder Aromastoff(en) zur Geschmackskorrektur [zum Beispiel ätherischen Ölen] gemischt.

Prinzipiell ist es auch möglich, die erfindungsgemäßen pharmazeutischen Mittel ohne Isolierung der Komplexe herzustellen. In jedem Fall muß besondere Sorgfalt darauf verwendet werden, die Chelatbildung so vorzunehmen, daß die erfindungsgemäßen Komplexe praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexes.

Die erfindungsgemäßen pharmazeutischen Mittel enthalten vorzugsweise 0,1 µMol - 1 Mol/l des Komplexes und werden in der Regel in Mengen von 0,0001 - 5 mMol/kg dosiert. Sie sind zur enteralen und parenteralen Applikation bestimmt. Die erfindungsgemäßen Komplexverbindungen kommen zur Anwendung
1. für die NMR- und Röntgen-Diagnostik in Form ihrer Komplexe mit den Ionen der Elemente mit den Ordnungszahlen 21 - 29, 39, 42, 44 und 57 - 83;
2. für die Radiodiagnostik und Radiotherapie in Form ihrer Komplexe mit den Radioisotopen der Elemente mit den Ordnungszahlen 27, 29, 31, 32, 37 - 39, 43, 49, 62, 64, 70, 75 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,0001 - 5 mMol/kg, vorzugsweise 0,005 - 0.5 mMol/kg, dosiert. Details der Anwendung werden zum Beispiel in H.-J. Weinmann et al., Am. J. of Roentgenology 142, 619 (1984) diskutiert.

Besonders niedrige Dosierungen (unter 1 mg/kg Körpergewicht) von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida, beschrieben.

Eine weitere bildgebende Methode mit Radioisotopen ist die Positronen-Emissions-Tomographie, die positronenemittierende Isotope wie z.B. ⁴³Sc, ⁴⁴Sc, ⁵²Fe, ⁵⁵Co und ⁶⁸Ga verwendet (Heiss, W.D.; Phelps, M.E.; Positron Emission Tomography of Brain, Springer Verlag Berlin, Heidelberg, New York 1983).

Die erfindungsgemäßen Verbindungen sind überraschenderweise auch zur Differenzierung von malignen und benignen Tumoren in Bereichen ohne Blut-Hirn-Schranke geeignet.

Sie zeichnen sich auch dadurch aus, daß sie vollständig aus dem Körper eliminiert werden und gut verträglich sind.

Da sich die erfindungsgemäßen Substanzen in malignen Tumoren anreichern (keine Diffusion in gesunde Gewebe, aber hohe Durchlässigkeit von Tumorgefäßen), können sie auch die Strahlentherapie von malignen Tumoren unterstützen. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Hierzu werden Wechselwirkungen der in den Komplexen enthaltenen Metalle (wie z.B. Eisen oder Gadolinium) mit ionisierenden Strahlungen (z.B. Röntgenstrahlen) oder mit Neutronenstrahlen ausgenutzt. Durch diesen Effekt wird die lokale Strahlendosis am Ort, wo sich der Metallkomplex befindet (z.B. in Tumoren) signifikant erhöht. Um die gleiche Strahlendosis im malignen Gewebe zu erzeugen, kann bei Anwendung solcher Metallkomplexe die Strahlenbelastung für gesunde Gewebe erheblich reduziert und damit belastende Nebenwirkungen für die Patienten vermieden werden. Die erfindungsgemäßen Verbindungen eignen sich deshalb auch als radiosensibilisierende Substanz bei Strahlentherapie von malignen Tumoren (z.B. Ausnutzen von Mössbauer-Effekten oder bei Neutroneneinfangtherapie). Geeignete β-emittierende Ionen sind zum Beispiel ⁴⁶Sc, ⁴⁷Sc, ⁴⁸Sc, ⁷²Ga, ⁷³Ga und ⁹⁰Y. Geeignete geringe Halbwertzeiten aufweisende α-emittierende Ionen sind zum Beispiel ²¹¹Bi, ²¹²Bi, ²¹³Bi und ²¹⁴Bi, wobei ²¹²Bi bevorzugt ist. Ein geeignetes Photonen- und Elektronen-emittierendes Ion ist ¹⁵⁸Gd, das aus ¹⁵⁷Gd durch Neutroneneinfang erhalten werden kann.

Ist das erfindungsgemäße Mittel zur Anwendung in der von R.L. Mills et al. (Nature Vol. 336, (1988), S. 787] vorgeschlagenen Variante der Strahlentherapie bestimmt, so muß sich das Zentralion von einem Mößbauer-Isotop wie beispielsweise ⁵⁷Fe oder ¹⁵¹Eu ableiten.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen therapeutischen Mittel werden parenteral, vorzugsweise i.v., appliziert.

Details der Anwendung von Radiotherapeutika werden z.B. in R.W. Kozak et al. TIBTEC, Oktober 1986, 262, diskutiert.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemischpharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 0,1 - 5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler "Einführung in die Röntgendiagnostik", G. Thieme, Stuttgart, New York (1977) diskutiert.

Insgesamt ist es gelungen, neue Verbindungen zu synthetisieren, die neue Möglichkeiten in der diagnostischen und therapeutischen Medizin erschließen.

Die nachfolgenden Beispiele dienen zur näheren Erläuterung des Erfindungsgegenstands. Dabei werden folgende Abkürzungen benutzt:
a) DTPA-monoanhydrid-ethylester: 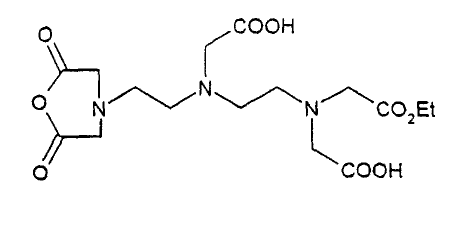
b) sym-DTPA-tetra-t.butylester: 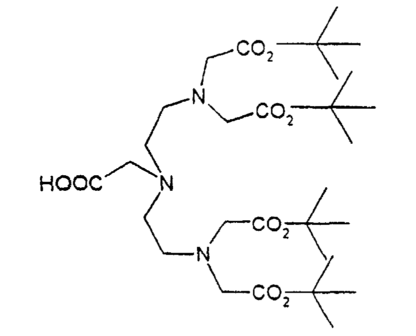
c) Gly-Methyl-DOTA-tri-t.butylester (Natriumbromid-Komplex)

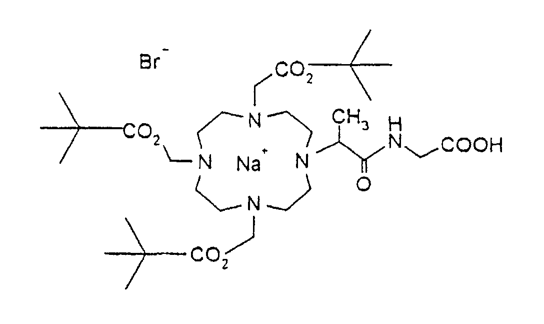

### Beispiel 1

### a) 3-(Bis(N-t.butyloxycarbonylmethyl)-6-[N-(3-aza-2-oxo-4-carboxy)-butyl]-3,6,9-triaza-undecan-1,11-dicarbonsäure-di-t.butylester

50 g (81 mmol) sym.-DTPA-tetra-t.butylester und 11,2 g (97 mmol) N-Hydroxisuccinimid werden in 300 ml Dimethylformamid gelöst und auf 0°C abgekühlt. Man gibt 20,6 g (100 mmol) Dicyclohexylcarbodiimid hinzu und rührt 30 Min. bei 0°C, anschließend 2 Stunden bei Raumtemperatur. Man kühlt auf 0°C und gibt 5,25 g (70 mmol) Glycin zu, anschließend 20,2 g (200 mmol) Triethylamin und läßt langsam auf Raumtemperatur kommen (ca. 4 Stunden). Es wird im Vakuum zur Tockene eingedampft, der Rückstand in 400 ml Methylenchlorid aufgenommen und vom Harnstoff abfiltriert. Das Filtrat wird 3 mal mit einer wäßrigen Salzsäure (pH 3, je 600 ml) extrahiert, über Magnesiumsulfat getrocknet und zur Trockne eingedampft. Der Rückstand wird in Kieselgel chromatographiert (Laufmittel: Methylenchlorid/MeOH = 20 : 1 + 2 % Essigsäure). Man dampft die produkthaltigen Fraktionen im Vakuum zur Trockne ein und kristallisiert den Rückstand aus Methyl-t.Butylether um.
Ausbeute: 43 g (91 % d. Th.) eines farblosen kristallinen Feststoffes

| Analyse: | | | |
|---|---|---|---|
| ber. | C 56,96 | H 8,66 | N 8,30 |
| gef. | C 56,75 | H 8,33 | N 8,13 |

### b) 1,4,7-Tris{3,9-bis(N-t.butyloxycarbonylmethyl)-6-[N-(3-aza-2,5-dioxo-)-pentan-1,5-diyl]-3,6,9-triazaundecandisäure-di-t.butylester}-1,4,7,10-tetraazacyclododecan

40 g (59,27 mmol) der Titelverbindung aus Beispiel 1a und 3,09 g (17,96 mmol) Cyclen (= 1,4,7,10-Tetraazacyclododecan) werden in 300 ml Dimethylformamid gelöst ,und bei 0°C werden 19,8 g (80 mmol) 2-Ethoxy-1-ethoxycarbonyl-1,2-dihydrochinolin zugegeben. Man rührt 24 Stunden bei Raumtemperatur. Es wird im Vakuum zur Trockne eingeengt und der Rückstand an Kieselgel chromatographiert. (Laufmittel: Methylenchlorid/2-Propanol = 20 : 1).
Ausbeute: 11,93 g (31 % d. Th.) eines farblosen, zähen Öls.

| Analyse: | | | |
|---|---|---|---|
| ber. | C 58,30 | H 8,84 | N 10,46 |
| gef. | C 58,12 | H 9,05 | N 10,25 |

### c) 1,4,7-Tris{3,9-bis(N-t.butyloxycarbonylmethyl)-6-[N-(3-aza-2,5-dioxo-pentan-1,5-diyl]-3,6,9-triazaundecandisäure-di-t.butylester}-10-[N-(n hexadecanoyl]-1,4,7,10-tetraazacyclododecan

10 g (4,67 mmol) der Titelverbindung aus Beispiel 1b und 2,93 g (9,33 mmol) Hexadecansäure werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert. (Laufmittel: Methylenchlorid/2-Propanol = 25 : 1).
Ausbeute: 10,12 g (91 % d. Th.) eines farblosen, zähen Öls.

| Analyse: | | | |
|---|---|---|---|
| ber. | C 60,53 | H 9,23 | N 9,41 |
| gef. | C 60,31 | H 9,40 | N 9,27 |

### d) 1,4,7-Tris{3,9-bis(N-carboxylatomethyl)-6-[N-(3-aza-2,5-dioxo-pentan-1,5-diyl]-3,6,9-triazaundecan-1-carboxylato-11-säure, Gd-Komplex, Mononatriumsalz}-10-[N-(n hexadecanoyl]-1,4,7,10-tetraazacyclododecan

10 g (4,2 mmol) der Titelverbindung aus Beispiel 1c werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4 gestellt. Anschließend gibt man 2,28 g (6,3 mmol) Gadoliniumoxid zu und rührt 2 Stunden bei 70°C. Man kühlt auf Raumtemperatur ab und stellt mit 10 %iger Natronlauge auf pH 7,2. Die Lösung wird mit 2 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange bis das Eluat eine Leitfähigkeit von 10 µS erreicht hat. Dann wird der Inhalt der Ultrafiltrationszelle gefriergetrocknet.
Ausbeute: 9,02 g (96 % d. Th.) eines farblosen, amorphen Pulvers
Wassergehalt: 10,1 %

| Analyse (errechnet auf wasserfreie Substanz): | | | | | |
|---|---|---|---|---|---|
| ber. | C 38,67 | H 4,96 | N 10,02 | Gd 21,09 | Na 3,08 |
| gef. | C 38,41 | H 5,18 | N 9,85 | Gd 20,84 | Na 2,84 |

Relaxivity R₁ (1 • mmol⁻¹ s ⁻¹) in Plasma (20 MHz, 40° C, 0,47 Tesla): 21,2

### Beispiel 2

### a) 1,4,7-Tris{3,9-bis(N-t.butyloxycarbonylmethyl)-6-[N-3-aza-2,5-dioxo-pentan-1,5-diyl]-3,6,9-triazaundecandisäure-di-t.butylester}-10-[N-(n pentadecanoyl)]- 1,4,7,10-tetraazacyclododecan

10 g (4,67 mmol) der Titelverbindung aus Beispiel 1b und 2,26 g (9,33 mmol) Pentadecansäure werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/2-Propanol = 25 : 1).
Ausbeute: 10,2 g (92 % d. Th.) eines farblosen, zähen Öls

| Analyse: | | | |
|---|---|---|---|
| ber. | C 60,38 | H 9,20 | N 9,47 |
| gef. | C 60,17 | H 9,35 | N 9,31 |

### b) 1,4,7-Tris{3,9-bis(N-carboxylatomethyl)-6-[N-(3-aza-2,5-dioxo-pentan-1,5-diyl)]-3,6,9-triazaundecan-1-carboxylato-11-säure, Gd-Komplex, Mononatriumsalz}-10-[N-(n pentadecanoyl)]-1,4,7,10-tetraazacyclododecan

9,94 g (4,2 mmol) der Titelverbindung aus Beispiel 2a werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4 gestellt. Anschließend gibt man 2,28 g (6,3 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wir mit 2 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange, bis das Eluat eine Leitfähigkeit von 10 µS erreicht hat. Dann wird der Inhalt der Ultrafiltrationszelle gefriergetrocknet.
Ausbeute: 9,05 g (97 % d. Th.) eines farblosen, amorphen Pulvers
Wassergehalt: 9,8 %

| Analyse (errechnet auf wasserfreie Substanz): | | | | | |
|---|---|---|---|---|---|
| ber. | C 38,37 | H 4,90 | N 10,08 | Gd 21,23 | Na 3,10 |
| gef. | C 38,15 | H 5,11 | N 9,87 | Gd 21,03 | Na 2,85 |

### Beispiel 3

### a) 1,4,7-Tris{3,9-bis(N-t.butyloxycarbonylmethyl)-6-[N-(3-aza-2,5-dioxo-pentan-1,5-diyl)]-3,6,9-triazaundecandisäure-di-t.butylester}-10-[N-(n tetradecanoyl)]-1,4,7,10-tetraazacyclododecan

10 g (4,67 mmol) der Titelverbindung aus Beispiel 1b und 2,13 g (9,33 mmol) Tetradecansäure werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/2-Propanol = 25 : 1).
Ausbeute: 10 g (91 % d Th.) eines farblosen, zähen Öls.

| Analyse: | | | |
|---|---|---|---|
| ber. | C 60,23 | H 9,17 | N 9,52 |
| gef. | C 60,04 | H 9,33 | N 9,36 |

### b) 1,4,7-Tris{3,9-bis(N-carboxylatomethyl)-6-[N-(3-aza-2,5-dioxo-pentan-1,5-diyl)]-3,6,9-triazaundecan-1-carboxylato-11-säure, Gd-Komplex, Mononatriumsalz}-10-[N-(n tetradecanoyl)]-1,4,7,10-tetraazacyclododecan

9,88 g (4,2 mmol) der Titelverbindung aus Beispiel 3a werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4 gestellt. Anschließend gibt man 2,28 g (6,3 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wir mit 2 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange, bis das Eluat eine Leitfähigkeit von 10 µS erreicht hat. Dann wird der Inhalt der Ultrafiltrationszelle gefriergetrocknet.
Ausbeute: 8,90 g (96 % d. Th.) eines farblosen, amorphen Pulvers
Wassergehalt: 11,0 %

| Analyse (errechnet auf wasserfreie Substanz): | | | | | |
|---|---|---|---|---|---|
| ber. | C 38,07 | H 4,84 | N 10,15 | Gd 21,36 | Na 3,12 |
| gef. | C 37,84 | H 4,97 | N 9,93 | Gd 21,14 | Na 2,80 |

Relaxivity R₁ (1 • mmol⁻¹ s ⁻¹) in Plasma (20 MHz, 40° C, 0.47 Tesla) : 19,2

### Beispiel 4

### 1,4,7-Tris{3,9-bis(N-carboxylatomethyl)-6-[N-3-aza-2,5-dioxo-pentan-1,5-diyl]-3,6,9-triazaundecan-1-carboxylato-11-säure, Dy-Komplex, Mononatriumsalz}-10-[N-(nhexadecanoyl]-1,4,7,10-tetraazacyclododecan

10 g (4,2 mmol) der Titelverbindung aus Beispiel 1c werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4 gestellt. Anschließend gibt man 2,35 g (6,3 mmol) Dysprosiumoxid zu und rührt 2 Stunden bei 70°C. Man kühlt auf Raumtemperatur ab und stellt mit 10 %iger aqu. Natronlauge auf pH 7,2. Die Lösung wird mit 2 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange bis das Eluat eine Leitfähigkeit von 10 µS erreicht hat. Dann wird der Inhalt der Ultrafiltrationszelle gefriergetrocknet.
Ausbeute: 8,18 g (93 % d. Th.) eines farblosen, amorphen Pulvers
Wassergehalt: 7,8 %

| Analyse (errechnet auf wasserfreie Substanz): | | | | | |
|---|---|---|---|---|---|
| ber. | C 38,40 | H 4,92 | N 9,95 | Dy 21,65 | Na 3,06 |
| gef. | C 38,20 | H 5,13 | N 9,84 | Dy 21,43 | Na 2,85 |

### Beispiel 5

### 1,4,7-Tris {3,9-bis(N-carboxylatomethyl)-6-[N-3-aza-2,5-dioxo-pentan-1,5-diyl]-3,6,9-triazaundecan-1-carboxylato-11-säure,Yb-Komplex, Mononatriumsalz}-10-[N-(npentadecanoyl]-1,4,7,10-tetraazacyclododecan

10 g (4,2 mmol) der Titelverbindung aus Beispiel 1c werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4 gestellt. Anschließend gibt man 3,31 g (6,3 mmol) Ytterbiumcarbonat zu und rührt 2 Stunden bei 70°C. Man kühlt auf Raumtemperatur ab und stellt mit 10 %iger aqu. Natronlauge auf pH 7,2. Die Lösung wird mit 2 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange bis das Eluat eine Leitfähigkeit von 10 µS erreicht hat Dann wird der Inhalt der Ultrafiltrationszelle gefriergetrocknet.
Ausbeute: 9,02 g (94 % d. Th.) eines farblosen, amorphen Pulvers
Wassergehalt: 9, 1 %

| Analyse (errechnet auf wasserfreie Substanz): | | | | | |
|---|---|---|---|---|---|
| ber. | C 37,87 | H 4,85 | N 9,81 | Yb 22,73 | Na 3,02 |
| gef. | C 37,61 | H 5,09 | N 9,62 | Yb 22,50 | Na 2,76 |

### Beispiel 6

### 1,4,7-Tris{3,9-bis(N-carboxylatomethyl)-6-[N-3-aza-2,5-dioxo-pentan-1,5-diyl]-3,6,9-triazaundecan-1-carboxylato-11-säure, Eu-Komplex, Mononatriumsalz}-10-[N-(ntetradecanoyl)]-1,4,7,10-tetraazacyclododecan

10 g (4,2 mmol) der Titelverbindung aus Beispiel 1c werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4 gestellt. Anschließend gibt man 3,05 g (6,3 mmol) Europiumcarbonat zu und rührt 2 Stunden bei 70°C. Man kühlt auf Raumtemperatur ab und stellt mit 10 %iger aqu. Natronlauge auf pH 7,2. Die Lösung wird mit 2 g Aktivkohle versetzt, 1 Stunde bei Raumtemperatur gekühlt und filtriert. Das Filtrat wird in eine Ultrafiltrationszelle gefüllt und dialysiert (Amicon® YM1). Man dialysiert solange bis das Eluat eine Leitfähigkeit von 10 µS erreicht hat. Dann wird der Inhalt der Ultrafiltrationszelle gefriergetrocknet.
Ausbeute: 8,67 g (93 % d. Th.) eines farblosen, amorphen Pulvers
Wassergehalt: 9,3 %

| Analyse (errechnet auf wasserfreie Substanz): | | | | | |
|---|---|---|---|---|---|
| ber. | C 38,94 | H 4,99 | N 10,09 | Eu 20,53 | Na 3,11 |
| gef. | C 38,75 | H 5,18 | N 9,87 | Eu 20,31 | Na 2,84 |

### Beispiel 7

### a) Heptansäure-N-(10-carboxy)-decylamid

10 g (49,7 mmol) 11-Aminoundecansäure werden in einer Mischung aus 100 ml Tetrahydrofuran/100 ml Wasser suspendiert und der pH-Wert wird durch Zugabe von 10 %iger aqu. Natronlauge auf pH 10 gebracht. Man kühlt auf 0°C und tropft innerhalb 30 Minuten 8,86 g (59,6 mmol) n-Heptansäurechlorid gelöst in 50 ml Tetrahydrofuran zu. Dabei hält man den pH-Wert durch gleichzeitige Zugabe von Natronlauge zwischen pH 9-10. Dann rührt man 1 Stunde bei Raumtemperatur. Es wird mit 10 %iger aqu. Salzsäure angesäuert (pH 1), 200 ml Essigsäureethylester zugegeben und die organische Phase abgetrennt. Die organische Phase wird mit 100 ml gesättigter Kochsalzlösung gewaschen, über Magnesiumsulfat getrocknet und im Vakuum zur Trockne eingedampft. Der Rückstand wird aus Diethylester umkristallisiert.
Ausbeute: 13,87 g (89 % d. Th.) eines farblosen, wachsartigen Feststoffes.

| Analyse: | | | |
|---|---|---|---|
| ber. | C 68,97 | H 11,25 | N 4,47 |
| gef. | C 68,75 | H 11,38 | N 4,29 |

### b) 1,4,7-Tris{3,9-bis(N-t.butyloxycarbonylmethyl)-6-[N-(3-aza-2,5-dioxo-pentan-1,5-diyl)]-3,6,9-triazaundecandisäure-di-t.butylester}-10-[N-(12-aza-13-oxo)-nonadecanoyl]-1,4,7,10-tetraazacyclododecan

10 g (4,67 mmol) der Titelverbindung aus Beispiel 1b und 2,92 g (9,33 mmol) der Titelverbindung aus Beispiel 7a werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird in Vakuum zur Trockne eingedampft und der Rückstand an Kieselgel chromatographiert (Laufmittel: Methylenchlorid/2-Propanol = 25 : 1).
Ausbeute: 10,47 g (92 % d. Th.) eines farblosen, zähen Öls

| Analyse: | | | |
|---|---|---|---|
| ber. | C 60,10 | H 9,14 | N 9,77 |
| gef. | C 59,91 | H 9,36 | N 9,63 |

### c) 1,4,7-Tris{3,9-bis(N-carboxylatomethyl)-6-[N-(3-aza-2,5-dioxo-pentan-1,5-diyl)]-3,6,9-triazaundecan-1-carboxylato-11-säure, Gd-Komplex, Mononatriumsalz}-10-[N-(12-aza-13-oxo)-monodecanoyl]-1,4,7,10-tetraazacyclododecan

10,24 g (4,2 mmol) der Titelverbindung aus Beispiel 7b werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4 gestellt. Anschließend gibt man 2,28 g (6,3 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 2 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange bis das Eluat eine Leitfähigkeit von 10 µS erreicht hat. Dann wird der Inhalt der Ultrafiltrationszelle gefriergetrocknet.
Ausbeute: 8,96 g (93 % d. Th.) eines farblosen, amorphen Pulvers
Wassergehalt: 9,4 %

| Analyse (errechnet auf wasserfreie Substanz): | | | | | |
|---|---|---|---|---|---|
| ber. | C 38,75 | H 4,97 | N 10,38 | Gd 20,57 | Na 3,01 |
| gef. | C 38,58 | H 5,18 | N 10,15 | Gd 20,39 | Na 2,78 |

### Beispiel 8

### a) 4-(11-Oxaundecyl)-benzoesäure

Zu einer Mischung aus 20 g (144,8 mmol) 4-Hydroxybenzoesäure und 46 g (434,4 mmol) Natriumcarbonat in 400 ml Dimethylformamid gibt man 38,83 g (144,8 mmol) Decyljodid und erwärmt 4 Stunden auf 70°C. Man filtriert vom Feststoff ab, dampft das Filtrat im Vakuum zur Trockne ein und nimmt den Rückstand in 300 ml Methylenchlorid auf und gibt 300 ml 5 %ige aqu. Salzsäure zu. Man rührt gut durch, trennt die organische Phase ab und trocknet sie über Magnesiumsulfat. Anschließend wird die organische Phase zur Trockne eingedampft. Der Rückstand wird an Kieselgel chromatographiert (Laufmittel: n-Hexan-Aceton = 20 : 1).
Ausbeute: 15,3 1 g (38 % d. Th.) eines farblosen, glasigen Feststoffes

| Analyse: | | |
|---|---|---|
| ber. | C 73,35 | H 9,41 |
| gef. | C 73,17 | H 9,65 |

### b) 1,4,7-Tris{3,9-bis(N-t.butyloxycarbonylmethyl)-6-[N-(3-aza-2,5-dioxo-pentan-1,5-diyl)]-3,6,9-triazaundecandisäure-di-t.butylester}-10-<N-[4-(11-oxaundecyl)]-benzoyl> 1,4,7,10-tetraazacyclododecan

10 g (4,67 mmol) der Titelverbindung aus Beispiel 1b und 2,60 (9,33 mmol) der Titelverbindung aus Beispiel 8a werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/2-Propanol = 25 : 1)
Ausbeute: 10,32 g (92 % d. Th.) eines farblosen, zähen Öls
Wassergehalt: 9,4 %

| Analyse: | | | |
|---|---|---|---|
| ber. | C 60,48 | H 8,89 | N 9,33 |
| gef. | C 60,29 | H 8,71 | N 9,15 |

### c) 1,4,7-Tris{3,9-bis(N-carboxylatomethyl)-6-[N-(3-aza-2,5-dioxo-pentan-1,5-diyl)]-3,6,9-triazaundecan-1-carboxylato-11-säure, Gd-Komplex, Mononatriumsalz}-10-[N-4-(11-oxaundecyl)-benzoyl]-1,4,7, 11-tetraazacyclododecan

10,1 g (4,2 mmol) der Titelverbindung aus Beispiel 8b werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4 gestellt. Anschließend gibt man 2,28 g (6,3 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 2 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange bis das Eluat eine Leitfähigkeit von 10 µS erreicht hat. Dann wird der Inhalt der Ultrafiltrationszeile gefriergetrocknet.
Ausbeute: 9,2 g (94 % d. Th.) eines farblosen, amorphen Pulvers
Wassergehalt: 7,9 %

| Analyse (errechnet auf wasserfreie Substanz): | | | | | |
|---|---|---|---|---|---|
| ber. | C 38,82 | H 4,64 | N 9,92 | Gd 20,89 | Na 3,05 |
| gef. | C 38,63 | H 4,78 | N 9,70 | Gd 20,64 | Na 2,81 |

### Beispiel 9

### a) 11-Bromundecansäure-benzylester

30 g (113,1 mmol) 11-Bromundecansäure, 18,35 g (169,7 mmol) Benzylalkohol und 0,5 g p-Toluolsulfonsäure werden in 200 ml Toluol 12 Stunden am Wasserabscheider erhitzt. Man läßt auf Raumtemperatur kommen und wäscht die organische Phase 2 mal mit je 100 ml 5 %iger aqu. Kalium-Carbonat-Lösung. Die organische Phase wird über Magnesiumsulfat getrocknet und im Vakuum eingeengt. Der Rückstand wird an Kieselgel chromatographiert (Laufmittel: n-Hexan/Aceton = 30 : 1).
Ausbeute: 32,55 g (81 % d. Th.) eines farblosen Öls

| Analyse: | | | |
|---|---|---|---|
| ber. | C 60,85 | H 7,66 | Br 22,49 |
| gef. | C 60,64 | H 7,83 | Br 22,31 |

### b) 11-(4-Methylphenyl)-11-oxaundecansäurebenzylester

Zu einer Mischung aus 9,73 g (90 mmol) 4-Hydroxy-Toluol und 37,32 g (270 mmol) Kaliumcarbonat in 200 ml Dimethylformamid gibt man 32 g (90 mmol) der Titelverbindung aus Beispiel 9a und erwärmt 4 Stunden auf 70°C.

Man filtriert vom Feststoff ab, dampft das Filtrat im Vakuum zur Trockne ein und nimmt den Rückstand in 300 ml Methylenchlorid auf und gibt 200 ml 5 %ige aqu. Salzsäure zu. Man rührt gut durch, trennt die organische Phase ab und trocknet sie über Magnesiumsulfat. Anschließend wird die organische Phase zur Trockne eingedampft. Der Rückstand wird an Kieselgel chromatographiert (Laufmittel = n-Hexan-Aceton = 30-: 1). Ausbeute: 26,5 g (77 % d. Th.) eines farblosen, zähen Öls.

| Analyse: | | |
|---|---|---|
| ber. | C 78,49 | H 8,96 |
| gef. | C 78,49 | H 8,96 |

### c) 1-(4-Methylphenyl)-11-oxaundecansäure

26 g (68 mmol) der Titelverbindung aus Beispiel 9b werden in 300 ml 2-Propanol gelöst und 4 g Palladiumkatalysator (10 % Pd/C) zugegeben. Man hydriert über Nacht bei Raumtemperatur. Es wird vom Katalysator abfiltriert und das Filtrat im Vakuum zur Trockne eingedampft.
Ausbeute: 19,87 g (quantitativ) eines glasigen, farblosen Feststoffes.

| Analyse: | | |
|---|---|---|
| ber. | C 73,93 | H 9,65 |
| gef. | C 73,70 | H 9,74 |

### d) 1,4,7-Tris{3,9-bis(N-tbutyloxycarbonylmethyl)-6-[N-(3-aza-2,5-dioxo-pentan-1,5-diyl)]-3,6,9-triazaundecandisäure-di-t.butylester}-10-[N-(11-(4-methylphenyl)-11-oxaundecanoyl]-1,4,7,10-tetraazacyclododecan

10 g (4,67 mmol) der Titelverbindung aus Beispiel 1b und 2,73 g (9,33 mmol) der Titelverbindung aus Beispiel 9c werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/2-Propanol = 25 : 1)
Ausbeute: 10,61 g (94 % d. Th.) eines farblosen, zähen Öls

| Analyse: | | | |
|---|---|---|---|
| ber. | C 60,62 | H 8,92 | N 9,27 |
| gef. | C 60,48 | H 9,10 | N 9,08 |

### e) 1,4,7-Tris{3,9-bis(N-carboxylatomethyl)-6-[N-(3-aza-2,5-dioxo-pentan-1,5-diyl)]-3,6,9-triazaundecan-1-carboxylato-11-säure, Gd-Komplex, Mononatriumsalz}-10-<N-[11-(4-methylphenyl)-11-oxaundecanoyl]>-1,4,7,10-tetraazacyclododecan

10,15 g (4,2 mmol) der Titelverbindung aus Beispiel 9e werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4 gestellt. Anschließend gibt man 2,28 g (6,3 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 2 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange bis das Eluat eine Leitfähigkeit von 10 µS erreicht hat. Dann wird der Inhalt der Ultrafiltrationszelle gefriergetrocknet.
Ausbeute: 8,97 g (94 % d. Th.) eines farblosen, amorphen Pulvers
Wassergehalt: 9,2 %

| Analyse (errechnet auf wasserfreie Substanz): | | | | | |
|---|---|---|---|---|---|
| ber. | C 39,11 | H 4,70 | N 9,86 | Gd 20,76 | Na 3,04 |
| gef. | C 39,01 | H 4,89 | N 9,70 | Gd 20,50 | Na 2,73 |

### Beispiel 10

### a) 4-(12-oxadodecansäurebenzylester)-benzoesäure

Zu einer Mischung aus 30 g (217,2 mmol) 4-Hydroxybenzoesäure und 90,05 g (651,6 mmol) Kaliumcarbonat in 400 ml Dimethylformamid gibt man 77,2 g (217,2 mmol) der Titelverbindung aus Beispiel 9a und erwärmt 4 Stunden auf 70°C. Man filtriert vom Feststoff ab, dampft das Filtrat im Vakuum zur Trockne ein und nimmt den Rückstand in 1000 ml Methylenchlorid auf und gibt 500 ml 5 %ige aqu. Salzsäure zu. Man rührt gut durch, trennt die organische Phase ab und trocknet sie über Magnesiumsulfat. Anschließend wird die organische Phase zur Trockne eingedampft. Der Rückstand wird an Kieselgel chromatographiert (Laufmittel: n-Hexan/Aceton = 20 : 1). Ausbeute: 48,38 g (54% d. Th.) eines farblosen, glasigen Feststoffes.

| Analyse: | | |
|---|---|---|
| ber. | C 72,79 | H 7,82 |
| gef. | C 72,58 | H 7,97 |

### b) 1,4,7-Tris{3,9-bis(N-t.butyloxycarbonylmethyl)-6-[N-(3-aza-2,5-dioxo-pentan-1,5-diyl)]-3,6,9-triazaundecandisäure-di-t.butylester}-10-<N-[4-(12-oxadodecansäurebenzylester)]-benzoyl>-1,4,7,10-tetraazacyclododecan

20 g (9,34 mmol) der Titelverbindung aus Beispiel 1b und 7,71 g (18,68 mmol) der Titelverbindung aus Beispiel 10a werden in 200 ml Dimethylformamid gelöst, und bei 0°C werden 7,42 g (30 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/2-Propanol = 25 : 1)
Ausbeute: 22.04 g (93 % d. Th.) eines farblosen, zähen Öls

| Analyse | | | |
|---|---|---|---|
| ber. | C 61,07 | H 8,66 | N 8,83 |
| gef. | C 60,94 | H 8,83 | N 8,70 |

### c) 1,4,7-Tris{3,9-bis(N-t.butyloxycarbonylmethyl)-6-[N-(3-aza-2,5-dioxo-pentan-1,5-diyl)]-3,6,9-triazaundecandisäure-di-t.butylester}-10-<N-[4-(12-oxadodecansäure)]-benzoyl>- 1,4,7,10-tetraazacyclododecan

20 g (7,88 mmol) der Titelverbindung aus Beispiel 10b werden in 300 ml 2-Propanol gelöst und 4 g Palladiumkatalysator (10 % Pd/C) zugegeben. Man hydriert über Nacht bei Raumtemperatur. Es wird vom Katalysator abfiltriert und das Filtrat im Vakuum zur Trockne eingeengt.
Ausbeute: 19,2 g (quantitativ) eines farblosen, zähen Öls.

| Analyse: | | | |
|---|---|---|---|
| ber. | C 59,88 | H 8,73 | N 9,16 |
| gef. | C 59,66 | H 8,95 | N 9,04 |

### d) 1,4,7-Tris{3,9-bis(N-carboxylatomethyl)-6-[N-(3-aza-2,5-dioxo-pentan-1,5-diyl)]-3,6,9-triazaundecan-1-carboxylato-11-säure, Gd-Komplex, Mononatriumsalz}-10-<N-[4-(12-oxadodecansäure,Natriumsalz)]-benzoyl>-1,4,7,10-tetraazacyclododecan

10,28 g (4,2 mmol) der Titelverbindung aus Beispiel 10c werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4
gestellt. Anschließend gibt man 2,28 g (6,3 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 2 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange, bis das Eluat eine Leitfähigkeit von 10 µS erreicht hat. Dann wird der Inhalt der Ultrafiltrationszelle gefriergetrocknet. Ausbeute: 9,18 g (94% d. Th.) eines farblosen, amorphen Pulvers
Wassergehalt: 7,8 %

| Analyse (errechnet auf wasserfreie Substanz): | | | | | |
|---|---|---|---|---|---|
| ber. | C 38,24 | H 4,47 | N 9,64 | Gd 20,30 | Na 3,96 |
| gef. | C 38,13 | H 4,62 | N 9,49 | Gd 20,13 | Na 3,59 |

### Beispiel 11

### a) 1,4,7-Tris{N-[2,6-bis(benzyloxycarbonylamino)]-hexanoyl}-1,4,7,10-tetraazacyclododecan

Zu 10 g (58 mmol) 1,4,7,10- Tetraazacyclododecan (= Cyclen) in 700 ml Toluol gibt man 92,13 g (180 mmol) Di-(benzyloxycarbonyl)-Lysin-N-Hydroxysuccinimidester (=Z₂-LysNHSI) und 20,24 g (200 mmol) Triethylamin und erhitzt 12 Stunden unter Rückfluß. Man dampft im Vakuum zur Trockne ein, nimmt den Rückstand in 1000 ml Dichlormethan auf und wäscht die organische Phase 2 mal mit 5 %iger aqu. Natriumcarbonat-Lösung. Man trocknet die organische Phase über Magnesiumsulfat und engt im Vakuum zur Trockne ein. Der Rückstand wird an Kieselgel chromatographiert (Laufmittel: Essigsäureethylester/Ethanol = 20 : 1).
Ausbeute: 48,26 g (61% d. Th.) eines farblosen Schaums.

| Elementaranalyse: | | | |
|---|---|---|---|
| ber. | C 65,28 | H 6,81 | N 10,29 |
| gef. | C65,15 | H6,97 | N 10,10 |

### b) 1,4,7-Tris{N-[2,6-bis(benzyloxycarbonylamino)]-hexanoyl}-10-(N-hexadecanoyl)-1,4,7,10-tetraazacyclododecan

12,72 g (9,34 mmol) der Titelverbindung aus Beispiel 11a und 4,79 g (18,68 mmol) Palmitinsäure werden in 100 ml Dimethylformamid gelöst, und bei 0°C werden 7,42 g (30 mmol) 2-Ethoxy-1-ethoxycarbonyl-1,2-dihydrochinolin zugegeben. Man rührt 24 Stunden bei Raumtemperatur.
Es wird im Vakuum zur Trockne eingedampft und der Rückstand an Kieselgel chromatographiert (Laufmittel: Essigsäureethylester/Ethanol = 25 : 1)
Ausbeute: 14,2 g (95 % d. Th.) eines farblosen, zähen Öls

| Analyse: | | | |
|---|---|---|---|
| ber. | C 67,56 | H 7,69 | N 8,75 |
| gef. | C 67,38 | H 7,83 | N 8,60 |

### c) 1,4,7-Tris-[N-(2,6-diamino)-hexanoyl]-10-(N-hexadecanoyl)- 1,4,7,10-tetraazacyclododecan, Hexahydrobromid

14 g (8,75 mmol) der Titelverbindung aus Beispiel 11b werden in 60 ml Essigsäure gelöst und zu einer 60°C heißen Lösung aus 150 ml Bromwasserstoff in Eisessig (33 %ig) getropft. Man rührt 1 Stunde bei 60°C. Es wird auf 0°C abgekühlt und unter Rühren 1000 ml Diethylether langsam zugetropft. Man filtriert den ausgefallenen Niederschlag ab, wäscht 2 mal mit 100 ml Ether nach und trocknet im Vakuumofen (60°C).
Ausbeute: 10,87 g (97 % d. Th.) cremfarbener, kristalliner Feststoff

| Elementaranalyse: | | | | |
|---|---|---|---|---|
| ber. | C 39,39 | H 7,24 | N 10,94 | Br 37,44 |
| gef. | C 39,28 | H 7,35 | N 10,81 | Br 37,25 |

### d) 1,4,7-Tris{N-hexanoyl-2,6-bis<amino-N-acetyl-2-[3,9-bis(N-t.butyloxycarboxylmethyl)-6-yl-3,6,9-triazaundecan-1,11-dicarbonsäure-di-t.butylester]>}-10-(Nhexadecanoyl)-1,4,7,10-tetraazacyclododecan

Zu 67,42 g (109,3 mmol) sym.-DTPA-tetra-t.butylester, gelöst in 450 ml Dimethylformamid, gibt man bei 0°C 23,02 g (200 mmol) N-Hydroxysuccinimid und 30,9 g (150 mmol) Dicyclohexylcarbodiimid und rührt eine Stunde bei dieser Temperatur. Anschließend rührt man 3 Stunden bei Raumtemperatur. Zu dieser Lösung gibt man 14 g (10,93 mmol) der Titelverbindung aus Beispiel 11c und 30,4 g (300 mmol) Triethylamin. Man rührt 24 Stunden bei Raumtemperatur. Die Lösung wird zur Trockne eingedampft und der Rückstand in 600 ml Dichlormethan aufgenommen. Man filtriert vom Dicyclohexylharnstoff ab und wäscht das Filtrat 2 mal mit 300 ml 5 %iger aqu. Natriumcarbonat-Lösung. Die organische Phase wird über Magnesiumsulfat getrocknet und im Vakuum zur Trockne eingedampft.
Der Rückstand wird an Kieselgel chromatographiert (Laufmittel: Dichlormethan/2-Propanol = 25 : 1).
Ausbeute: 32,2 g (67 % d. Th.) eines farblosen, zähen Öls

| Elementaranalyse: | | | |
|---|---|---|---|
| ber. | C 60,69 | H 9,27 | N 8,93 |
| gef. | C 60,48 | H 9,38 | N 8,75 |

### e) 1,4,7-Tris{N-hexanoyl-2,6-bis<amino-N-acetyl-2-[3,9-bis(N-carboxylatomethyl)-6-yl-3,6,9-triazaundecan-1-carboxylato-11-säure, Gd-Komplex, Mononatriumsalz]>}-10-(N-hexadecanoyl)-1,4,7,10-tetraazacyclododecan

18,45 g (4,2 mmol) der Titelverbindung aus Beispiel 11d werden in 300 ml Trifluoressigsäure gelöst und 12 Stunden bei Raumtemperatur gerührt. Man dampft im Vakuum zur Trockne ein. Der Rückstand wird in 150 ml Wasser gelöst und mit 10 %iger aqu. Natronlauge auf pH 4 gestellt. Anschließend gibt man 4,56 g (12,6 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 4 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange, bis das Eluat eine Leitfähigkeit von 10 µS erreicht hat. Dann wird der Inhalt der Ultrafiltrationszelle gefriergetrocknet.
Ausbeute: 16,2 g (94 % d. Th.) eines farblosen, amorphen Pulvers
Wassergehalt: 10,1 %

| Analyse (berechnet auf wasserfreie Substanz): | | | | | |
|---|---|---|---|---|---|
| ber. | C 36,87 | H 4,62 | N 9,56 | Gd 22,99 | Na 3,36 |
| gef. | C 36,65 | H 4,78 | N 9,38 | Gd 22,75 | Na 3,10 |

Relaxivity R₁ (1 • mmol⁻¹ s ⁻¹) in Plasma (20 MHz, 40° C, 0,47 Tesla) : 15,9

### Beispiel 12

### 1,4,7-Tris{N-hexanoyl-2,6-bis[amino-3,6,9-tris(carboxylatomethyl)-3,6,9-triazaundecan-1-oyl-11-säure, Gd-Komplex, Mononatriumsalz]}-10-(N-hexadecanoyl)-1,4,7,10-tetraazacyclododecan

Zu 14 g (10,93 mmol) der Titelverbindung aus Beispiel 11c und 79,1 g (1000 mmol) Pyridin in 400 ml Dimethylformamid gibt man 41,5 g (109,3 mmol) DTPA-monoanhydrid-ethylester und 3 g (24,6 mmol) 4-(Dimethylamino)-pyridin. Man rührt 24 Stunden bei 50°C. Es wird im Vakuum zur Trockne eingedampft, der Rückstand in 400 ml Wasser aufgenommen und stellt mit 3N aqu. Natriumhydroxid auf pH 13. Man rührt 6 Stunden bei Raumtemperatur. Man stellt mit 10 %iger aqu. Salzsäure auf pH 4 und gibt 21,94 g (65,6 mmol) Gadoliniumacetat zu. Anschließend rührt man 1 Stunde bei 60°C. Man läßt auf Raumtemperatur abkühlen, stellt mit 2N aqu. Natronlauge auf pH 7,2 und dialysiert die Lösung in einer Ultrafiltrationszelle (Amicon® YM-1) (6 Durch-läufe). Der Inhalt der Ultrafiltrationszelle wird gefriergetrocknet.
Ausbeute: 40,8 g (91 % d. Th.)
Wassergehalt: 11,3 %

| Elementaranalyse (berechnet auf wasserfreie Substanz): | | | | | |
|---|---|---|---|---|---|
| ber. | C 36,87 | H 4,62 | N 9,56 | Gd 22,99 | Na 3,36 |
| gef. | C 36,67 | H 4,74 | N 9,48 | Gd 22,75 | Na 3,12 |

### Beispiel 13

### a) 1,4,7-Tris{N-hexanoyl-2,6-bis<amino-[1,4,7-tris(N-t.butyloxycarbonylmethyl)-10-[N-(1-methyl-3-aza-2,5-dioxo-pentan-1,5-diyl)]-1,4,7,10-tetraazacyclododecan>]}-10-(N-hexadecanoyl)-1,4,7,10-tetraazacyclododecan

Zu 81,62 g (109,3 mmol) Gly-Methyl-DOTA-tri-tbutylester (Natriumbromid-Komplex), gelöst in 500 ml Dimethylformamid, gibt man bei 0°C 23,02 g (200 mmol) N-Hydroxysuccinimid und 30,9 g (150 mmol) Dicyclohexylcarbodiimid und rührt eine Stunde bei dieser Temperatur. Anschließend rührt man 3 Stunden bei Raumtemperatur. Zu dieser Lösung gibt man 14 g (10,93 mmol) der Titelverbindung aus Beispiel 11c und 30,4 g (300 mmol) Triethylamin. Man rührt 24 Stunden bei Raumtemperatur. Die Lösung wird zur Trockne eingedampft und der Rückstand in 600 ml Dichlormethan aufgenommen. Man filtriert vom Dicyclohexylharnstoff ab und wäscht das Filtrat 2 mal mit 300 ml 5 %iger aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel: Dichlormethan/Methanol = 25 : 1). Ausbeute: 37,3 g (75 % d. Th.) eines farblosen, zähen Öls

| Elementaranalyse: | | | |
|---|---|---|---|
| ber. | C 60,19 | H 9,22 | N 12,31 |
| gef. | C 60,05 | H 9,41 | N 12,18 |

### b) 1,4,7-Tris{N-hexanoyl-2,6-bis<amino-[1,4,7-tris(N-carboxylatomethyl)-10-[N-(1-methyl-3-aza-2,5-dioxo-pentan-1,5-diyl)]-1,4,7,10-tetraazacyclododecan, Gd-Komplex]>}-10-(N-hexadecanoyl)-1,4,7,10-tetraazacyclododecan

19,11 g (4,2 mmol) der Titelverbindung aus Beispiel 13a werden in 300 ml Trifluoressigsäure gelöst und 12 Stunden bei Raumtemperatur gerührt. Man dampft zur Trockne ein. Der Rückstand wird in 150 ml Wasser gelöst und mit 5 %iger aqu. Natronlauge auf pH 3,3 eingestellt. Anschließend gibt man 4,56 g (12,6 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 17,82 g (95 % d. Th.) eines farblosen, amorphen Feststoffes
Wassergehalt: 9,6 %

| Elementaranalyse (berechnet auf wasserfreie Substanz): | | | | |
|---|---|---|---|---|
| ber. | C 41,96 | H 5,73 | N 12,55 | Gd 21,13 |
| gef. | C 41,85 | H 5,86 | N 12,45 | Gd 21,02 |

### Beispiel 14

### a) 3,5-Bis[2-(benzyloxycarbonylamino)-acetylamino]benzoesäure

Zu 30 g (197,2 mmol) 3,5-Diaminobenzoesäure und 59,85 g (591,5 mmol) Triethylamin in 400 ml Dimethylfonnamid gibt man 122,5 g (400 mmol) N-(Benzyloxycarbonyl)-Glycin-N-Hydroxysuccinimidester und rührt 24 Stunden bei Raumtemperatur. Man dampft im Vakuum zur Trockne ein, löst den Rückstand in 1000 ml Dichlormethan und extrahiert 2 mal mit je 5 %iger aqu. Salzsäure. Die organische Phase wird über Magnesiumsulfat getrocknet und zur Trockne eingedampft. Der Rückstand wird aus Diiopsopylether umkristallisiert.
Ausbeute: 95,92 g (91 % d. Th.) eines kristallinen Feststoffes

| Elementaranalyse : | | | |
|---|---|---|---|
| ber. | C 60,67 | H 4,90 | N 10,48 |
| gef. | C 60,48 | H 5,10 | N 10,33 |

### b) 1,4,7-Tris{N-3,5-bis[(2-benzyloxycarbonylamino)-acetylamino]-benzoyl}-1,4,7,10-tetraazacyclododecan

90 g (168,4 mmol) der Titelverbindung aus Beispiel 14a und 8,79 g (51 mmol) 1,4,7,10-Tetraazacyclododecan (= Cyclen) werden in 400 ml Dimethylformamid gelöst und bei 0°C werden 74,2 g (300 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Essigsäureethylester/Ethanol = 20 : 1)
Ausbeute: 50,93 g (58 % d. Th.) eines farblosen Feststoffs

| Elementaranalyse: | | | |
|---|---|---|---|
| ber. | C 62,08 | H 5,39 | N 13,02 |
| gef. | C 61,91 | H 5,51 | N 12,85 |

### c) 1,4,7-Tris{N-3,5-bis[(2-benzyloxycarbonylamino)-acetylamino]-benzoyl}-10-(Ntetradecanoyl)-1,4,7,10-tetraazacyclododecan

16,08 g (9,34 mmol) der Titelverbindung aus Beispiel 14b und 3,81 g (16,68 mmol) Tetradecansäure werden in 150 ml Dimethylformamid gelöst, und bei 0°C werden 7,42 g (30 mmol) 2-Ethoxy-1-ethoxycarbonyl-1,2-dihydrochinolin zugegeben. Man rührt 24 Stunden bei Raumtemperatur.
Es wird im Vakuum zur Trockne eingedampft und der Rückstand an Kieselgel chromatographiert (Laufmittel: Essigsäureethylester/Ethanol = 20 : 1)
Ausbeute: 16,6 g (92 % d. Th.) eines farblosen, zähen Öls

| Analyse: | | | |
|---|---|---|---|
| ber. | C 64,03 | H 6,16 | N 11,60 |
| gef. | C 63,87 | H 6,28 | N 11,43 |

### d) 1,4,7-Tris{N-3,5-bis[(2-amino)-acetylamino]-benzoyl}-10-(N-tetradecanoyl)-1,4,7,10-tetraazacyclododecan, Hexahydrobromid

15 g (7,76 mmol) der Titelverbindung aus Beispiel 14c werden in 70 ml Essigsäure gelöst und zu einer 60°C heißen Lösung aus 200 ml Bromwasserstoff in Eisessig (33 %ig) getropft. Man rührt 1 Stunde bei 60°C. Es wird auf 0°C abgekühlt und unter Rühren 1000 ml Diethylether langsam zugetropft. Man filtriert den ausgefallenen Niederschlag ab, wäscht 2 mal mit 100 ml Ether nach und trocknet im Vakuumofen (60°C).
Ausbeute: 12,26 g (98 d. Th.) cremfarbener, kristalliner Feststoff

| Elementaranalyse: | | | | |
|---|---|---|---|---|
| ber. | C 40,96 | H 5,50 | N 13,90 | Br 29,73 |
| gef. | C 40,85 | H 5,71 | N 13,72 | Br 29,41 |

### e) 1,4,7-Tris{N-3,5-bis<[1,4,7-tris(N-t.butyloxycarbonylaminomethyl)-1,4,7,10-tetraazacyclododecan-10-(N-4,7-diaza-3,6,9-trioxa-nonan-2,9-diyl)]amino>benzoyl}-10-(N-tetradecanoyl)-1,4,7,10-tetraazacyclododecan

Zu 81,62 g (109,3 mmol) Gly-Methyl-DOTA-tri-t.butylester (Natriumbromid-Komplex), gelöst in 500 ml Dimethylformamid, gibt man bei 0°C 23,02 g (200 mmol) N-Hydroxysuccinimid und 30,9 g (150 mmol) Dicyclohexylcarbodiinid und rührt 1 Stunde bei dieser Temperatur. Anschließend rührt man 3 Stunden bei Raumtemperatur. Zu dieser Lösung gibt man 17,63 g (10,93 mmol) der Titelverbindung aus Beispiel 14d und 30,4 g (300 mmol) Triethylamin Man rührt 24 Stunden bei Raumtemperatur. Die Lösung wird zur Trockne eingedampft und der Rückstand in 600 ml Dichlormethan aufgenommen. Man filtriert vom Dicyclohexylharnstolf ab und wäscht das Filtrat 2 mal mit 300 ml 5 %iger aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel = Dichlormethan/Methanol = 25 : 1). Ausbeute: 40,02 g (75 % d. Th.) eines farblosen Feststoffs

| Elementaranalyse: | | | |
|---|---|---|---|
| ber. | C 59,29 | H 8,51 | N 13,20 |
| gef. | C 59,05 | H 8,75 | N 13,01 |

### f) 1,4,7-Tris{N-3,5-bis<[1,4,7-tris-(N-carboxylatomethyl)-1,4,7,10-tetraazacyclododecan-10-(N-4,7-diaza-3,6,9-trioxa-nonan-2,9-diyl)]-amino>-benzoyl, Gd-Komplex}-10-(N-tetradecanoyl)-1,4,7,10-tetraazacyclododecan

20,51 g (4,2 mmol) der Titelverbindung aus Beispiel 14e werden in 300 ml Trifluoressigsäure gelöst und 12 Stunden bei Raumtemperatur gerührt. Man dampft zur Trockne ein. Der Rückstand wird in 150 ml Wasser gelöst und mit 5 %iger aqu. Natronlauge auf pH 3,3 eingestellt. Anschließend gibt man 4,56 g (12,6 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 18,94 g (94 % d. Th.) eines farblosen, amorphen Feststoffes
Wassergehalt: 10,1 %

| Elementaranalyse (berechnet auf wasserfreie Substanz): | | | | |
|---|---|---|---|---|
| ber. | C 42,31 | H 5,25 | N 13,43 | Gd 19,67 |
| gef. | C 42,17 | H 5,41 | N 13,28 | Gd 19,45 |

### Beispiel 15

### a) 1,4,7-Tris[N-(2-benzoyloxycarbonylamino)-acetyl]-1,4,7,10-tetraazacyclododecan

Zu 8,79 g (51 mmol) 1,4,7,10- Tetraazacyclododecan (= Cyclen) in 400 ml Toluol gibt man 47,5 g (155 mmol) N-(Benzyloxycarbonyl)-Glycin-N-Hydroxysuccininidester und 25,3 g (250 mmol) Triethylamin und erhitzt 12 Stunden unter Rückfluß. Man dampft im Vakuum zur Trockne ein, nimmt den Rückstand in 500 ml Dichlormethan auf und wäscht die organische Phase 2 mal mit 5 %iger aqu. Natriumcarbonat-Lösung. Man trocknet die organische Phase über Magnesiumsulfat und engt im Vakuum zur Trockne ein. Der Rückstand wird an Kieselgel chromatographiert (Laufmittel: Essigsäureethylester/Ethanol = 20 : 1).
Ausbeute: 20,92 g (55 % d. Th.) eines farblosen Schaums.

| Elementaranalyse: | | | |
|---|---|---|---|
| ber. | C 61,20 | H6,35 | N 13,15 |
| gef. | C 61,03 | H 6,48 | N 13,02 |

### b) 1,4,7-Tris[N-(2-amino)-acetyl]-1,4,7,10-tetraazacyclododecan, Tetrahydrobromid

20 g (26,82 mmol) der Titelverbindung aus Beispiel 15a werden in 100 ml Essigsäure gelöst und zu einer 60°C heißen Lösung aus 350 ml Bromwasserstoff in Eisessig (33 %ig) getropft. Man rührt I Stunde bei 60°C. Es wird auf 0°C abgekühlt und unter Rühren 1500 ml Diethylether langsam zugetropft. Man filtriert den ausgefallenen Niederschlag ab, wäscht 2 mal mit 200 ml Ether nach und trocknet im Vakuumofen (60°C).
Ausbeute: 17,35 g (97 d. Th.) cremfarbener, kristalliner Feststoff

| Elementaranalyse: | | | | |
|---|---|---|---|---|
| ber. | C 25,21 | H 4,99 | N 14,70 | Br 47,91 |
| gef. | C 25,03 | H 5,14 | N 14,53 | Br 47,65 |

### c) 1,4,7-Tris{1,4,7-tris(N-t.butyloxycarbonylmethyl)-10-[N-(4,7-diaza-3,6,9-trioxononan-2,9-diyl)]-1,4,7,10-tetraazacyclododecan}-1,4,7,10-tetraazacyclododecan

Zu 67,16 g (89,94 mmol) Gly-Methyl-DOTA-tri-t.butylester (Natriumbromid-Komplex), gelöst in 500 ml Dimethylformamid, gibt man bei 0°C 20,72 g (180 mmol) N-Hydroxysuccinimid und 41,3 g (200 mmol) Dicyclohexylcarbodiinid und rührt 1 Stunde bei dieser Temperatur. Anschließend rührt man 3 Stunden bei Raumtemperatur. Zu dieser Lösung gibt man 15 g (22,49 mmol) der Titelverbindung aus Beispiel 15b und 30,4 g (300 mmol) Triethylamin. Man rührt 24 Stunden bei Raumtemperatur. Die Lösung wird zur Trockne eingedampft und der Rückstand in 500 ml Dichlormethan aufgenommen. Man filtriert vom Dicyclohexylharnstoff ab und wäscht das Filtrat 2 mal mit 300 ml 5 %iger aqu. Natriumcarbonat-Lösung. Die organische Phase wird über Magnesiumsulfat getrocknet und im Vakuum zur Trockne eingedampft; der Rückstand wird an Kieselgel chroinatographiert (Laufmittel = Dichlormethan/Methanol = 20 : 1). Ausbeute: 38,96 g (78 % d. Th.) eines farblosen, zähen Ols

| Elementaranalyse: | | | |
|---|---|---|---|
| ber. | C 57,87 | H 8,80 | N 13,88 |
| gef. | C 57,65 | H 8,97 | N 13,64 |

### d) 1,4,7-Tris{1,4,7-tris(N-t.butyloxycarbonylmethyl)-10-[N-(4,7-diaza-3,6,9-trioxononan-2,9-diyl)]-1,4,7,10-tetraazacyclododecan}-10-(N-hexadecanoyl)-1,4,7,10-tetraazacyclododecan

20,74 g (9,34 mmol) der Titelverbindung aus Beispiel 15c und 4,28 g (16,68 mmol) Palmitinsäure werden in 150 ml Dimethylformamid gelöst, und bei 0°C werden 7,42 g (30 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/Methanol = 20 : 1)
Ausbeute: 21,13 g (92 % d. Th.) eines farblosen, zähen Öls

| Analyse: | | | |
|---|---|---|---|
| ber. | C 60,07 | H 9,18 | N 12,53 |
| gef. | C 60,10 | H 9,35 | N 12,37 |

### e) 1,4,7-Tris{1,4,7-tris(N-carboxylatomethyl)-10-[N-(4,7-diaza-3,6,9-trioxo-nonan-2,9-diyl)]-1,4,7,10-tetraazacyclododecan, Gd-Komplex}-10-(N-hexadecanoyl)-1,4,7,10-tetraazacyclododecan

10,33 g (4,2 mmol) der Titelverbindung aus Beispiel 15d werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 9,44 g (93 % d. Th.) eines farblosen, amorphen Feststoffes
Wassergehalt: 10.2 %

| Elementaranalyse (berechnet auf wasserfreie Substanz): | | | | |
|---|---|---|---|---|
| ber. | C 43,23 | H 5,96 | N 12,75 | Gd 19,52 |
| gef. | C 43,36 | H 6,18 | N 12,49 | Gd 19,28 |

Relaxivity R₁ (1 • mmol⁻¹ s ⁻¹) in Plasma (20 MHz, 40° C, 047 Tesla) : 27,7

### Beispiel 16

### a) 1,4,7-Tris{1,4,7-tris(N-t.butyloxycarbonylmethyl)-10-[N-(4,7-diaza-3,6,9-trioxononan-2,9-diyl)]-1,4,7,10-tetraazacyclododecan}-10-(N-pentadecanoyl)-1,4,7,10-tetraazacyclododecan

10,37 g (4,67 mmol) der Titelverbindung aus Beispiel 15c und 2,26 g (9,33 mmol) Pentadecansäure werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/Methanol = 20 : 1)
Ausbeute: 10,73 g (94 % d. Th.) eines farblosen, zähen Öls

| Analyse: | | | |
|---|---|---|---|
| ber. | C 59,93 | H 9,15 | N 12,60 |
| gef. | C 59,71 | H 9,34 | N 12,43 |

### b) 1,4,7-Tris{1,4,7-tris(N-carboxylatomethyl)-10-[N-(4,7-diaza-3,6,9-trioxo-nonan-2,9-diyl)]-1,4,7,10-tetraazacyclododecan, Gd-Komplex}-10-(N-pentadecanoyl)-1,4,7,10-tetraazacyclododecan

10,27 g (4,2 mmol) der Titelverbindung aus Beispiel 16a werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 9,69 g (96 % d. Th.) eines farblosen, amorphen Feststoffes
Wassergehalt: 11,4 %

| Elementaranalyse (berechnet auf wasserfreie Substanz): | | | | |
|---|---|---|---|---|
| ber. | C 42,99 | H 5,91 | N 12,82 | Gd 19,63 |
| gef. | C 42,75 | H 6,15 | N 12,64 | Gd 19,47 |

### Beispiel 17

### a) 3-Oxa-hexadecansäure-t.butylester

Zu einer Mischung aus 4,32 g (21,55 mmol) Tridecan-1-ol und 0,73 g (2,15 mmol) Tetrabutylammoniumhydrogensulfat in 100 ml 60 %iger Kalilauge/50 ml Toluol tropft man unter starkem Rühren bei 0°C 10,51 g (53,9 mmol) Bromessigsäure-tert.-butylester zu. Man rührt 1 Stunde bei 0°C. Es werden 200 ml Toluol zugegeben, die wässrige Phase abgetrennt und 2 mal mit je 50 ml Toluol extrahiert. Die vereinigten organische Phasen werden über Magnesiumsulfat getrocknet und im Vakuum eingedampft. Der Rückstand wird an Kieselgel chromatographiert (Laufmittel: Hexan/Dichlormethan/Aceton = 20 : 10 : 1).
Ausbeute: 5,29 g (78 % d. Th.) eines farblosen, viskosen Öls

| Elementaranalyse: | | |
|---|---|---|
| ber. | C 72,56 | H 12,18 |
| gef. | C 72,34 | H 12,39 |

### b) 3-Oxa-hexadecansäure

30 g (95,39 mmol) der Titelverbindung aus Beispiel 17a werden in 300 ml Trifluoressigsäure gelöst und 6 Stunden bei Raumtemperatur gerührt. Man dampft zur Trockne ein. Der Rückstand wird aus Hexan/Ether kristallisiert.
Ausbeute: 22,92 g (93 % d. Th.) eines farblosen Feststoffs.

| Elementaranalyse: | | |
|---|---|---|
| ber. | C 69,72 | H 11,70 |
| gef. | C 69,57 | H 11,84 |

### c) 1,4,7-Tris{1,4,7-tris(N-t.butyloxycarbonylmethyl)-10-[N-(4,7-diaza-3,6,9-trioxononan-2,9-diyl)]-1,4,7,10-tetraazacyclododecan}-10-(N-3-oxa-hexadecanoyl)-1,4,7,10-tetraazacyclododecan

10,37 g (4,67 mmol) der Titelverbindung aus Beispiel 15c und 2,41 g (9,33 mmol) der Titelverbindung aus Beispiel 17b werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/Methanol = 20 : 1)
Ausbeute: 10,57 g (92 % d. Th.) eines farblosen, zähen Öls

| Analyse: | | | |
|---|---|---|---|
| ber. | C 59,94 | H 9,09 | N 12,52 |
| gef. | C 59,31 | H 9,30 | N 12,40 |

### d) 1,4,7-Tris{1,4,7-tris(N-carboxylatomethyl)-10-[N-(4,7-diaza-3,6,9-trioxo-nonan-2,9-diyl)]-1,4,7,10-tetraazacyclododecan, Gd-Komplex}-10-(N-3-oxahexadecanoyl)-1,4,7,10-tetraazacyclododecan

10,34 g (4,2 mmol) der Titelverbindung aus Beispiel 17c werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 9,75 g (96 % d. Th.) eines farblosen, amorphen Feststoffes
Wassergehalt: 11,0%

| Elementaranalyse (berechnet auf wasserfreie Substanz): | | | | |
|---|---|---|---|---|
| ber. | C 42,70 | H 5,88 | N 12,74 | Gd 19,50 |
| gef. | C 42,54 | H 5,99 | N 12,61 | Gd 19,32 |

### Beispiel 18

### a) 1,4,7-Tris{1,4,7-tris((N-tbutyloxycarbonylmethyl)-10-[N-(4,7-diaza-3,6,9-trioxononan-2,9-diyl)]-1,4,7,10-tetraazacyclododecan}-10-(N-tetradecanoyl)-1,4,7,10-tetraazacyclododecan

10,37 g (4,67 mmol) der Titelverbindung aus Beispiel 15c und 2,13 g (9,33 mmol) Tetradecansäure werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/Methanol = 20:1)
Ausbeute: 10,79 g (95 % d. Th.) eines farblosen, zähen Öls

| Analyse: | | | |
|---|---|---|---|
| ber. | C 59,78 | 119.12 | N 12,67 |
| gef. | C 59,65 | H 9,30 | N 12,44 |

### b) 1,4,7-Tris{1,4,7-tris(N-carboxylatomethyl)-10-[N-(4,7-diaza-3,6,9-trioxo-nonan-2,9-diyl)]-1,4,7,10-tetraazacyclododecan, Gd-Komplex}-10-(N-tetradecanoyl)-1,4,7,10-tetraazacyclododecan

10,21 g (4,2 mmol) der Titelverbindung aus Beispiel 18a werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe.

Der Inhalt der Ultrafiltrationszelle wird gefriergetrocknet.
Ausbeute: 9,73 g (97 % d. Th.) eines farblosen, amorphen Feststoffes
Wassergehalt: 9,6 %

| Elementaranalyse (berechnet auf wasserfreie Substanz): | | | | |
|---|---|---|---|---|
| ber. | C 42,74 | H 5,86 | N 12,90 | Gd 19,75 |
| gef. | C 42,55 | H 5,99 | N 12,68 | Gd 19,54 |

Relaxivity R₁ (1 • mmol⁻¹ s⁻¹) in Plasma (20 MHz, 40° C, 0,47 Tesla) : 24,3

### Beispiel 19

### a) 2-Amino-heptadecansäure

In einer Druckflasche gibt man eine kalt gesättigte Lösung von 29,42 g (0,55 mol) Ammoniumchlorid, 100 ml konz. Ammoniaklösung und eine Lösung von 26.95 g (0,55 mol) Natriumcyanid in 50 ml Wasser. Dann wird in Eiswasser gekühlt und 48,1 g (0,2 mol) Hexadecanaldehyd gelöst, in 100 ml Isopropanol. werden zugegeben. Man verschließt die Druckflasche und rührt über Nacht bei 80 °C. Man engt im Vakuum zur Trockne ein, nimmt den Rückstand und in 300 ml Satzsäure 100 ml Ethanol auf und kocht 8 Stunden unter Rückfluß. Man dampft im Vakuum zur Trockne ein, extrahiert den Rückstand 3 mal mit je 200 ml Methanol und vereinigt die Filtrate. Man engt zur Trockne ein und chromatographiert den Rückstand an RP-18 (Laufmittel: gradiert aus Acetonitril/Tetrahydrofuran/Wasser).
Ausbeute: 21,7 g (38 % d. Th.) eines farblosen Feststoffs

| Elementaranalyse: | | | |
|---|---|---|---|
| ber. | C 71,53 | H 12,36 | N 4,91 |
| gef. | C 71,30 | H 12,51 | N 4,81 |

### b) 2-Benzyloxycarbonylamino-heptadecansäure

Zu 10 g (35,03 mmol) der Titelverbindung aus Beispiel 19a und 8,86 g (87,57 mmol) Triethylamin in 200 ml Tetrahydrofuran tropft man bei 0°C 6,82 g (40 mmol) Chlorameisensäurebenzylester (Z-Cl). Man rührt 5 Stunden bei 0°C. Es wird im Vakuum zur Trockne eingedampft, der Rückstand in 300 ml Dichlormethan aufgenommen und 2 mal mit je 200 ml 5 %iger aqu. Salzsäure gewaschen. Die organische Phase wird über Magnesiumsulfat getrocknet und zur Trockne eingedampft. Der Rückstand wird aus Diosopropylether umkristallisiert.
Ausbeute: 11,02 g (75 % d. Th.) eines farblosen, kristallinen Feststoffes

| Analyse: | | | |
|---|---|---|---|
| ber. | C 71,56 | H 9,85 | N 3,34 |
| gef. | C 71,43 | H 10,04 | N 3,25 |

### c) 1,4,7-Tris{1,4,7-tris(N-t.butyloxycarbonylmethyl)-10-[N-(4,7-diaza-3,6,9-trioxononan-2,9-diyl)]-1,4,7,10-tetraazacyclododecan}-10-[N-(2-benzyloxycarbonylamino)-heptadecanoyl]-1,4,7,10-tetraazacyclododecan

10,37 g (4,67 mmol) der Titelverbindung aus Beispiel 15c und 3,91 g(9,33 mmol) der Titelverbindung aus Beispiel 19b werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/Methanol = 20 : 1)
Ausbeute: 11,63 g (95 % d. Th.) eines farblosen, zähen Öls

| Analyse: | | | |
|---|---|---|---|
| ber. | C 60,46 | H 8,96 | N 12,28 |
| gef. | C 60,25 | H 8,92 | N 12,05 |

### d) 1,4,7-Tris{1,4,7-tris(N-t.butyloxycarbonylmethyl)-10-[N-(4,7-diaza-3,6,9-trioxononan-2,9-diyl)]-1,4,7,10-tetraazacyclododecan}-10-(N-2-aminoheptadecanoyl)-1,4,7,10-tetraazacyclododecan

11,5 g (4.39 mmol) der Titelverbindung aus Beispiel 19c werden in 300 ml 2-Propanol gelöst und 3 g Palladiumkatalysator (10 % Pd/C) zugegeben. Man hydriert 6 Stunden bei Raumtemperatur. Der Katalysator wird abfiltriert und das Filtrat im Vakuum zur Trockne eingedampft.
Ausbeute: 10,91 g (quantitativ) eines farblosen, zähen Öls

| Elementaranalyse: | | | |
|---|---|---|---|
| ber. | C 59,85 | H 9,19 | N 12,95 |
| gef. | C 59,75 | H 9,31 | N 12,74 |

### e) 1,4,7-Tris{1,4,7-tris(N-t.butyloxycarbonylmethyl)-10-[N-{4,7-diaza-3,6,9-trioxononan-2,9-diyl)]-1,4,7,10-tetraazacyclododecan}- 10-{N-heptadecanoyl-2-amino-<1,4,7-tris(N-t.butyloxycarbonylmethyl)-10-[N-(1-methyl-3-aza-2,5-dioxo-pentan-1,5-diyl)]-1,4,7,10-tetraazacyclododecan>}-1,4,7,10-tetraazacyclododecan

Zu 5,97 g (8 mmol) Gly-Methyl-DOTA-tri-t.butylester (Natriumbromid-Komplex), gelöst in 100 ml Dimethylformamid, gibt man bei 0°C 1,84 g (16 mmol) N-Hydroxysuccinimid und 2,48 g (12 mmol) Dicyclohexylcarbodiimid und rührt 1 Stunde bei dieser Temperatur. Anschließend rührt man 3 Stunden bei Raumtemperatur. Zu dieser Lösung gibt man 10 g (4,02 mmol) der Titelverbindung aus Beispiel 19d und 2 g (20 mmol) Triethylamin. Man rührt 24 Stunden bei Raumtemperatur. Die Lösung wird zur Trockne eingedampft und der Rückstand in 300ml Dichlormethan aufgenommen. Man filtriert vom Dicyclohexylharnstoff ab und wäscht das Filtrat 2 mal mit 300 ml 5 %iger aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel = Dichlormethan/Methanol = 20 : 1).
Ausbeute: 12,29 g (94 % d. Th.) eines farblosen, zähen Öls

| Elementaranalyse: | | | |
|---|---|---|---|
| ber. | C 59,78 | H 9,13 | N 12,59 |
| gef. | C 59,61 | H 9,31 | N 12,38 |

### f) 1,4,7-Tris{1,4,7-iris(N-carboxylato)-10-[N-(4,7-diaza-3,6,9-trioxo-nonan-2,9-diyl)]-1,4,7,10-tetraazacyclododecan, Gd-Komplex}-10-{N-heptadecanoyl-2-amino-<1,4,7-tris(N-carboxylatomethyl-10-[N-(1-methyl-3-aza-2,5-dioxo-pentan-1,5-diyl)]-1,4,7,10-tetraazacyclododecan, Gd-Komplex>}-1,4,7,10-tetraazacyclododecan

10 g (3,21 mmol) der Titelverbindung aus Beispiel 19e werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschließend gibt man 2,33 g (6,42 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 9,42 g (96 % d. Th.) eines farblosen, amorphen Feststoffes
Wassergehalt: 9,2 %

| Elementaranalyse (berechnet auf wasserfreie Substanz): | | | | |
|---|---|---|---|---|
| ber. | C 42,03 | H 5,74 | N 12,83 | Gd 20,57 |
| gef. | C 42,19 | H 5,61 | N 12,64 | Gd 20,38 |

### Beispiel 20

### a) 1,4,7-Tris[N-(2-benzyloxycarbonylamino)-acetyl]-10-[N-(2-benzyloxycarbonylamino)-heptadecanoyl]-1,4,7,10-tetraazacyclododecan

20 g (26,82 mmol) der Titelverbindung aus Beispiel 15a und 16,78 g (40 mmol) der Titelverbindung aus Beispiel 19b werden in 150 ml Dimethylformamid gelöst, und bei 0°C werden 17,3 g (70 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromalographiert (Laufmittel: Methylenchlorid/2-Propanol = 25 : 1)
Ausbeute: 26,16 g (85 % d. Th.) eines farblosen, zähen Öls

| Analyse: | | | |
|---|---|---|---|
| ber. | C 65,95 | H 7,55 | N 9,77 |
| gef. | C 65,78 | H 7,73 | N 9,52 |

### b) 1,4,7-Tris[N-(2-amino)-acetyl]-10-[N-2-aminoheptadecanoyl]-1,4,7,10-tetraazacyclododecan, Tetrahydrobromid

20 g (17,43 mmol) der Titelverbindung aus Beispiel 20a werden in 100 ml Essigsäure gelöst und zu einer 60°C heißen Lösung aus 300 ml Bromwasserstoff in Eisessig (33 %ig) getropft. Man rührt 1 Stunde bei 60°C. Es wird auf 0°C abgekühlt und unter Rühren 2000 ml Diethylether langsam zugetropft. Man filtriert den ausgefallenen Niederschlag ab, wäscht 2 mal mit 200 ml Ether nach und trocknet im Vakuumofen (60°C).
Ausbeute: 15,8 g (97 d. Th.) farbloser, kristalliner Feststoff

| Elementaranalyse: | | | | |
|---|---|---|---|---|
| ber. | C 39,84 | H 7,12 | N 11,99 | Br 34,20 |
| gef. | C 39,65 | H 7.29 | N 11,84 | Br 34,06 |

### c) 1,4,7-Tris{N-[3,9-bis(N-t.butyloxycarbonylmethyl)-6-(N-3-aza-1,4-dioxo-pentan-1,5-diyl)-3,6,9-triazaundecandisäure-di-t.butylester)}-10-<N-heptadecanoyl-2-amino-[3,9-bis(N-t.butyloxycarbonylmethyl)-6-(N-3-aza-1,4-dioxo-pentan-1,5-diyl)-3,6,9-triazaundecandisäure-di-t.butylester]>-1,4,7,10-tetraazacyclododecan

Zu 24,79 g (40,2 mmol) sym.-DTPA-tetra-t.butylester gelöst in 300 ml Dimethylformamid gibt man bei 0°C 9,21 g (80 mmol) N-Hydroxysuccinimid und 12,38 g (60 mmol) Dicyclohexylcarbodiinid und rührt 1 Stunde bei dieser Temperatur. Anschließend rührt man 3 Stunden bei Raumtemperatur. Zu dieser Lösung gibt man 3,76 g (4,02 mmol) der Titelverbindung aus Beispiel 20b und 5,06 g (50 mmol) Triethylamin. Man rührt 24 Stunden bei Raumtemperatur. Die Lösung wird zur Trockne eingedampft und der Rückstand in 300 ml Dichlormethan aufgenommen. Man filtriert vom Dicyclohexylharnstoff ab und wäscht das Filtrat 2 mal mit 200 ml 5 %leer aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel = Dichlormethan/Ethanol = 30 : 1).
Ausbeute: 10,77 g (89 % d. Th.) eines farblosen, zähen Öls

| Elementaranalyse: | | | |
|---|---|---|---|
| ber. | C 60,26 | H 9,18 | N 9,31 |
| gef. | C 60,05 | H 9,37 | N 9,15 |

### d) 1,4,7-Tris{N-[3,9-bis(N-carboxylatomethyl)-6-(N-3-aza-1,4-dioxo-pentan-1,5-diyl)-3,6,9-triazaundecan-1-carboxylato-11-säure, Gd-Komplex, Mononatriumsalz]}-10-<N-heptadecanoyl-2-amino-[3,9-bis(N-carboxylatomethyl)-6-(N-3-aza-1,4-dioxopentan-1,5-diyl)-3,6,9-triazaundecan-1-carboxylato-11-säure, Gd-Komplex, Mononatriumsalz]>-1,4,7,10-tetraazacyclododecan

9,66 g (3,21 mmol) der Titelverbindung aus Beispiel 20c werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4 gestellt. Anschließend gibt man 2,33 g (6,42 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 2 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange, bis das Eluat eine Leitfähigkeit von 10 µS erreicht hat. Dann wird der Inhalt der Ultrafiltrationszelle gefriergetrocknet.
Ausbeute. 8,68 g (96 % d. Th.) eines farblosen, amorphen Pulvers
Wassergehalt: 10,5 %

| Analyse (berechnet auf wasserfreie Substanz): | | | | | |
|---|---|---|---|---|---|
| ber. | C 37,09 | H 4,65 | N 9,94 | Gd 22,33 | Na 3,26 |
| gef. | C 36,91 | H 4,82 | N 9,75 | Gd 22,18 | Na 3,01 |

### Beispiel 21

### a) 1,4,7-Tris[N-succinoyl-(3-benzyloxycarbonylamino-4-benzylester)]-1,4,7,10-tetraazacyclododecan

34,23 g (95,78 mmol) N-(Benzyloxycarbonyl)-asparaginsäure-benzylester und 5 g (29,02 mmol) 1,4,7,10-Tetraazacyclododecan (= Cyclen) werden in 200 ml Dimethylformamid gelöst, und bei 0°C werden 29,68 g (120 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Chloroform/Methanol = 30 : 1)
Ausbeute: 13,47 g (39 % d. Th.) eines farblosen Feststoffs

| Elementaranalyse: | | | |
|---|---|---|---|
| ber. | C 65,59 | H 6,01 | N 8,24 |
| gef. | C 65,41 | H 6,18 | N 8,13 |

### b) 1,4,7-Tris[N-succinoyl-(3-benzyloxycarbonylamino-4-benzylester)]-10-[Nhexadecanoyl)-1,4,7,10-tetraazacyclododecan

13 g (10,92 mmol) der Titelverbindung aus Beispiel 21 a und 4,10 g (16 mmol) Palmitinsäure werden in 100 ml Dimethylformamid gelöst, und bei 0°C werden 6,18 g (25 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/2-Propanol = 25 : 1)
Ausbeute: 14,53 g (93 % d. Th.) eines farblosen, zähen Öls

| Analyse: | | | |
|---|---|---|---|
| ber. | C 68,00 | H 7,26 | N 6,85 |
| gef. | C 67,85 | H 7,41 | N 6,66 |

### c) 1,4,7-Tris[N-(3-amino)-bernsteinsaure-halbamid]-10-(N-hexadecanoyl)-1,4,7,10-tetraazacyclododecan

14,3 g (10 mmol) der Titelverbindung aus Beispiel 21b werden in 300 ml Methanol und 30 ml Wasser gelöst und 3 g Palladiumkatalysator (10 % Pd/C) zugegeben. Man hydriert 6 Stunden bei Raumtemperatur. Der Katalysator wird abfiltriert und das Filtrat im Vakuum zur Trockne eingedampft.
Ausbeute: 7,55 g (quantitativ) eines farblosen Farbstoffs.

| Elementaranalyse: | | | |
|---|---|---|---|
| ber. | C 57,20 | H 8,67 | N 12,97 |
| gef. | C 57,03 | H 8,78 | N 12,84 |

### d) 1,4,7-Tris{1,4,7-tris(N-t.butyloxycarbonylmethyl)-10-[N-(4,7-diaza-3,6,10-trioxo-8-carboxy-decan-2,10-diyl)]-1,4,7,10-tetraazacyclododecan}-10-(N-hexadecanoyl)-1,4,7,10-tetraazacyclododecan

Zu 55,26 g (74 mmol) Gly-Methyl-DOTA-tri-tbutylester (Natriumbromid-Komplex), gelöst in 300 ml Dimethylformamid. gibt man bei 0°C 11,51 g (100 mmol) N-Hydroxysuccinimid und 16,51 g (80 mmol) Dicyclohexylcarbodiinid und rührt 1 Stunde bei dieser Temperatur. Anschließend rührt man 3 Stunden bei Raumtemperatur. Zu dieser Lösung gibt man 7 g (9,26 mmol) der Titelverbindung aus Beispiel 21c und 10,12 g (100 mmol) Triethylamin. Man rührt 24 Stunden bei Raumtemperatur. Die Lösung wird zur Trockne eingedampft und der Rückstand in 500 ml Dichlormethan aufgenommen. Man filtriert vom Dicyclohexylharnstoff ab und wäscht das Filtrat 2 mal mit 300 ml 5 %iger aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel = Dichlormethan/Methanol = 10 : 1; + 1 % Essigsäure).
Ausbeute: 21,21 g (87 % d. Th.) eines farblosen Feststoffs

| Elementaranalyse: | | | |
|---|---|---|---|
| ber. | C 58,84 | H 8,80 | N 11,70 |
| gef. | C 58,65 | H 8,97 | N 11,53 |

### e) 1,4,7-Tris{1,4,7-tris(N-carboxylatomethyl)-10-[N-(4,7-diaza-3,6,10-trioxo-8-carboxy-decan-2,10-diyl)]-1,4,7,10-tetraazacyclododecan, Gd-Komplex, Mononatriumsalz}-10-(N-hexadecanoyl)-1,4,7,10-tetraazacyclododecan

11,06 g (4,2 mmol) der Titelverbindung aus Beispiel 21d werden in 200 ml Trifluoressigsäure gelöst und I2 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. An-schließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,2 und dialysiert die Lösung in einer Ultrafiltrationszelle (Amicon® YM-1). Der Inhalt der Ultrafiltrationszelle wird gefriergetrocknet.
Ausbeute: 10,5 g (94 % d. Th.) eines farblosen, amorphen Feststoffs
Wassergehalt: 10,5 %

| Analyse (berechnet auf wasserfreie Substanz): | | | | | |
|---|---|---|---|---|---|
| ber. | C 41,99 | H 5,65 | N 11,58 | Gd 17,73 | Na 2,59 |
| gef. | C 41,71 | H 5,81 | N 11,37 | Gd 17,51 | Na 2,32 |

### Beispiel 22

### a) 1,4,7-Tris[N-succinoyl-(3-benzyloxycarbonylamino-4-benzylester)]-10-[N-(3-oxahexadecanoyl)]-1,4,7,10-tetraazacyclododecan

13 g (10,92 mmol) der Titelverbindung aus Beispiel 21a und 4,13 g (16 mmol) der Titelverbindung aus Beispiel 17b werden in 100 ml Dimethylformamid gelöst, und bei 0°C werden 6,18 g (25 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/2-Propanol = 25 : 1)
Ausbeute: 14,69 g (95 % d. Th.) eines farblosen, zähen Öls

| Analyse: | | | |
|---|---|---|---|
| ber. | C 67,82 | H 7,19 | N 6,92 |
| gef. | C 67,64 | H 7,34 | N 6,71 |

### b) 1,4,7-Tris[N-(3-amino)-bernsteinsäurehalbamid]-10-[N-(3-oxa)-hexadecanoyl)-1,4,7,10-tetraazacyclododecan

14,5 g (10,23 mmol) der Titelverbindung aus Beispiel 22a werden in 300 ml Methanol und 30 ml Wasser gelöst und 3 g Palladiumkatalysator (10 % Pd/C) zugegeben. Man hydriert 6 Stunden bei Raumtemperatur. Der Katalysator wird abfiltriert und das Filtrat im Vakuum zur Trockne eingedampft.
Ausbeute: 7,76 g (quantitativ) eines farblosen Feststoffs

| Elementaranalyse: | | | |
|---|---|---|---|
| ber. | C 55,47 | H 8,38 | N 12,94 |
| gef. | C 55,31 | H 8,49 | N 12,75 |

### c) 1,4,7-Tris{1,4,7-tris (N-t.butyloxycarbonylmethyl)-10-[N-(4,7-diaza-3,6,10-trioxo-8-carboxy-decan-2,10-diyl)]-1,4,7,10-tetraazacyclododecan }-10-[N-(3-oxa)-hexadecanoyl]-1,4,7,10-tetraazacyclododecan

Zu 55,26 g (74 mmol) Gly-Methyl-DOTA-tri-t.butylester (Natriumbromid-Komplex), gelöst in 300 ml Dimethylformamid, gibt man bei 0°C 11,51 g (100 mmol) N-Hydroxysuccinimid und 16,51 g (80 mmol) Dicyclohexylcarbodiinid und rührt 1 Stunde bei dieser Temperatur. Anschließend rührt man 3 Stunden bei Raumtemperatur. Zu dieser Lösung gibt man 7,02 g (9,26 mmol) der Titelverbindung aus Beispiel 22a und 10,12 g (100 mmol) Triethylamin. Man rührt 24 Stunden bei Raumtemperatur. Die Lösung wird zur Trockne eingedampft und der Rückstand in 500 ml Dichlormethan aufgenommen. Man filtriert vom Dicyclohexylharnstoff ab und wäscht das Filtrat 2 mal mit 300 ml 5 %iger aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel: Chloroform/Methanol = 15 : 1). Ausbeute: 22,2 g (91 % d. Th.) eines farblosen Feststoffs

| Elementaranalyse: | | | |
|---|---|---|---|
| ber. | C 58,34 | H 8,72 | N 11,69 |
| gef. | C 58,23 | H 8,89 | N 11,60 |

### d) 1,4,7-Tris{1,4,7-tris (N-carboxylatomethyl)-10-[N-(4,7-diaza-3,6,10-trioxo-8-carboxy-decan-2,10-diyl)]-1,4,7,10-tetraazacyclododecan, Gd-Komplex, Mononatriumsalz}-10-(N-(3-oxa)-hexadecanoyl]-1,4,7,10-tetraazacyclododecan

11,07 g (4,2 mmol) der Titelverbindung aus Beispiel 22c werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,2 und dialysiert die Lösung in einer Ultrafiltrationszelle (Amicon® YM-1). Der Inhalt der Ultrafiltrationszelle wird gefriergetrocknet.
Ausbeute: 10,62 g (95 % d. Th.) eines farblosen, amorphen Feststoffs
Wassergehalt: 7,8 %

| Analyse (berechnet auf wasserfreie Substanz): | | | | | |
|---|---|---|---|---|---|
| ber. | C 41,51 | H 5,57 | N 11,58 | Gd 17,72 | Na 2,59 |
| gef. | C 41,35 | H 5,72 | N 11,39 | Gd 17,52 | Na 2,33 |

### Beispiel 23

### a) 1,4,7-Tris[N-succinoyl-(3-benzyloxycarbonylamino-4-benzylester)]-10-(Ntetradecanoyl)-1,4,7,10-tetraazacyclododecan

13 g (10.92 mmol) der Titelverbindung aus Beispiel 21a und 3,65 g (16 mmol) Tetradecansäure werden in 100 ml Dimethylformamid gelöst, und bei 0°C werden 6,18 g (25 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/2-Propanol = 25 : 1)
Ausbeute: 14,24 g (93 % d. Th.) eines farblosen, zähen Öls

| Analyse: | | | |
|---|---|---|---|
| ber. | C 67,65 | H 7,11 | N 6,99 |
| gef. | C 67,48 | H 7,29 | N 6,78 |

### b) 1,4,7-Tris[N-(3-amino)-bernsteinsäurehalbamid]-10-(N-tetradecanoyl)-1,4,7,10-tetraazacyclododecan

14 g (9,98 mmol) der Titelverbindung aus Beispiel 23a werden in 300 ml Methanol und 30 ml Wasser gelöst und 3 g Palladiumkatälysator (10 % Pd/C) zugegeben. Man hydriert 6 Stunden bei Raumtemperatur. Der Katalysator wird abfiltriert und das Filtrat im Vakuum zur Trockne eingedampft.
Ausbeute: 7,26 g (quantitativ) eines farblosen zähen Öls

| Elementaranalyse: | | | |
|---|---|---|---|
| ber. | C 56,10 | H 8,45 | N 13,47 |
| gef. | C 56,03 | H 8,63 | N 13,30 |

### c) 1,4,7-Tris{1,4,7-tris (N-t.butyloxycarbonylmethyl)-10-[N-(4,7-diaza-3,6,10-trioxo-8-carboxy-decan-2,10-diyl)]-1,4,7,10-tetraazacyclododecan}-10-(N-tetradecanoyl)-1,4,7,10-tetraazacyclododecan

Zu 55,26 g (74 mmol) Gly-Methyl-DOTA-t.butylester (Natriumbromid-Komplex), gelöst in 300 ml Dimethylformamid, gibt man bei 0°C 11,51 g (100 mmol) N-Hydroxysuccinimid und 16,51 g (80 mmol) Dicyclohexylcarbodiinid und rührt 1 Stunde bei dieser Temperatur. Anschließend rührt man 3 Stunden bei Raumtemperatur. Zu dieser Lösung gibt man 6,74 g (9,26 mmol) der Titelverbindung aus Beispiel 23b und 10,12 g (100 mmol) Triethylamin. Man rührt 24 Stunden bei Raumtemperatur. Die Lösung wird zur Trockne eingedampft und der Rückstand in 500 ml Dichlormethan aufgenommen Man filtriert vom Dicyclohexylharnstoff ab und wäscht das Filtrat 2 mal mit 300 ml 5 %iger aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel = Chloroform/Methanol = 15 : 1). Ausbeute: 21,95 g (91 % d. Th.) eines farblosen Feststoffs

| Elementaranalyse: | | | |
|---|---|---|---|
| ber. | C 58,55 | H 8,74 | N 11,83 |
| gef. | C 58,35 | H 8,91 | N 11,66 |

### d) 1,4,7-Tris{1,4,7-tris (N-carboxylatomethyl)-10-[N-(4,7-diaza-3,6,10-trioxo-8-carboxy-decan-2,10-diyl)]-1,4,7,10-tetraazacyclododecan, Gd-Komplex, Mononatriumsalz}-10-(N-tetradecanoyl]-1,4,7,10-tetraazacyclododecan

10,94 g (4,2 mmol) der Titelverbindung aus Beispiel 23c werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschliessend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,2 und dialysiert die Lösung in einer Ultrafiltrationszelle (Amicon® YM-1). Der Inhalt der Ultrafiltrationszelle wird gefriergetrocknet.
Ausbeute: 10,72 g (97 % d. Th.) eines farblosen, amorphen Feststoffs
Wassergehalt: 11,2%

| Elementaranalyse (berechnet auf wasserfreie Substanz): | | | | | |
|---|---|---|---|---|---|
| ber. | C 41,53 | H 5,55 | N 11,71 | Gd 17,92 | Na 2,62 |
| gef. | C 41,41 | H 5,72 | N 11,55 | Gd 17,74 | Na 2,41 |

### Beispiel 24

### a) 1,4,7,10-Tetrakis[3,9-bis(N-t.butyloxycarbonylmethyl)-3,6,9-triaza-undecan-1,11-dicarbonsäure-di-t.butylester-6-(2-oxo-methyl)]-1,4,7,10,13-pentaazacyclopentadecan

46,25 g (75 mmol sym.-DTPA-tetra-t.butylester und 2 g (9,29 mmol) 1,4,7,10,13-Pentaazacyclopentadecan werden in 300 ml Dimethylformamid gelöst, und bei 0°C werden 37,1 g (150 mmol) 2-Ethoxy-1-ethoxycarbonyl-1,2-dihydrochinolin zugegeben. Man rührt 24 Stunden bei Raumtemperatur. Man gibt 1000 ml Wasser zu und extrahiert 2 mal.mit je 300 ml Chloroform. Die organische Phase wird über Magnesiumsulfat getrocknet. Es wird im Vakuum zur Trockne eingedampft und der Rückstand an Kieselgel chromatographiert (Laufmittel: Methylenchlorid/ Methanol = 25 : 1) Ausbeute: 17,97 g (74 % d. Th.) eines farblosen, zähen Öls

| Elementaranalyse: | | | |
|---|---|---|---|
| ber. | C 59,72 | H 9,14 | N 9,11 |
| gef. | C 59,51 | H 9,32 | N 8,95 |

### b) 1,4,7,10-Tetrakis[3,9-bis(N-tbutyloxycarbonylmethyl)-3,6,9-triaza-undecan-1,11-dicarbonsäure-di-t.butylester-6-(2-oxo-methyl)]-13-<N-[4-(11-oxaundecyl)]-benzoyl>-1,4,7,10,13-pentaazacyclopentadecan

17 g (6,50 mmol) der Titelverbindung aus Beispiel 24a und 3,06 g (11 mmol) der Titelverbindung aus Beispiel 8a werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/2-Propanol = 25 : 1)
Ausbeute: 17,19 g (92 % d. Th.) eines farblosen, zähen Öls

| Analyse: | | | |
|---|---|---|---|
| ber. | C 61,42 | H 9,15 | N 8,28 |
| gef. | C 61,27 | H 9,30 | N 8,05 |

### c) 1,4,7,10-Tetrakis{[3,9-bis(N-carboxylatomethyl)-3,6,9-triaza-undecan-1-carboxylato-11-säure-6-(2-oxo-methyl)]-Gd-Komplex, Natriumsalz}-13-<N-[4-(11-oxaundecyl)]-benzoyl>-1,4,7,10,13-pentaazacyclopentadecan

10 g (3,48 mmol) der Titelverbindung aus Beispiel 24b werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4 gestellt. Anschließend gibt man 2,52 g (6,96 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 3 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange, bis das Eluat eine Leitfähigkeit von 10 µS erreicht hat. Dann wird der Inhalt der Ultrafiltrationszelle gefriergetrocknet.
Ausbeute: 9,05 g (97 % d. Th.) eines farblosen, amorphen Pulvers
Wassergehalt: 9,6 %

| Analyse (berechnet auf wasserfreie Substanz): | | | | | |
|---|---|---|---|---|---|
| ber. | C 37,17 | H 4,40 | N 8,88 | Gd 23,45 | Na 3,43 |
| gef. | C 36,94 | H 4,60 | N 8,71 | Gd 23,23 | Na 3,17 |

### Beispiel 25

### a) 1,4,7,10,13-Pentakis{1,4,7-tris(N-t.butyloxycarbonylmethyl)-10-[N-(4-aza-3,6-dioxo-hexan-2,6-diyl)]-1,4,7,10-tetraazacyclododecan}-1,4,7,13,16-hexaazacyclooctadecan

47,5 g (77 mmol) sym.-DTPA-tetra-t.butylester und 2 g (7,74 mmol) 1,4,7,10,13,16-Hexaazacyclooctadecan werden in 300 ml Dimethylformamid gelöst, und bei 0°C werden 37,1 g (150 mmol) 2-Ethoxy-1-ethoxycarbonyl-1,2-dihydrochinolin zugegeben. Man rührt 24 Stunden bei Raumtemperatur. Man gibt 1000 ml Wasser zu und extrahiert 2 mal.mit je 300 ml Chloroform. Die organische Phase wird über Magnesiumsulfat getrocknet. Es wird im Vakuum zur Trockne eingedampft und der Rückstand an Kieselgel chromatographiert (Laufmittel: Methylenchlorid/ Methanol = 25 : 1)
Ausbeute: 18,61 g (71 % d. Th.) eines farblosen, zähen Öls

| Elementaranalyse: | | | |
|---|---|---|---|
| ber. | C 59,21 | H 9,08 | N 12,82 |
| gef. | C 59,01 | H 9,24 | N 12,71 |

### b) 1,4,7,10,13-Pentakis{1,4,7-tris(N-t.butyloxycarbonylmethyl)-10-[N-(4-aza-3,6-dioxo-hexan-2,6-diyl)]-1,4,7,10-tetraazacyclododecan)-16-(N-hexadecanoyl)-1,4,7,13,16-hexaaza-cyclooctadecan

18 g (5,3 mmol) der Titelverbindung aus Beispiel 25a und 2,31 g (9 mmol) Palmitinsäure werden in 100 ml Dimethylformamid gelöst, und bei 0°C werden 4,2 g (17 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/2-Propanol = 25 : 1).
Ausbeute: 18,29 g (95 % d. Th.) eines farblosen, zähen Öls

| Analyse: | | | |
|---|---|---|---|
| ber. | C 60,62 | H 9,31 | N 11,98 |
| gef. | C 60,47 | H 9,48 | N 11,75 |

### c) 1,4,7,10,13-Pentakis{1,4,7-tris(N-carboxylatomethyl)-10-[N-(4-aza-3,6-dioxohexan-2,6-diyl)]-1,4,7,10-tetraazacyclododecan, Gd-Komplex}-16-(Nhexadecanoyl)-1,4,7,13,16-hexaaza-cyclooctadecan

12,61 g (3,48 mmol) der Titelverbindung aus Beispiel 25b werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschliessend gibt man 3,15 g (8,7 mmol) Gadoliniumoxid zu und rührt 3 Stunden bei 90°C. Man läßt auf Raumtemperatur kommen, stellt mit 5 %iger aqu. Natronlauge auf pH 7,2 und dialysiert die Lösung in einer Ultrafiltrationszelle (Amicon® YM-1). Der Inhalt der Ultrafiltrationszelle wird gefriergetrocknet.
Ausbeute: 12,21 g (96 % d. Th.) eines farblosen, amorphen Feststoffs
Wassergehalt: 10,2 %

| Elementaranalyse (berechnet auf wasserfreie Substanz): | | | | | |
|---|---|---|---|---|---|
| ber. | C 40,43 | H 5,52 | N 11,88 | Gd 21,52 | Na 3,15 |
| gef. | C 40,21 | H 5,75 | N 11,67 | Gd 21,35 | Na 2,84 |

### Beispiel 26

### a) 3-Oxa-tridecan-1-ol

Zu 32,93 g (200 mmol) des Tetrahydropyranylethers von 2-Chlorethanol, 1 g (3,6 mmol) Tetrabutylamoniumchlorid und 20 g (126,36 mmol) Decan-1-ol in 300 ml Toluol gibt man 44,88 g (800 mmol) fein gepulvertes Kaliumhydroxid und kocht 24 Stunden unter Rückfluß. Man filtriert vom Feststoff ab und dampft das Filtrat zur Trockne ein. Zum so erhaltenen Rückstand (Öl) gibt man 500 ml Ethanol/50 ml 10 %ige aqu. Salzsäure und rührt eine Stunde bei Raumtemperatur. Man dampft im Vakuum zur Trockne ein und chromatographiert den Rückstand an Kieselgel (Laufmittel: Hexan/Aceton = 20 : 1).
Ausbeute: 21,73 g (85 % d. Th.) eines farblosen Öls

| Elementaranalyse: | | |
|---|---|---|
| ber. | C 71,23 | H 12,95 |
| gef. | C 71,05 | H 13,10 |

### b) 3,6-Dioxa-hexadecansaure-tbutylester

Zu einer Mischung aus 4,36 g (21,55 mmol) der Titelverbindung aus Beispiel 26a und 0,73 g (2,15 mmol) Tetrabutylammoniumhydrogensulfat in 100 ml 60 %iger Kalilauge/50 ml Toluol tropft man unter starkem Rühren bei 0°C 10,51 g (53,9 mmol) Bromessigsäure-tert.butylester zu. Man rührt 1 Stunde bei 0°C. Es werden 200 ml Toluol zugegeben, die wässrige Phase abgetrennt und 2 mal mit je 50 ml Toluol extrahiert. Die vereinigten organische Phasen werden über Magnesiumsulfat getrocknet und im Vakuum eingedampft. Der Rückstand wird an Kieselgel chromatographiert (Laufmittel: Hexan/Dichlormethan/Aceton = 20 : 10 : 1).
Ausbeute: 6,34 g (93 % d. Th.) eines farblosen, viskosen Öls

| Elementaranalyse: | | |
|---|---|---|
| ber. | C 68,31 | H 11,46 |
| gef. | C 68,15 | H 11,28 |

### c) 3,6-Dioxa-hexadecansäure

6,2 g (19,59 mmol) der Titelverbindung aus Beispiel 26b werden in 100 ml Trifluoressigsäure gelöst und 6 Stunden bei Raumtemperatur gerührt. Man dampft zur Trockne ein. Der Rückstand wird aus Hexan/Ether kristallisiert.
Ausbeute: 4,69 g (92 % d. Th) eines farblosenFeststoffs

| Elementaranalyse (berechnet auf wasserfreie Substanz): | | |
|---|---|---|
| ber. | C 64,58 | H 10,84 |
| gef. | C 64,37 | H 11,15 |

### d) 1,4,7-Tris{1,4,7-tris(N-t.butyloxycarbonylmethyl)-10-[N-(4,7-diaza-3,6,9-trioxononan-2,9-diyl)]-1,4,7,10-tetraazacyclododecan}-10-[N-(3,6-dioxa-hexanoyl)]-1,4,7, 10-tetraazacyclododecan

20,74 g (9,34 mmol) der Titelverbindung aus Beispiel 15c und 3,65 g (14 mmol) der Titelverbindung aus Beispiel 26c werden in 150 ml Dimethylformamid gelöst, und bei 0°C werden 7,42 g (30 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/Methanol = 20 : 1)
Ausbeute: 20,02 g (87% d. Th.) eines farblosen, zähen Öls

| Analyse: | | | |
|---|---|---|---|
| ber. | C 59,00 | H 9,00 | N 12,51 |
| gef. | C 58,78 | H 9,17 | N 12,35 |

### e) 1,4,7-Tris{1,4,7-tris (N-carboxylatomethyl)-10-[N-(4,7-diaza-3,6,9-trioxo-nonan-2,9-diyl)]-1,4,7,10-tetraazacyclododecan, Gd-Komplex}-10-[N-(3,6-dioxahexanoyl)]-1,4,7,10-tetraazacyclododecan

10,35 g (4,2 mmol) der Titelverbindung aus Beispiel 26d werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 9,66 g (95 % d. Th.) eines farblosen, amorphen Feststoffs
Wassergehalt: 11,1 %

| Elementaranalyse (berechnet auf wasserfreie Substanz): | | | | |
|---|---|---|---|---|
| ber. | C 42,17 | H 5,79 | N 12,73 | Gd 19,49 |
| gef. | C 42,01 | H 5,94 | N 12,60 | Gd 19.28 |

Relaxivity R₁ (1 • mmol⁻¹ s⁻¹) in Plasma (20 MHz, 40° C, 0,47 Tesla) : 22,0

### Beispiel 27

### a) 1,4,7-Tris[N-(2-benzyloxycarbonylamino-3-phenyl)-propionyl]-1,4,7,10-tetraazacyclododecan

Zu 5 g (29,02 mmol) 1,4,7,10- Tetraazacyclododecan (= Cyclen) in 300 ml Toluol gibt man 35,66 g (89,96 mmol) N-(Benzyloxycarbonyl)-Phenylalanin-N-Hydroxysuccinimidester und 10,12 g (100 mmol) Triethylamin und erhitzt 12 Stunden unter Rückfluß. Man dampft im Vakuum zur Trockne ein, nimmt den Rückstand in 400 ml Dichlormethan auf und wäscht die organische Phase 2 mal mit 5 %iger aqu. Natriumcarbonat-Lösung. Man trocknet die organische Phase über Magnesiumsulfat und engt im Vakuum zur Trockne ein. Der Rückstand wird an Kieselgel chromatographiert (Laufmittel: Essigsäureethylester/Ethanol = 20 : 1).
Ausbeute: 18,58 g (63 d. Th.) eines farblosen Schaums

| Elementaranalyse: | | | |
|---|---|---|---|
| ber. | C 69,73 | H 6,45 | N 9,65 |
| gef. | C 69,41 | H 6,63 | N 9,47 |

### b) 1,4,7-Tris[N-(2-benzyloxycarbonylamino-3-phenyl)-propionyl]-10-(N-heptanoyl)-1,4,7,10-tetraazacyclododecan

18 g (17,71 mmol) der Titelverbindung aus Beispiel 27a und 3,25 g (25 mmol) Heptansäure werden in 100 ml Dimethylformamid gelöst, und bei 0°C werden 9,89 g (40 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/2-Propanol = 25 : 1)
Ausbeute: 18,19 g (91 % d. Th.) eines farblosen, zähen Öls

| Analyse: | | | |
|---|---|---|---|
| ber. | C 70,25 | H 6,88 | N 8,69 |
| gef. | C 70,11 | H 6,99 | N 8,70 |

### c) 1,4,7-Tris[N-(2-amino-3-phenyl)-propionyl]-10-(N-heptanoyl)-1,4,7,10-tetraazacyclododecan, Trihydrobromid

18 g (15,95 mmol) der Titelverbindung aus Beispiel 27b werden in 100 ml Essigsäure gelöst und zu einer 60°C heißen Lösung aus 300 ml Bromwasserstoffin Eisessig (33 %ig) getropft. Man rührt 1 Stunde bei 60°C. Es wird auf0°C abgekühlt und unter Rühren 2000 ml Diethylether langsam zugetropft. Man filtriert den ausgefallenen Niederschlag ab, wäscht 2 mal mit 200 ml Ether nach und trocknet im Vakuumofen (60°C).
Ausbeute: 14,83 g (96 d. Th.) farbloser, kristalliner Feststoff

| Elementaranalyse: | | | | |
|---|---|---|---|---|
| ber. | C 52,08 | H 6,45 | N 10,12 | Br 24,75 |
| gef. | C 51,85 | H 6,61 | N 10,00 | Br 24,48 |

### d) 1,4,7-Tris{N-[3-phenylpropionyl-2-amino-<N-(2-benzyl-3,6-diaza-7-oxo-8-ylnonanoyl-8-yl-[1,4,7-tris(N-t.butyloxycarbonylmethyl)-1,4,7,10-tetraazacyclododecan-10-yl>]}10-(N-heptanoyl)-1,4,7,10-tetraazacyclododecan

Zu 55,26 g (74 mmol) Gly-Methyl-DOTA-tri-tbutylester (Natriumbromid-Komplex, gelöst in 300 ml Dimethylformamid, gibt man bei 0°C 11,51 g (100 mmol) N-Hydroxysuccinimid und 16,51 g (80 mmol) Dicyclohexylcarbodiimid und rührt 1 Stunde bei dieser Temperatur. Anschließend rührt man 3 Stunden bei Raumtemperatur. Zu dieser Lösung gibt man 8,97 g (9,26 mmol) der Titelverbindung aus Beispiel 27c und 10,12 g (100 mmol) Triethylamin. Man rührt 24 Stunden bei Raumtemperatur. Die Lösung wird zur Trockne eingedampft und der Rückstand in 800 ml Dichlormethan aufgenommen. Man filtriert vom Dicyclohexylharnstoff ab und wäscht das Filtrat 2 mal mit 300 ml 5 %iger aqu Natriumcarbonat-Lösung. Die organische Phase wird über Magnesiumsulfat getrocknet und im Vakuum zur Trockne eingedampft. Der Rückstand wird an Kieselgel chromatographiert (Laufmittel: Dichlormethan/Methanol = 20 : 1). Ausbeute: 21,7 g (90% d. Th.) eines farblosen, zähen Öls

| Elementaranalyse: | | | |
|---|---|---|---|
| ber. | C 62,28 | H 8,67 | N 11,84 |
| gef. | C 62,09 | H 8,84 | N 11,69 |

### e) 1,4,7-Tris{N-[3-phenylpropionyl-2-amino-<N-(2-benzyl-3,6-diaza-7-oxo)-nonanoyl-8-Y1-[1,4,7-tris[N-carboxylatomethyl)-1,4,7,10-tetraazacyclododecan-10-yl]>]}-10-(N-heptanoyl)-1,4,7,10-tetraazacyclododecan

10,93 g (4,2 mmol) der Titelverbindung aus Beispiel 27d werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 10,43 g (97 % d. Th.) eines farblosen, amorphen Feststoffs
Wassergehalt: 12,0 %

| Elementaranalyse (berechnet auf wasserfreie Substanz): | | | | |
|---|---|---|---|---|
| ber. | C 46,43 | H 5,63 | N 12,03 | Gd 18,42 |
| gef. | C 46,22 | H 5,75 | N 11,85 | Gd 18,23 |

Relaxivity R₁ (1 • mmol⁻¹ s⁻¹) in Plasma (20 MHz. 40° C, 0,47 Tesla) : 12,8

### Beispiel 28

### a) 1,4,7-Tris{3,9-bis(N-t.butyloxycarbonylmethyl)-6-[N-(3-aza-2,5-dioxo)-pentan-1,5-diyl]-3,6,9-triazaundecandisäure-di-t.butylester}-10-(N-pentadecyl)-1,4,7,10-tetraazacyclododecan

Zu 20 g (9,33 mmol) der Titelverbindung aus Beispiel 1b, 6,91 g (50 mmol) Kaliumcarbonat und 5,07 g (15 mmol) Pentadecyl-1-jodid gibt man 150 ml Acetonitril und erhitzt 12 Stunden auf 60°C. Man filtriert vom Feststoff ab, dampft das Filtrat im Vakuum zur Trockne ein und chromatographiert den Rückstand an Kieselgel (Laufmittel: Dichlormethan/2-Propanol =20 : 1)
Ausbeute: 17,78 g (81 % d. Th.) eines farblosen, zahen Öls

| Elementaranalyse: | | | |
|---|---|---|---|
| ber. | C 60,74 | H 9,34 | N 9,52 |
| gef. | C 60,54 | H 9,51 | N 9,37 |

### b) 1,4,7-Tris{3,9-bis(N-carboxylatomethyl)-6-[N-(3-aza-2,5-dioxo)-pentan-1,5-diyl]-3,6,9-triazaundecan-1-carboxylato-11-säure, Gd-Komplex} -dinatriumsalz-10-[N-(pentadecyl)]-1,4,7,10-tetraazacyclododecan

9,88 g (4,2 mmol) der Titelverbindung aus Beispiel 28a werden in 200 ml Trifluoressigsäure gelöst und 12 Stunden bei Raumtemperatur gerührt. Man dampft im Vakuum zur Trockne ein. Der Rückstand wird in 100 ml Wasser gelöst und mit 10 %iger aqu. Natronlauge auf pH 4 gestellt. Anschließend gibt man 2,28 g (6,3 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 2 g Aktivkohle versetzt, 1 Stunde bei Raumtemperatur gekühlt und filtriert. Das Filtrat wird in eine Ultrafiltrationszelle gefüllt und dialysiert (Amicon® YM-1). Man dialysiert solange, bis das Eluat eine Leitfähigkeit von 10 µS erreicht hat. Dann wird der Inhalt der Ultrafiltrationszelle gefriergetrocknet.
Ausbeute: 8,81 g (96 % d. Th.) eines farblosen, amorphen Pulvers
Wassergehalt: 7,6 %

| Analyse (berechnet auf wasserfreie Substanz); | | | | | |
|---|---|---|---|---|---|
| ber. | C 39,00 | H 5,12 | N 10,25 | Gd 21,58 | Na 2,10 |
| gef. | C 38,83 | H 5,29 | N 10,11 | Gd 21,39 | Na 1,87 |

### Beispiel 29

### a) 1,4,7-Tris{1,4,7-tris(N-t.butyloxycarbonylmethyl)-10-[N-(4,7-diaza-3,6,9-trioxononan-2,9-diyl)]-1,4,7,10-tetraazacyclododecan}-10-(N-tetradecyl)-1,4,7,10-tetraazacyclododecan

Zu 20 g (9,33 mmol) der Titelverbindung aus Beispiel 15c, 6,91 g (50 mmol) Kaliumcarbonat und 4,86 g (15 mmol) Tetradecyl-1-jodid gibt man 150 ml Acetonitril und erhitzt 12 Stunden auf 60°C. Man filtriert vom Feststoff ab, dampft das Filtrat im Vakuum zur Trockne ein und chromatographiert den Rückstand an Kieselgel (Laufmittel: Dichlormethan/Methanol = 20 : 1)
Ausbeute: 20,52 g (91 % d. Th.) eines farblosen, zähen Öls

| Elementaranalyse: | | | |
|---|---|---|---|
| ber. | C 60,12 | H 9,26 | N 12,75 |
| gef. | C 59,87 | H 9,48 | N 12,55 |

### b) 1,4,7-Tris{1,4,7-tris(N-carboxylatomethyl)-10-[N-(4,7-diaza-3,6,9-trioxo-nonan-2,9-diyl)]-1,4,7,10-tetraazacyclododecan, Gd-Komplex}-10-(N-tetradecyl)-1,4,7,10-tetraazacyclododecan

10,15 g (4,2 mmol) der Titelverbindung aus Beispiel 29a werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 9,47 g (95 % d. Th.) eines farblosen, amorphen Feststoffs
Wassergehalt: 11,5%

| Elementaranalyse (berechnet auf wasserfreie Substanz): | | | | |
|---|---|---|---|---|
| ber. | C 42,99 | H 5,98 | N 12,98 | Gd 19,86 |
| gef. | C 42,75 | H 6,18 | N 12,75 | Gd 19,64 |

### Beispiel 30

### a) 7,7-Diphenyl-hept-6-en-carbonsäure

Zu 13,54 g (120,7 mmol) Kalium-t.-butylat, gelöst in 35 ml Dimethylsulfoxid/35 ml Tetrahydrofuran, tropft man bei 0°C eine Lösung bestehend aus 10 g (54,9 mmol) Benzophenon und 25,10 g (54,9 mmol) 6-(Triphenylphosphonium)-hexansäurebromid gelöst in 150 ml Dimethylsulfoxid/ 150 ml Tetrahydrofuran. Man rührt über Nacht bei Raumtemperatur. Die Lösung wird auf 2 l Eiswasser gegeben und mit 20 %iger aqu. Salzsäure auf pH 2 gestellt. Man extrahiert 3 mal mit je 200 ml Diethylether. Die vereinigten organischen Phasen werden 2 mal mit je 200 ml gesättigter aqu. Natriumchlorid-Lösung gewaschen, über Magnesiumsulfat getrocknet und im Vakuum zur Trockne eingedampft. Der Rückstand wird an Kieselgel chromatographiert (Laufmittel: Hexan/ Essigsäureethylester = 5 : 1 / + 1 % Essigsäure),
Ausbeute. 12 g (78 % d. Th.) eines farblosen Feststoffs

| Elementaranalyse: | | |
|---|---|---|
| ber. | C 81,40 | H 7,19 |
| gef. | C 81,17 | H 7,38 |

### b) 7,7-Diphenylheptansäure

11,5 g (41 mmol) der Titelverbindung aus Beispiel 30a werden in 200 ml 2-Propanol gelöst und 3 g Palladiumkatalysator (10 % Pd/C) zugegeben. Man hydriert 6 Stunden bei Raumtemperatur. Der Katalysator wird abfiltriert und das Filtrat im Vakuum zur Trockne eingedampft.
Ausbeute: 11,58 g (quantitativ) eines farblosen Feststoffs.

| Elementaranalyse: | | |
|---|---|---|
| ber. | C 80,82 | H 7,85 |
| gef. | C 80,65 | H 7,99 |

### c) 1,4,7-Tris{1,4,7-tris(N-t.butyloxycarbonylmethyl)-10-[N-(4,7-diaza-3,6,9-trioxononan-2,9-diyl)]-1,4,7,10-tetraazacyclododecan}-10-[N-(7,7-diphenylheptanoyl)]-1,4,7,10-tetraazacyclododecan

2,63 g (9,33 mmol) der Titelverbindung aus Beispiel 30b und 10,37 g (4,67 mmol) der Titelverbindung aus Beispiel 15c werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/Methanol = 25 : 1)
Ausbeute: 11,03 g (95 % d. Th.) eines farblosen, zähen Öls

| Analyse: | | | |
|---|---|---|---|
| ber. | C 60,90 | H 8,68 | N 12,40 |
| gef. | C 60,75 | H 8,83 | N 12,27 |

### d) 1,4,7-Tris{1,4,7-tris(N-carboxylatomethyl)-10-[N-(4,7-diaza-3,6,9-trioxo-nonan-2,9-diyl)]-1,4,7,10-tetraazacyclododecan, Gd-Komplex}-10-[N-(7,7-diphenylheptanoyl)]-1,4,7,10-tetraazacyclododecan

10,44 g (4,2 mmol) der Titelverbindung aus Beispiel 30c werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1). Der Inhalt der Ultrafiltrationszelle wird gefriergetrocknet.
Ausbeute: 9,85 g (96 % d. Th.) eines farblosen, amorphen Feststoffs
Wassergehalt: 9,7 %

| Elementaranalyse (berechnet auf wasserfreie Substanz): | | | | |
|---|---|---|---|---|
| ber. | C 44,25 | H 5,49 | N 12,61 | Gd 19,31 |
| gef. | C 44,09 | H 5,72 | N 12,40 | Gd 19,13 |

### Beispiel 31

### a) 7-(Biphen-4-yl)-hept-6-en-säure

Zu 13,54 g (120,7 mmol) Kalium-t.-butylat, gelöst in 35 ml Dimethylsulfoxid/35 ml Tetrahydrofuran, tropft man bei 0°C eine Lösung bestehend aus 10 g (54,9 mmol) 4-Biphenylaldehyd und 25,10 g (54,9 mmol) 6-(Triphenylphosphonium)-hexansäurebromid, gelöst in 150 ml Dimethylsulfoxid/150 ml Tetrahydrofuran. Man rührt über Nacht bei Raumtemperatur. Die Lösung wird auf 2 l Eiswasser gegeben und mit 20 %iger aqu. Salzsäure auf pH 2 gestellt. Man extrahiert 3 mal mit je 200 ml Diethylether. Die vereinigten organischen Phasen werden 2 mal mit je 200 ml gesättigter aqu. Natriumchlorid-Lösung gewaschen, über Magnesiumsulfat getrocknet und im Vakuum zur Trockne eingedampft. Der Rückstand wird an Kieselgel chromatographiert (Laufmittel: a-Hexan/Essigsaureethylester = 5 : 1 / + 1 % Essigsäure).
Ausbeute: 12,47 g (81 % d. Th.) eines farblosen Feststoffs

| Elementaranalyse: | | |
|---|---|---|
| ber. | C 81,40 | H 7,19 |
| gef. | C 81,28 | H 7,31 |

### b) 7-(Biphen-4-yl)-heptansäure

12 g (42,8 mmol) der Titelverbindung aus Beispiel 31a werden in 200 ml 2-Propanol gelöst und 3 g Palladiumkatalysator (10 % Pd/C) zugegeben. Man hydriert 6 Stunden bei Raumtemperatur. Der Katalysator wird abfiltriert und das Filtrat im Vakuum zur Trockne eingedampft.
Ausbeute: 12,07 g (quantitativ) eines farblosen Feststoffs.

| Elementaranalyse: | | |
|---|---|---|
| ber. | C 80,82 | H 7,85 |
| gef. | C 80,68 | H 7,99 |

### c) 1,4,7-Tris{1,4,7-tris(N-t.butyloxycarbonylmethyl)-10-[N-(4,7-diaza-3,6,9-trioxononan-2,9-diyl)]-1,4,7,10-tetraazacyclododecan}-10-[N-7-(biphen-4-yl-heptanoyl]-1,4,7,10-tetraazacyclododecan

2,63 g (9,33 mmol) der Titelverbindung aus Beispiel 31b und 10,37 g (4,67 mmol) der Titelverbindung aus Beispiel 15c werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/Methanol = 25 : 1)
Ausbeute: 10,79 g (93 % d. Th.) eines farblosen, zähen Öls

| Analyse: | | | |
|---|---|---|---|
| ber. | C 60,90 | H 8,68 | N 12,40 |
| gef. | C 60,57 | H 8,89 | N 12.18 |

### d) 1,4,7-Tris{1,4,7-tris(N-carboxylatomethyl)-10-[N-(4,7-diaza-3,6,9-trioxo-nonan-2,9-diyl)]-1,4,7,10-tetraazacyclododecan, Gd-Komplex}-10-[N-7-(biphen-4-ylheptanoyl]-1,4,7,10-tetraazacyclododecan

10,44 g (4,2 mmol) der Titelverbindung aus Beispiel 31c werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3.3 eingestellt. Anschließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 9,95 g (97 % d. Th.) eines farblosen, amorphen Feststoffs
Wassergehalt: 8,4 %

| Elementaranalyse (berechnet auf wasserfreie Substanz): | | | | |
|---|---|---|---|---|
| ber. | C 44,25 | H 5,49 | N 12,61 | Gd 19,31 |
| gef. | C 44,08 | H 5,68 | N 12,43 | Gd 19,12 |

### Beispiel 32

### a) 7-(2,3,4-Trihydronaphtalin-1-yl)-hept-6-en-säure

Zu 13,54 g (120,7 mmol) Kalium-t.-butylat gelöst in 35 ml Dimethylsulfoxid/35 ml Tetrahydrofuran, tropft man bei 0°C eine Lösung bestehend aus 8,03 g (54,9 mmol) α-Tetralon und 25,10 g (54,9 mmol) 6-(Triphenylphosphonium)-hexansäurebromid, gelöst in 150 ml Dimethylsulfoxid/150 ml Tetrahydrofuran. Man rührt über Nacht bei Raumtemperatur. Die Lösung wird auf 2 l Eiswasser gegeben und mit 20 %iger aqu. Salzsäure auf pH 2 gestellt. Man extrahiert 3 mal mit je 200 ml Diethylether. Die vereinigten organischen Phasen werden 2 mal mit je 200 ml gesättigter aqu. Natriumchlorid-Lösung gewaschen, über Magnesiumsulfat getrocknet und im Vakuum zur Trockne eingedampft. Der Rückstand wird an Kieselgel chromatographiert (Laufmittel: Hexan/Essigsäureethylester = 5 : 1 / + 1 % Essigsäure).
Ausbeute: 10,33 g (77 % d. Th.) eines farblosen Feststoffs

| Elementaranalyse. | | |
|---|---|---|
| ber. | C 78,65 | H 8,25 |
| gef. | C 78,49 | H 8,37 |

### b) 7-(1,2,3,4,-Tetrahydronaphthalin-1-yl)-heptansäure

10 g (40,93 mmol) der Titelverbindung aus Beispiel 32a werden in 200 ml 2-Propanol gelöst und 3 g Palladiumkatalysator (10 % Pd/C) zugegeben. Man hydriert 6 Stunden bei Raumtemperatur. Der Katalysator wird abfiltriert und das Filtrat im Vakuum zur Trockne eingedampft.
Ausbeute: 10,08 g (quantitativ) eines farblosen, zähen Öls

| Elementaranalyse: | | |
|---|---|---|
| ber. | C 78,01 | H 9,00 |
| gef. | C 77,88 | H 9,19 |

### c) 1,4,7-Tris{1,4,7-tris(N-t.butyloxycarbonylmethyl)-10-[N-(4,7-diaza-3,6,9-trioxononan-2,9-diyl)]-1,4,7,10-tetraazacyclododecan}-10-<N-[7-(1,2,3,4-tetrahydronaphthalin-1-yl)-heptanoyl]>-1,4,7,10-tetraazacyclododecan

2,29 g (9,33 mmol) der Titelverbindung aus Beispiel 32b und 10,37 g (4,67 mmol) der Titelverbindung aus Beispiel 15c werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/Methanol = 25:1)
Ausbeute: 10,75 g (94 % d. Th.) eines farblosen, zähen Öls

| Analyse: | | | |
|---|---|---|---|
| ber. | C 60,32 | H 8,81 | N 12,58 |
| gef. | C 60,17 | H 8,95 | N 12,39 |

### d) 1,4,7-Tris{1,4,7-tris(N-carboxylatomethyl)-10-[N-(4,7-diaza-3,6,9-trioxo-nonan-2,9-diyl)]-1,4,7,10-tetraazacyclododecan, Gd-Komplex}-10-<N-[7-(1,2,3,4-tetrahydronaphthalin-1-yl)-heptanoyl]>-1,4,7,10-tetraazacyclododecan

10,29 g (4,2 mmol) der Titelverbindung aus Beispiel 32c werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt Anschließend gibt man 2,28 g (6,3 mmol) Gadoliniumoxid zu und rührt 3 Stunden bei 90°C. Man läßt auf Raumtemperatur kommen, stellt mit 5 %ige aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 9,81 g (97 % d. Th.) eines farblosen, amorphen Feststoffs
Wassergehalt: 10,4 %

| Elementaranalyse (berechnet auf wasserfreie Substanz): | | | | |
|---|---|---|---|---|
| ber. | C 43,42 | H 5,57 | N 12,80 | Gd 19,60 |
| gef. | C 43,26 | H 5,73 | N 12,61 | Gd 19,42 |

### Beispiel 33

### a) 3-(4,4-Diphenylcyclohexyl)-3-oxapropionsäure-t.butylester

Zu einer Mischung aus 5,44 g (21,55 mmol) 4,4-Diphenyl-cyclohexanol und 0,73 g (2,15 mmol) Tetrabutylammoniumhydrogensulfat in 100 ml 60 %iger Kalilauge/50 ml Toluol tropft man unter starkem Rühren bei 0°C 10,51 g (53,9 mmol) Bromessigsäuretert.butylester zu. Man rührt 1 Stunde bei 0°C. Es werden 200 ml Toluol zugegeben, die wässrige Phase abgetrennt und 2 mal mit je 50 ml Toluol extrahiert. Die vereinigten organische Phasen werden über Magnesiumsulfat getrocknet und im Vakuum eingedampft. Der Rückstand wird an Kieselgel chromatographiert (Laufmittel: Hexan/Dichlormethan/Aceton = 20 : 10 : 1).
Ausbeute: 6,16 g (78 % d. Th.) eines farblosen, viskosen Öls

| Elementaranalyse: | | |
|---|---|---|
| ber. | C 78,65 | H 8,25 |
| gef. | C 78,48 | H 8,41 |

### b) 3-(4,4-Diphenylcyclohexyl)-3-oxapropionsäure

6 g (16,37 mmol) der Titelverbindung aus Beispiel 33a werden in 100 ml Trifluoressigsäure gelöst und 6 Stunden bei Raumtemperatur gerührt. Man dampft zur Trockne ein und chromatographiert den Rückstand an Kieselgel. (Laufmittel: Hexan/Diethylether = 5 : 1 / + 2 % Essigsäure)
Ausbeute: 4,7 g (93 % d. Th.) eines farblosen Feststoffs

| Elementaranalyse: | | |
|---|---|---|
| ber. | C 77,39 | H 7,14 |
| gef. | C 77,18 | H 7,38 |

### c) 1,4,7-Trist{1,4,7-tris(N-tbutyloxycarbonylmethyl)-10-[N-(4,7-diaza-3,6.9-trioxononan-2,9-diyl)]-1,4,7,10-tetraazacyclododecan}-10-<N-[3-(4,4-diphenylcyclohexyl)-3-oxapropionyl]>-1,4,7,10-tetraazacyclododecan

2,90 g (9,33 mmol) der Titelverbindung aus Beispiel 33b und 10,37 g (4,67 mmol) der Titelverbindung aus Beispiel 15c werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/Methanol = 25 : 1)
Ausbeute: 10,84 g (93 % d. Th.) eines farblosen, zähen Öls

| Analyse: | | | |
|---|---|---|---|
| ber. | C 61,08 | H 8,64 | N 12,34 |
| gef. | C 60,91 | H 8,85 | N 12,21 |

### d) 1,4,7-Tris{1,4,7-tris(N-carboxylatomethyl)-10-[N-(4,7-diaza-3,6,9-trioxo-nonan-2,9-diyl)]-1,4,7,10-tetraazacyclododecan, Gd-Komplex}-10-<N-[3-(4,4-diphenylcyclohexyl)-3-oxapropionyl]>-1,4,7,10-tetraazacyclododecan

10,49 g (4,2 mmol) der Titelverbindung aus Beispiel 33c werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 9,86 g (95 % d. Th.) eines farblosen, amorphen Feststoffs
Wassergehalt: 10,1 %

| Elementaranalyse (berechnet auf wasserfreie Substanz): | | | | |
|---|---|---|---|---|
| ber. | C 44,23 | H 5,43 | N 12,47 | Gd 19,09 |
| gef. | C 44,08 | H 5,65 | N 12,28 | Gd 18,91 |

### Beispiel 34

### a) 3-(4-Nonanylphenyl)-3-oxapropionsäure-t.butylester

Zu einer Mischung aus 4,75 g (21,55 mmol) 4-(Nonanyl)-phenol und 0,73 g (2,15 mmol) Tetrabutylammoniumhydrogensulfat in 100 ml 60 %iger Kalilauge/50 ml Toluol tropft man unter starkem Rühren bei 0°C 10,51 g (53,9 mmol) Bromessigsäure-tert.butylester zu. Man rührt 1 Stunde bei 0°C. Es werden 200 ml Toluol zugegeben, die wässrige Phase abgetrennt und 2 mal mit je 50 ml Toluol extrahiert. Die vereinigten organische Phasen werden über Magnesiumsulfat getrocknet und im Vakuum eingedampft. Der Rückstand wird an Kieselgel chromatographiert (Laufmittel: Hexan/Dichlormethan/Aceton = 20 : 10 : 1).
Ausbeute: 6,7 g (93 % d. Th.) eines farblosen, viskosen Öls

| Elementaranalyse: | | |
|---|---|---|
| ber. | C 75,41 | H 10,24 |
| gef. | C 75,28 | H 10,39 |

### b) 3-(4-Nonanylphenyl)-3-oxapropionsäure

6,6 g (19,73 mmol) der Titelverbindung aus Beispiel 34a werden in 100 ml Trifluoressigsäure gelöst und 6 Stunden bei Raumtemperatur gerührt. Man dampft zur Trockne ein und chromatographiert den Rückstand an Kieselgel. (Laufmittel: Hexan/Diethylether = 5 : 1 / + 2 % Essigsäure)
Ausbeute: 5,16 g (94 % d. Th.) eines farblosen Feststoffs

| Elementaranalyse: | | |
|---|---|---|
| ber. | C 73,35 | H 10,24 |
| gef. | C 73,21 | H 10,41 |

### c) 1,4,7-Tris{1,4,7-tris(N-t.butyloxycarbonylmethyl)-10-[N-(4,7-diaza-3,6,9-trioxononan-2,9-diyl)]- 1,4,7,10-tetraazacyclododecan}-10-<N-[3-(4-Nonanylphenyl)-3-oxapropionyl]>-1,4,7,10-tetraazacyclododecan

2,59 g (9,33 mmol) der Titelverbindung aus Beispiel 34b und 10,37 g (4,67 mmol) der Titelverbindung aus Beispiel 8a werden in 100 ml Dimethylformamid gelöst, und bei 0°C werden 3,71 g (15 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Methylenchlorid/2-Propanol = 25 : 1)
Ausbeute: 10,34 g (93 % d. Th.) eines farblosen, zähen Öls

| Elementaranalyse: | | | |
|---|---|---|---|
| ber. | C 61,42 | H 9,15 | N 8,28 |
| gef. | C 61,27 | H 9,30 | N 8,05 |

### d) 1,4,7-Tris{1,4,7-tris(N-carboxylatomethyl)-10-[N-(4,7-diaza-3,6,9-trioxo-nonan-2,9-diyl)]-1,4,7,10-tetraazacyclododecan, Gd-Komplex}-10-<N-[3-(4-Nonanylphenyl)-3-oxapropionyl]>-1,4,7,10-tetraazacyclododecan

10 g (4,2 mmol) der Titelverbindung aus Beispiel 34c werden in 200 ml Trifluoressigsäure gelöst und 12 Stunden bei Raumtemperatur gerührt. Man dampft zur Trockne ein. Der Rückstand wird in 100 ml Wasser gelöst und mit 5 %iger aqu. Natronlauge auf pH 3,3 eingestellt. Anschließend gibt man 2,28 g (6,3 mmol) Gadoliniumoxid zu und rührt 3 Stunden bei 90°C. Man läßt auf Raumtemperatur kommen, stellt mit 5 %iger aqu. Natronlauge auf pH 7,0 und dialysiert die Lösung in einer Ultrafiltrationszelle (Amicon® YM-1), 6 Durchläufe. Der Inhalt der Ultrafiltrationszelle wird gefriergetrocknet.
Ausbeute: 9,93 g (97 % d. Th.) eines farblosen, amorphen Feststoffs
Wassergehalt: 11,0%

| Elementaranalyse (berechnet auf wasserfreie Substanz): | | | | |
|---|---|---|---|---|
| ber. | C 43,34 | H 5,66 | N 12,63 | Gd 19,34 |
| gef. | C 43,18 | H 5,81 | N 12,47 | Gd 19,15 |

### Beispiel 35: MRI Experimente an Tieren mit induzierten Niereninfarkten

Das Enhancement im MRI Experiment wurde nach einmaliger intravenöser Applikation der Substanz aus Beispiel 1d an Tieren mit experimentell erzeugten Nieren-Nekrosen bzw. -Infarkten untersucht.

Die Induktion der Niereninfarkte erfolgte an narkotisierten (Rompun®/Ketavet®, i. p.) Ratten (Han. Wistar. Schering SPF, ca. 200 g Körpergewicht) durch Okklusion eines (caudalen) Astes der linken Nierenarterie. Die Kontrastmittelapplikation (Dosis: 100 µmol Gd / kg Körpergewicht) erfolgte ca. 24 h nach der Infarktinduktion. Die Tiere wurden vor und bis 24 h nach Kontrastmittelapplikation MR-tomographisch (SISCO SIS 85, 2 Tesla; SE-Sequenz, T_{R}: 400 ms, T_{E}: 15 ms, nt=4, ni=128, FOV: 12·7 cm, SD ≈ 3 mm, je 1 Schicht axial bzw. coronar) untersucht.

Nach Abschluß der MRI-Experimente wurden die narkotisierten Tiere durch Entbluten (via V. cava) getötet und beide Nieren präpariert. Zur Verifizierung des Infarktes (Größe und Lage) wurde die linke (infarzierte) Niere entnommen, in Scheiben geschnitten und anschließend eine NBT ("Vital-") Färbung durchgerührt.

Direkt nach der Substanzapplikation stellte sich der nicht-perfundierte Anteil der Niere jeweils als hypointenses Areal dar (s. Fig. 1, Teil a). Ab ca. 15-30 min p. i. stieg allerdings die Signalintensität im nicht-perfundierten Areal etwas an bzw. die Größe des abgegrenzten (signalarmen) Areals nahm ab (langsame Diffusion in die Nekrose). In der späten Phase (24 h p. i.) war bei allen untersuchten Tieren ein deutliches Enhancement im nekrotischen Areal der Niere festzustellen (s. Fig. 1, Teil b). Die Abgrenzung des nekrotischen Areals im MRI-Experiment korreliert sehr gut mit den Ergebnissen der histologischen "Vital"-Färbung.

### Beispiel 36: MRI Experimente an Tieren mit ethanol-induzierter Nekrose

Das Enhancement nach einmaliger Applikation der Substanz aus Beispiel 1d wurde im MRI Experiment an Tieren mit experimentell erzeugten Leber-Nekrosen untersucht.

Die Induktion der Nekrose erfolgte an Ratten (Han.. Wistar, Schering SPF, ca. -200 g Körpergewicht) durch percutane Applikation von 200 µl Ethanol (80 %) in die Leber. Die Kontrastmittelapplikation (Dosis: 100 µmol Gd / kg Körpergewicht) erfolgte ca. 24 h nach der Nekroseinduktion. Die Tiere wurden vor und bis 24 h nach Kontrastmittelapplikation MR-tomographisch (SISCO SIS 85, 2 Tesla; SE-Sequenz, T_{R}: 400 ms. T_{E}: 15 ms, nt=4, ni=128, FOV: 7*7 cm, SD ≈ 3 mm, je 1 Schicht axial) untersucht.

Nach Abschluß der MRI-Experimente wurden die narkotisierten Tiere durch Entbluten (via V. cava) getötet, die Leber präpariert und zur Beurteilung der Lebernekrose (hinsichtlich Größe und Lage) wurde eine NBT-("Vital-") Färbung durchgeführt.

Direkt nach der Substanzapplikation stellte sich der nekrotische Anteil der Leber jeweils als schlecht abzugrenzendes, schwach hypointenses Areal dar (s. Fig. 2, Teil b). In der späten Phase (24 h p. i.) war dagegen bei allen untersuchten Tieren ein deutliches Enhancement im nekrotischen Areal der Leber festzustellen (s. Fig. 2, Teil c). Die Abgrenzung des nekrotischen Areals im MRI-Experiment korreliert sehr gut mit den Ergebnissen der histologischen "Vital"-Färbung.

### Beispiel 37: MRI Experimente an Tieren mit induziertem Myocardinfarkt

Das Enhancement im MRI Experiment wurde nach einmaliger intravenöser Applikation der Substanz aus Beispiel 18b an Tieren mit experimentell erzeugtem Myocardinfarkt untersucht.

Die Induktion der Herzinfarkte erfolgte an narkotisierten (Rompun®/Ketavet®, i. p.) Ratten (Han. Wistar, Schering SPF, ca. 200 g Körpergewicht) durch Okklusion der linken Coronararterie. Die Kontrastmittelapplikation (Dosis: 200 µmol Gd / kg Körpergewicht) erfolgte ca. 24 h nach der Infarktinduktion. Die Tiere wurden vor und bis 24 h nach Kontrastmittelapplikation MR-tomographisch (SISCO SIS 85, 2 Tesla; SE-Sequenz, T_{R}. 400 ms, T_{E}: 15 ms, nt=4, ni=128, FOV: 12*7 cm, SD ≈ 3 mm, je 1 Schicht axial bzw coronar) untersucht.

Nach Abschluß der MRI-Experimente wurden die narkotisierten Tiere durch Entbluten (via V. cava) getötet und das Herz präpariert. Zur Verifizierung des Infarktes (Größe und Lage) wurde das Herz entnommen, in Scheiben geschnitten und anschließend eine NBT ("Vital-") Färbung durchgeführt.

Direkt nach der Substanzapplikation stellte sich der nicht-perfundierte Anteil des Myocards jeweils als hypointenses Areal dar (s. Fig. 3, Teil a). Ab ca. 30 min p. i. stieg allerdings die Signalintensität im nicht-perfundierten Areal etwas an bzw. die Größe des abgegrenzten (signalarmen) Areals nahm ab (langsame Diffusion in die Nekrose). In der späten Phase (24 h p. i.) war ein deutliches Enhancement im nekrotischen Areal des Myocards festzustellen (s. Fig. 3, Teil b). Die Abgrenzung des Myocards festzustellen (s. Fig. 3, Teil b). Die Abgrenzung des nekrotischen Areals im MRI-Experiment korreliert sehr gut mit den Ergebnissen der histologischen "Vital"-Färbung.

## Patentansprüche

1. Verbindungen der allgemeinen Formel I 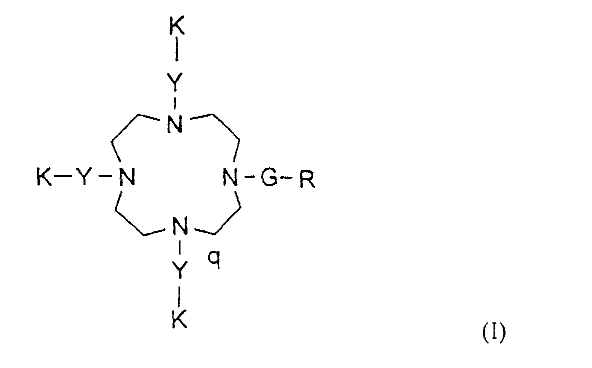 worin
q eine Zahl 1, 2 oder 3 bedeutet,
G eine direkte Bindung, eine SO₂- oder eine CO-Gruppe ist,
K für einen Metallkomplex oder dessen Salze organischer und/oder anorganischer Basen oder Aminosäuren oder Aminosäurearnide steht,
R eine unverzweigte oder verzweigte C₄-C₃₀-Kohlenwasserstoffkette ist, die gegebenenfalls substituiert ist durch 1 - 2 Aminogruppen, 1 - 2 SO₂-Gruppen, 1 - 5 OH-Gruppen, 1 - 5 OR¹-Gruppen mit R¹ in der Bedeutung einer C₁-C₆-Alkylgruppe, 1 NH-K-Gruppe, 1 - 3 Carboxygruppen, 1 - 2 aliphatische oder 1 - 3 aromatische Ringe,
und die gegebenenfalls 1 - 6 Amidgruppen, 1 - 2 Schwefelatome, 1 - 6 Sauerstoffatome, 1 - 2 aliphatische oder 1 - 3 aromatische Ringe enthält, wobei die aromatischen Ringe gegebenenfalls substituiert sind mit 1 - 2 Chloratomen, 1 - 2 Säuregruppen, 1 - 2 OR¹-Gruppen oder 1 - 2 C₁-C₆-Alkylgruppen,
Y eine direkte Bindung oder eine Kette der allgemeinen Formel II oder III ist: 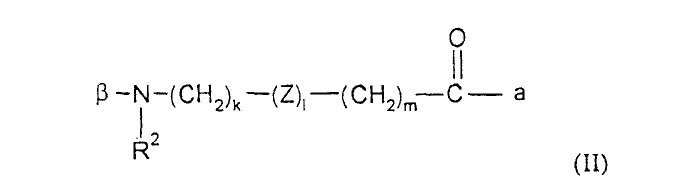 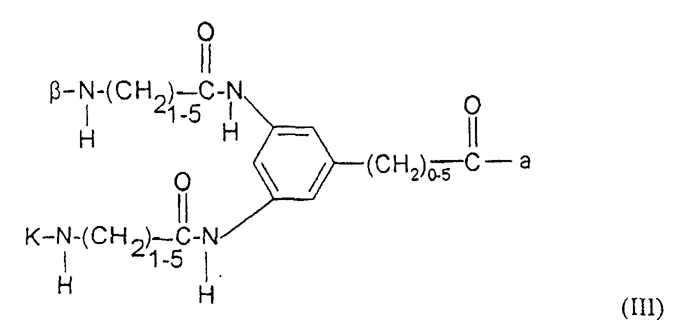 worin
R² ein Wasserstoffatom, eine Phenylgruppe, eine Benzylgruppe oder eine C₁-C₇-Alkylgruppe ist, die gegebenenfalls substituiert ist mit einer Carboxy-, einer Methoxy- oder einer Hydroxygruppe,
Z eine Polyglycolethergruppe mit bis zu 5 Glycoleinheiten oder ein Molekülteil der allgemeinen Formel IV ist
―CH(R³)- (IV),
worin R³ ein C₁-C₁₀-Alkylrest, eine Carbonsäure mit 1 - 7 Kohlenstoffatomen, eine Phenylgruppe, eine Benzylgruppe oder eine -(CH₂)₁₋₅-NH-K-Gruppe ist, α die Bindung an das Stickstoffatom der Gerüstkette, β die Bindung zum Metallkomplex K angibt,
und in der die Variablen k und m für natürliche Zahlen zwischen 0 und 10 und 1 für 0 oder 1 stehen.

2. Verbindungen gemäß Anspruch 1,
**dadurch gekennzeichnet,**
**daß** K einen Komplex der allgemeinen Formel V, VI, VII oder VIII darstellt, 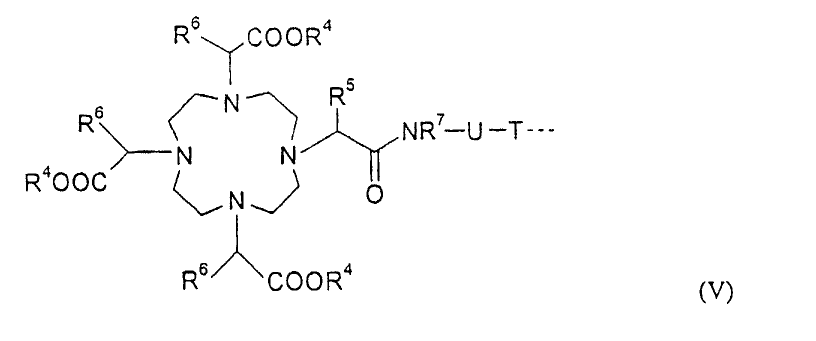 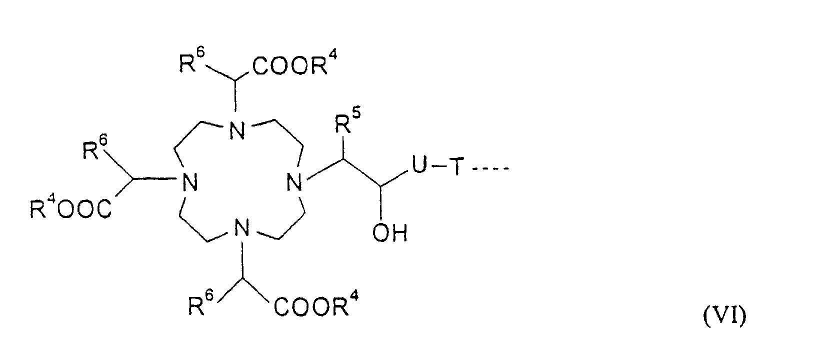 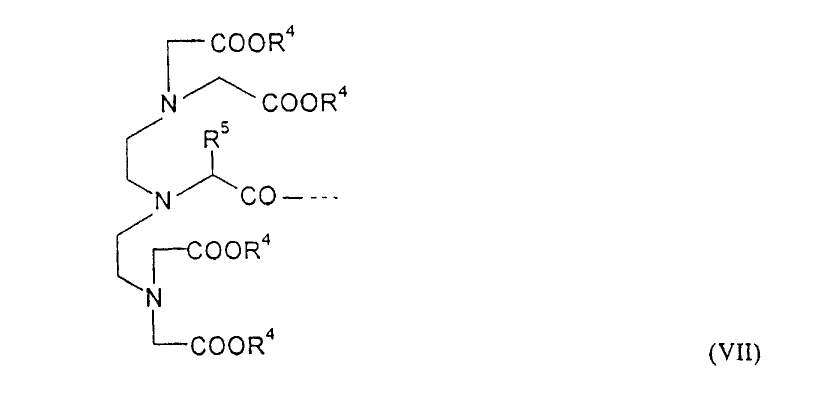 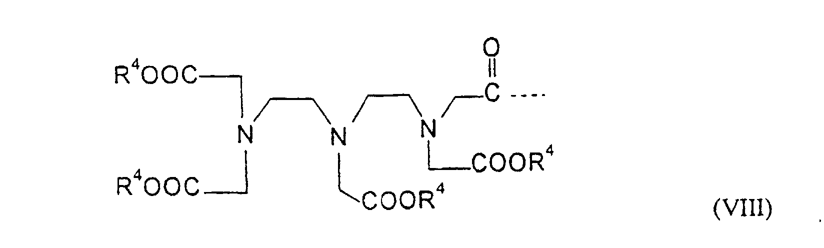 wobei
• R⁴ unabhängig voneinander ein Wasserstoffatom oder ein Metailionenäquivalent der Elemente der Ordnungszahlen 20 - 32, 37 - 39, 42 - 44, 49 oder 57 - 83 ist, mit der Maßgabe, daß mindestens zwei der Substituenten R⁴ für ein Metallionenäquivalent der genannten Elemente stehen,
• R⁵ ein Wasserstoffatom oder eine geradkettige, verzweigte, gesättigte oder ungesättigte C₁-C₃₀-Kohlenwasserstoffkette ist, die gegebenenfalls substituiert ist durch 1 -5 Hydroxy-, 1 - 3 Carboxy- oder 1 Phenylgruppe(n) und/oder gegebenenfalls durch 1 - 10 Sauerstoffatome, 1 Phenylen- oder 1 Phenylenoxygruppe unterbrochen ist,
• R⁶ ein Wasserstoffatom, ein geradkettiger oder verzweigter C₁ -C₇-Alkylrest, ein Phenyl- oder Benzylrest ist,
• R⁷ ein Wasserstoffatom, eine Methyl- oder Ethylgruppe ist, die gegebenenfalls substituiert ist durch eine Hydroxy- oder Carboxygruppe,
• U eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1 Thioharnstoff-, 1 - 2 Carboxyalkylimino-, 1 - 2 Estergruppen, 1 - 10 Sauerstoff-, 1 - 5 5 Schwefel- und/oder 1 - 5 Stickstoffatome enthaltende und/oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppen substituierte, geradkettige, verzweigte, gesättigte oder ungesättigte C₁-C₂₀-Kohlenwasserstoffkette ist, wobei die gegebenenfalls enthaltenen Phenylengruppen durch 1 - 2 Carboxy-, 1 - 2 Sulfon- oder 1 - 2 Hydroxygruppen substituiert sein können,
• T für eine -CO-β, -NHCO-b oder -NHCS-β-Gruppe steht, wobei β die Bindungsstelle an Y angibt.

3. Verbindungen gemäß Anspruch 1,
**dadurch gekennzeichnet,**
**daß** R eine Gruppe der allgemeinen Formel IX ist, 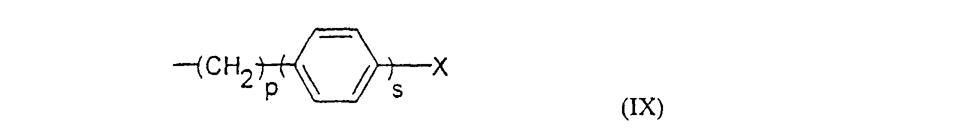 worin p für die Zahlen 0 bis 25 und s für 0, 1 oder 2 stehen und p und s nicht
gleichzeitig Null sind,
und worin X ein Wasserstoffatom, eine Methylgruppe, eine Carboxylgruppe, eine OH-Gruppe, eine OCH₃-Gruppe, eine CONH₂-Gruppe, ein Chloratom, eine C₁-C₁₀-Alkylkette, eine O-CₙH₂₊₁-Gruppe, eine O-(CH₂)ₙ-COOH-Gruppe, eine -O-(CH₂CH₂)ᵣ-CₙH₂ₙ₊₁-Gruppe oder eine NH-CO-CₙH₂ₙ₊₁-Gruppe ist, mit n= 1 - 15 und r = 1 - 5.

4. Verbindungen gemäß Anspruch 1,
**dadurch gekennzeichnet,**
**daß** R eine der folgenden Gruppen ist: 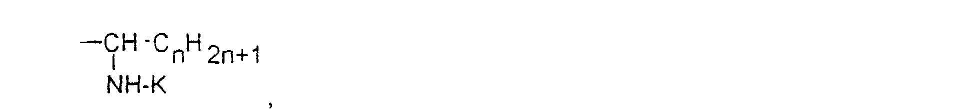 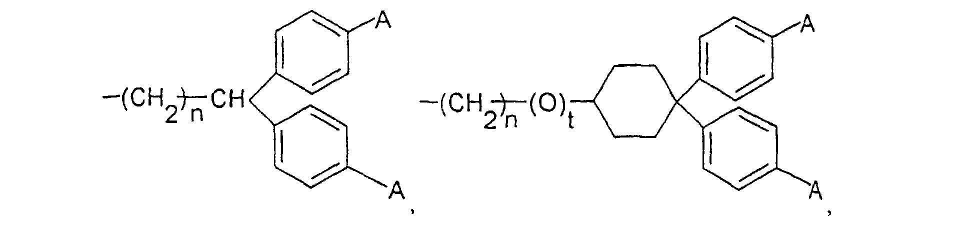 worin n= 1 - 15. t = 0 oder 1 ist und A ein Wasserstoffatom, ein Chloratom oder eine OCH₃-Gruppe bedeutet.

5. Verbindungen gemäß Anspruch 1,
**dadurch gekennzeichnet,**
**daß** G und R zusammen eine der folgenden Strukturen aufweisen:
- CO-C₁₅H₃₁, -CO-C₁₄H₂₉, -CO-C₁₃H₂₇, -CO-C₁₀H₂₀-NH-CO-C₆H₁₃, -CO-C₆H₁₃,
- CO-CH₂-O-CH₂CH₂-O-C₁₀H₂₁, -CO-CH₂-O-C₁₃H₂₇, -C₁₅H₃₁, -C₁₄H₂₉,
- SO₂-C₁₃H₂₇, -SO₂-C₁₄H₂₉, -SO₂-C₁₅H₃₁,

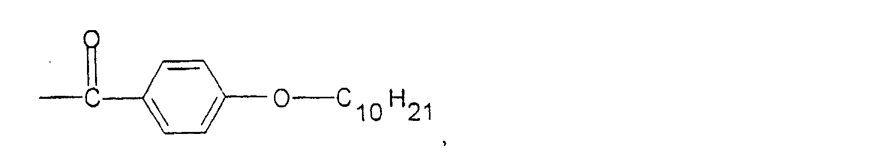

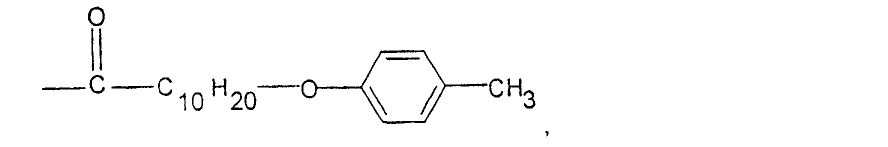

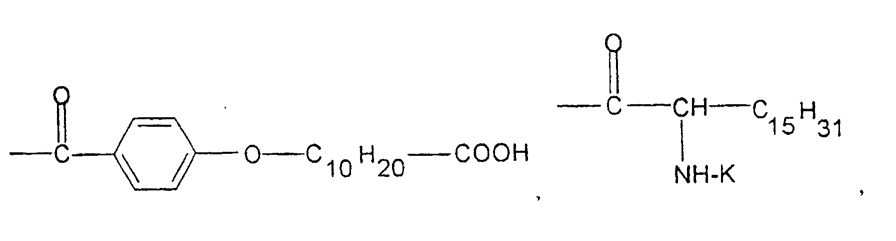

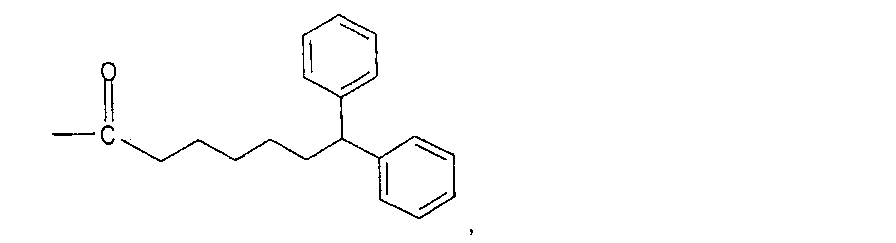

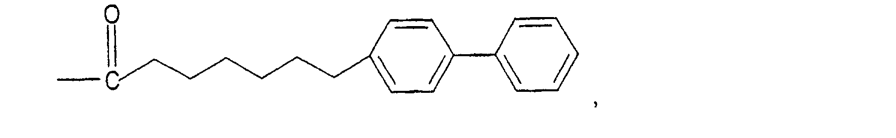

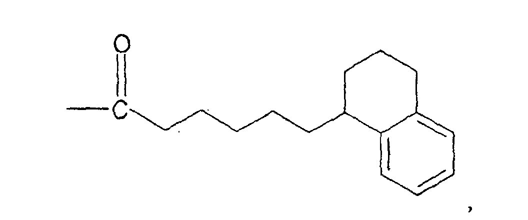

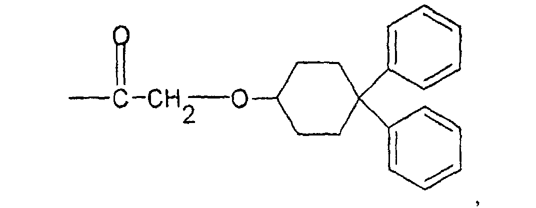

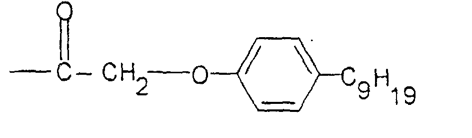

6. Verbindungen gemäß Anspruch 1,
**dadurch gekennzeichnet,**
**daß** Y ein Molekülteil mit einer der folgenden Strukturen ist:

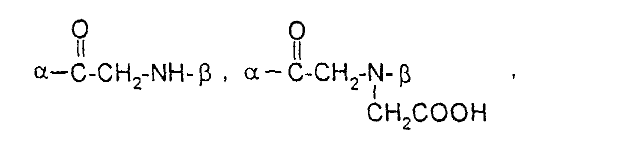

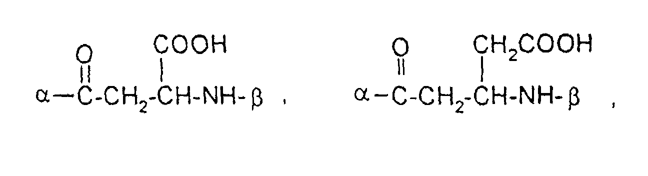

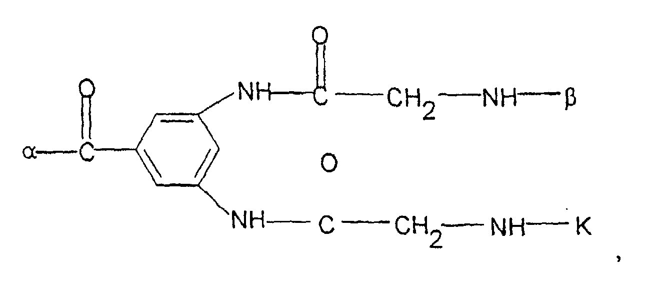

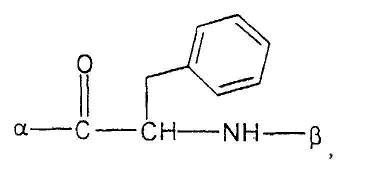

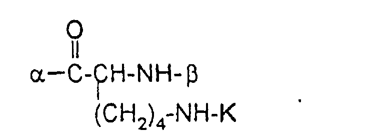

7. Verbindungen gemäß Anspruch 2,
**dadurch gekennzeichnet,**
**daß** die für U stehende C₁-C₂₀-Alkylenkette die Gruppen
-CH₂NHCO-, -NHCOCH₂O-, -NHCOCH₂OC₆H₄-, -N(CH₂CO₂H)-,
-CH₂OCH₂-,
-NHCOCH₂C₆H₄-, -NHCSNHC₆H₄-, -CH₂OC₆H₄-, -CH₂CH₂Oenthält und/oder durch die Gruppen -COOH, -CH₂COOH substituiert ist.

8. Verbindungen gemäß Anspruch 2,
**dadurch gekennzeichnet,**
**daß** U für eine
-CH₂-, -CH₂CH₂-, -CH₂CH₂CH₂-, -C₆H₄-, -C₆H₁₀-, -CH₂C₆H₄-,
-CH₂NHCOCH₂CH(CH₂CO₂H)-C₆H₄-,
-CH₂NHCOCH₂OCH₂-,
-CH₂NHCOCH₂C₆H₄-gruppe steht.

9. Verbindungen gemäß Anspruch 1,
**dadurch gekennzeichnet,**
**daß** K eine der folgenden Strukturen aufweist: 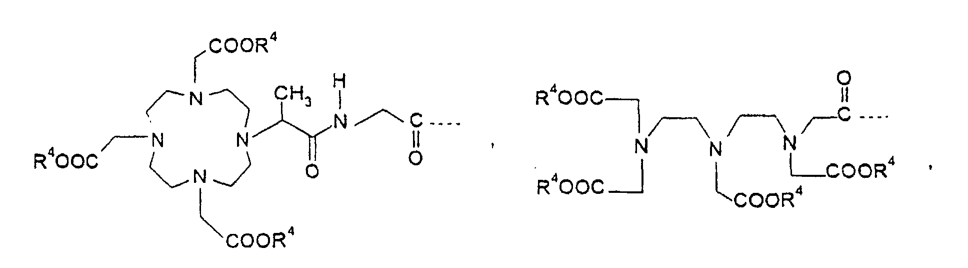 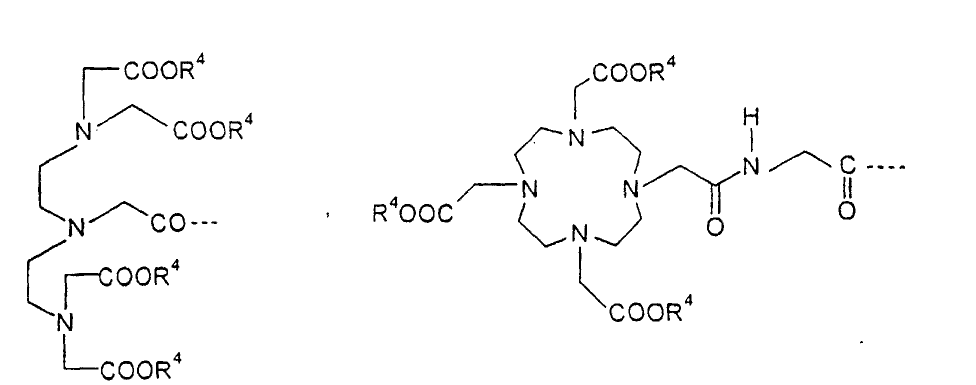 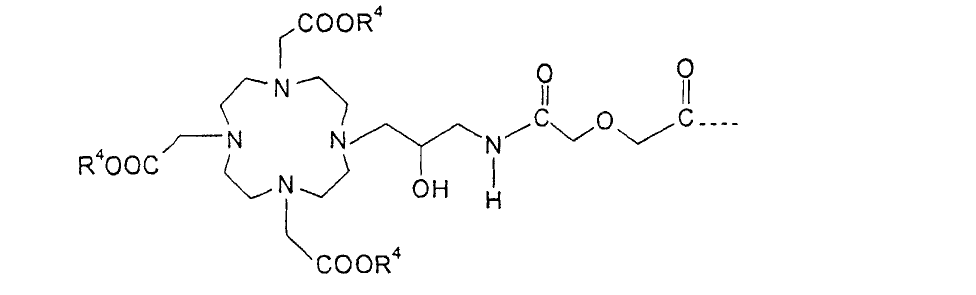 worin R⁴ die in Anspruch 2 definierte Bedeutung hat.

10. Verbindungen gemäß Anspruch 1,
**dadurch gekennzeichnet,**
**daß** q für die Zahl 1 steht.

11. Verfahren zur Herstellung von Verbindungen der allgemeinen Formel I,
**dadurch gekennzeichnet,**
**daß** man Verbindungen der allgemeinen Formel I' 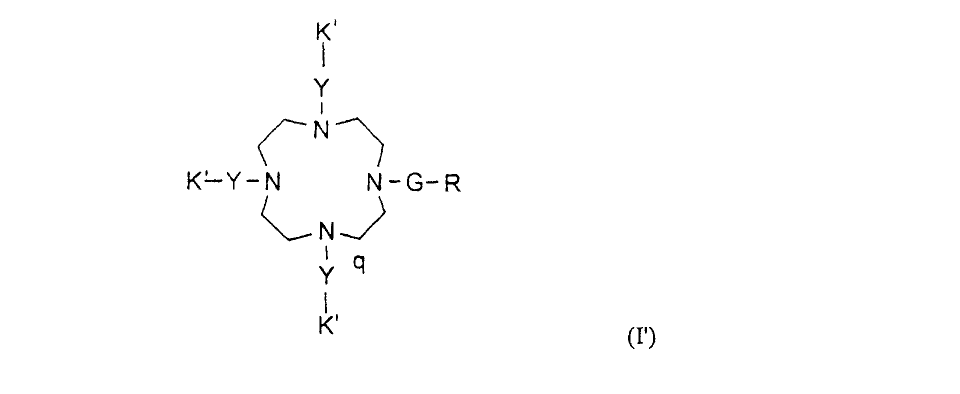 worin K' für K steht mit R⁴ in der Bedeutung eines Wasserstoffatoms oder einer Carboxyschutzgruppe,
nach Abspaltung der gegebenenfalls vorhandenen Schutzgruppen in an sich bekannter Weise mit mindestens einem Metalloxid oder Metallsalz eines Elementes der Ordnungszahlen 20 - 32, 37 - 39, 42 - 44, 49 oder 57 - 83 umsetzt und gegebenenfalls anschließend in den so erhaltenen Komplexverbindungen noch vorhandene acide Wasserstoffatome ganz oder teilweise durch Kationen von anorganischen und/oder organischen Basen, Aminosäuren oder Aminosaureamiden substituiert.

12. Verfahren zur Herstellung von Verbindungen der allgemeinen Formel I,
**dadurch gekennzeichnet,**
**daß** man eine Verbindung der allgemeinen Formel I" 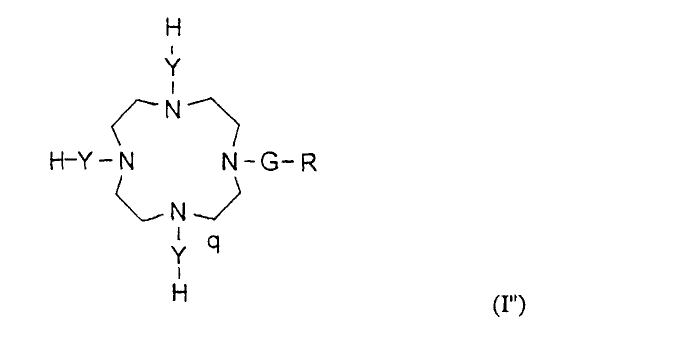 mit einem Komplex V' oder VI' umsetzt, 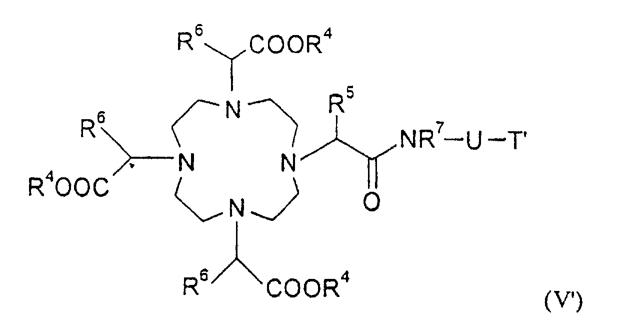 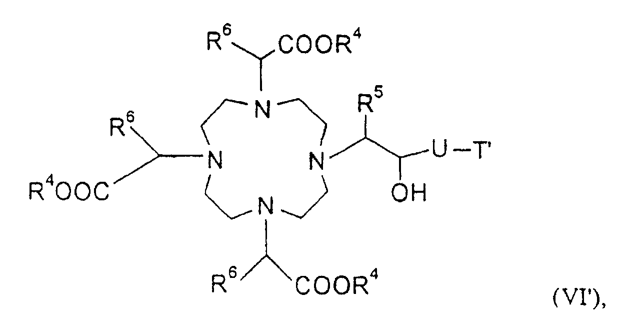 wobei T' für eine -C*O-, -COOH, -N=C=O- oder -N=C=S-Gruppe und -C*O für eine aktivierte Carboxylgruppe steht, und die Reste R⁴ und R⁷ wie in Anspruch 2 definiert sind, mit der Maßgabe, daß mindestens zwei (bei zweiwertigen Metallen) bzw. drei (bei dreiwertigen Metallen) der Substituenten R⁴ für ein Metallionenäquivalent der oben genannten Elemente stehen und daß gewünschtenfalls weitere Carboxylgruppen in Form ihrer Salze mit anorganischen und/oder organischen Basen, Aminosäuren oder Aminosäureamiden vorliegen.

13. Pharmazeutische Mittel enthaltend mindestens eine physiologisch verträgliche Verbindung gemäß Anspruch 1, gegebenenfalls mit den in der Galenik üblichen Zusätzen.

14. Verwendung von mindestens einer physiologisch verträglichen Verbindung gemäß Anspruch I oder eines pharmazeutischen Mittels gemäß Anspruch 13 als Kontrastmittel in der ¹H-NMR-Diagnostik und -Spektroskopie.

15. Verwendung von mindestens einer physiologisch verträglichen Verbindung gemäß Anspruch 1 oder eines pharmazeutischen Mittels gemäß Anspruch 13 als Kontrastmittel in der Röntgendiagnostik.

16. Verwendung von mindestens einer physiologisch verträglichen Verbindung gemäß Anspruch 1 oder eines pharmazeutischen Mittels gemäß Anspruch 13 als pharmazeutisches Mittel für Radio-Diagnostik und -Therapie.

17. Verwendung gemäß Anspruch 14 oder 15 als Diagnostikum zur Lokalisation eines Infarktes oder einer Nekrose.

18. Verfahren zur Herstellung der pharmazeutischen Mittel gemäß Anspruch 13,
**dadurch gekennzeichnet,**
**daß** man die in Wasser oder physiologischer Salzlösung vorliegenden Verbindungen, gegebenenfalls mit den in der Galenik üblichen Zusätzen, in eine für die enterale oder parenterale Applikation geeignete Form bringt.

## Claims

1. Compounds of general formula I 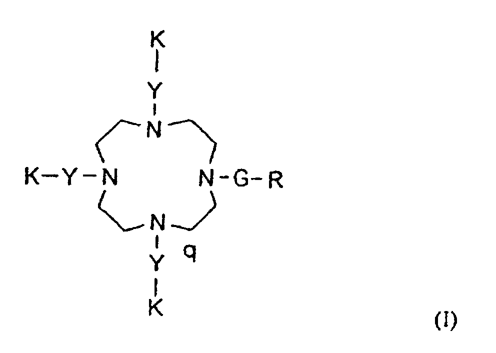 in which q means a number 1, 2 or 3,
G is a direct bond, an SO₂ or a CO group,
K stands for a metal complex or its salts of organic and/or inorganic bases or amino acids or amino acid amides,
R is an unbranched or branched C₄-C₃₀ hydrocarbon chain, which optionally is substituted by 1-2 amino groups, 1-2 SO₂ groups, 1-5 OH groups, 1-5 OR¹ groups with R¹ meaning a C₁-C₆ alkyl group, 1 NH-K group, 1-3 carboxy groups, 1-2 aliphatic or 1-3 aromatic rings, and which optionally contains 1-6 amide groups, 1-2 sulphur atoms, 1-6 oxygen atoms, 1-2 aliphatic or 1-3 aromatic rings, where the aromatic rings are optionally substituted with 1-2 chlorine atoms, 1-2 acid groups, 1-2 OR¹ groups or 1-2 C₁-C₆ alkyl groups,
Y is a direct bond or a chain of general formula II or III: 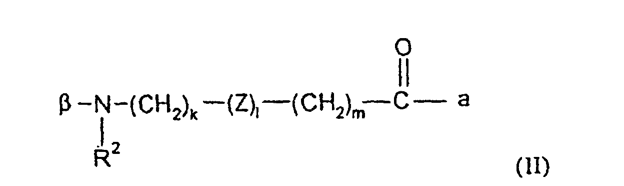 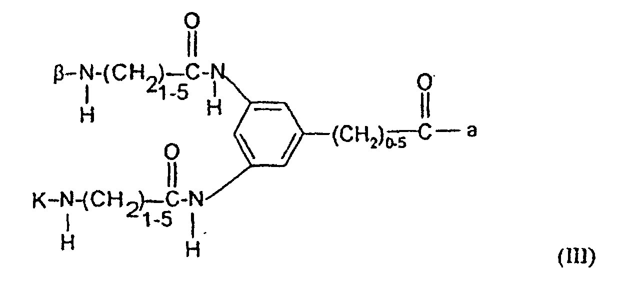 in which R² is a hydrogen atom, a phenyl group, a benzyl group or a C₁-C₇ alkyl group, which optionally is substituted with a carboxy group, a methoxy group or a hydroxy group,
Z is a polyglycol ether group with up to 5 glycol units or a molecule portion of general formula IV
-CH(R³)- (IV),
in which R³ is a C₁-C₁₀ alkyl radical, a carboxylic acid with 1-7 carbon atoms, a phenyl group, a benzyl group or a -(CH₂)₁-₅-NH-K group,
α indicates the bond to the nitrogen atom of the skeleton chain, β indicates the bond to metal complex K,
and in which variables k and m stand for natural numbers between 0 and 10 and 1 stands for 0 or 1.

2. Compounds according to Claim 1, **characterized in that**
K represents a complex of general formula V, VI, VII or VIII, 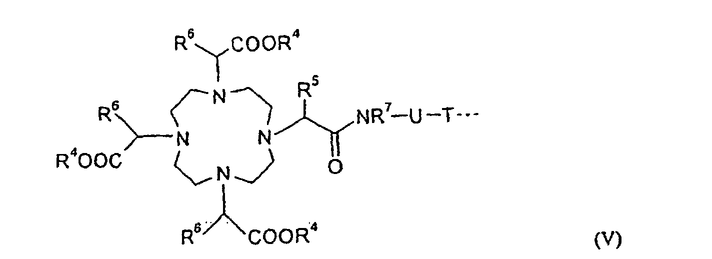 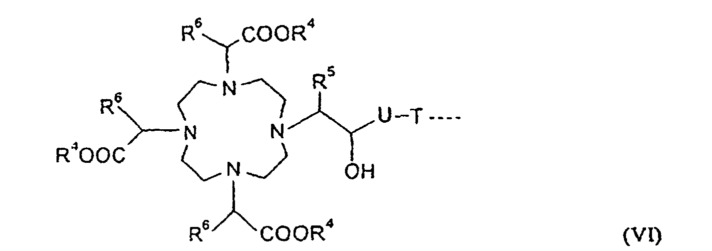 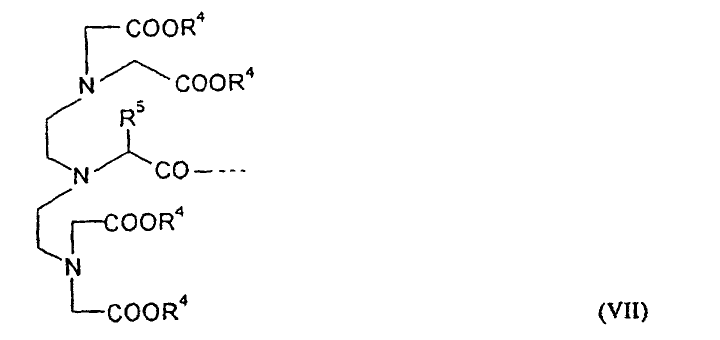 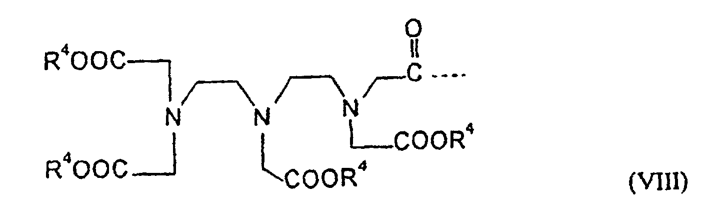 where R⁴, independently of one another, are a hydrogen atom or a metal ion equivalent of the elements of atomic numbers 20-32, 37-39, 42-44, 49 or 57-83, provided that at least two of substituents R⁴ stand for a metal ion equivalent of the above-mentioned elements,
R⁵ is a hydrogen atom or a straight-chain, branched, saturated or unsaturated C₁-C₃₀ hydrocarbon chain, which optionally is substituted by 1-5 hydroxy, 1-3 carboxy or 1 phenyl group(s) and/or optionally is interrupted by 1-10 oxygen atoms, 1 phenylene or 1 phenyleneoxy group,
R⁶ is a hydrogen atom, a straight-chain or branched C₁-C₇ alkyl radical, a phenyl or benzyl radical,
R⁷ is a hydrogen atom, a methyl or ethyl group, which optionally is substituted by a hydroxy or carboxy group,
U is a straight-chain, branched, saturated or unsaturated C₁-C₂₀ hydrocarbon chain that optionally contains 1-5 imino, 1-3 phenylene, 1-3 phenyleneoxy, 1-3 phenyleneimino, 1-5 amide, 1-2 hydrazide, 1-5 carbonyl, 1-5 ethyleneoxy, 1 urea, 1 thiourea, 1-2 carboxyalkylimino, 1-2 ester groups, 1-10 oxygen, 1-5 sulphur and/or 1-5 nitrogen atoms and/or optionally is substituted by 1-5 hydroxy, 1-2 mercapto, 1-5 oxo, 1-5 thioxo, 1-3 carboxy, 1-5 carboxyalkyl, 1-5 ester and/or 1-3 amino groups, where the optionally contained phenylene groups can be substituted by 1-2 carboxy, 1-2 sulphone or 1-2 hydroxy groups,
T stands for a -CO-β, -NHCO-β or -NHCS-β group, where β indicates the binding site to Y.

3. Compounds according to Claim 1, **characterized in that**
R is a group of general formula IX, 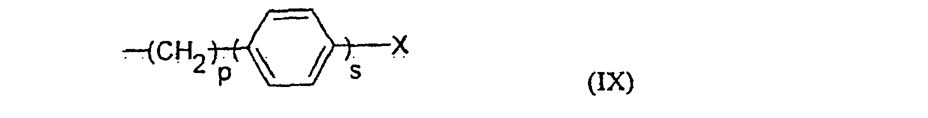 in which p stands for numbers 0 to 25 and s stands for 0, 1 or 2, and p and s are not zero at the same time,
and in which X is a hydrogen atom, a methyl group, a carboxyl group, an OH group, an OCH₃ group, a CONH₂ group, a chlorine atom, a C₁-C₁₀ alkyl chain, an O-CₙH₂ₙ₊₁ group, an O-(CH₂)ₙ-COOH group, an -O-(CH₂CH₂)ᵣ-CₙH₂ₙ₊₁ group or an NH-CO-CₙH₂ₙ₊₁ group, with n=1-15 and r=1-5.

4. Compounds according to Claim 1, **characterized in that**
R is one of the following groups: 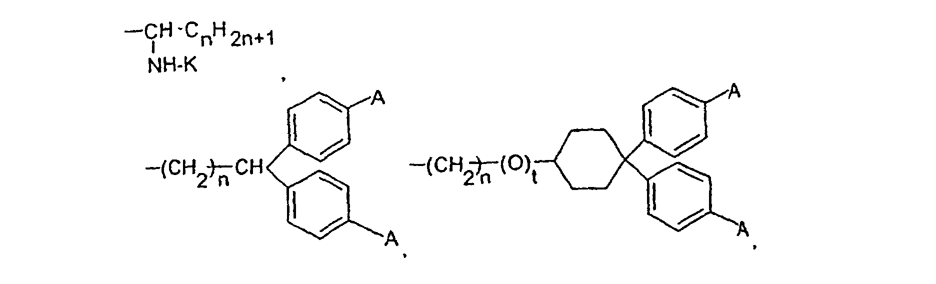 in which n=1-15, t=0 or 1, and A means a hydrogen atom, a chlorine atom or an OCH₃ group.

5. Compounds according to Claim 1, **characterized in that**
G and R together have one of the following structures: -CO-C₁₅H₃₁, -CO-C₁₄H₂₉, -CO-C₁₃H₂₇, -CO-C₁₀H₂₀-NH-CO-C₆H₁₃, -CO-C₆H₁₃, -CO-CH₂-O-CH₂CH₂-O-C₁₀H₂₁, -CO-CH₂-O-C₁₃H₂₇, -C₁₅H₃₁, -C₁₄H₂₉, -SO₂-C₁₃H₂₇, -SO₂-C₁₄H₂₉, -SO₂-C₁₅H₃₁,

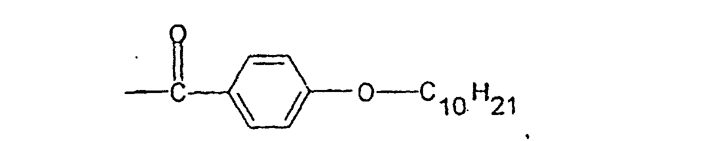

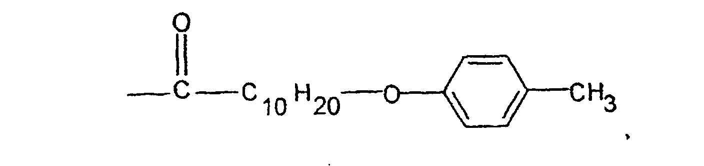

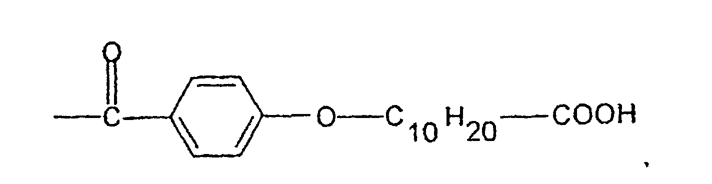

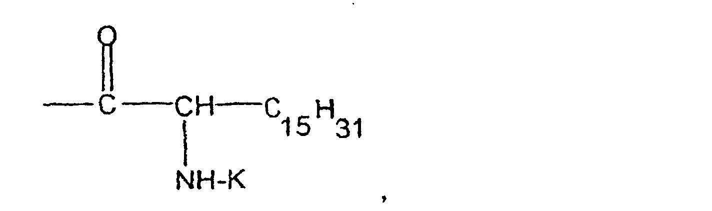

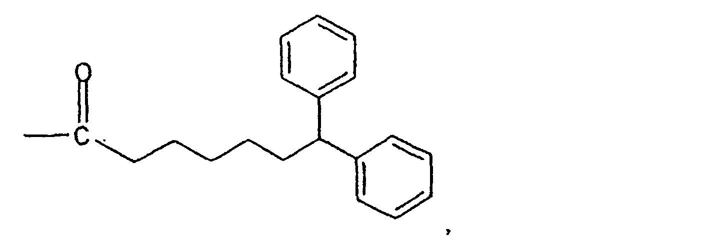

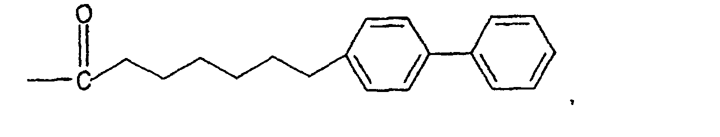

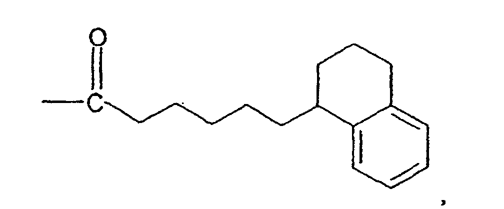

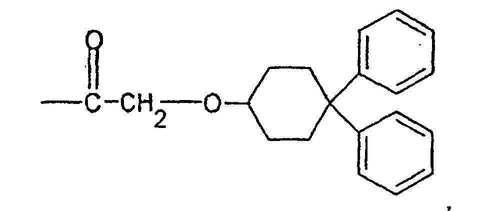

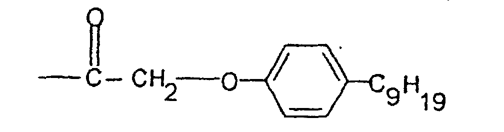

6. Compounds according to Claim 1, **characterized in that**
Y is a molecule portion with one of the following structures:

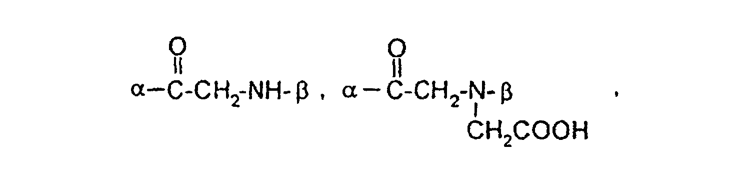

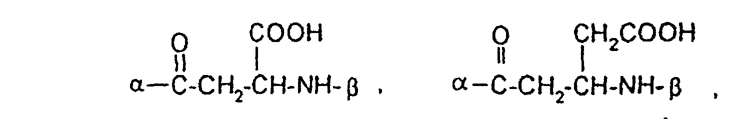

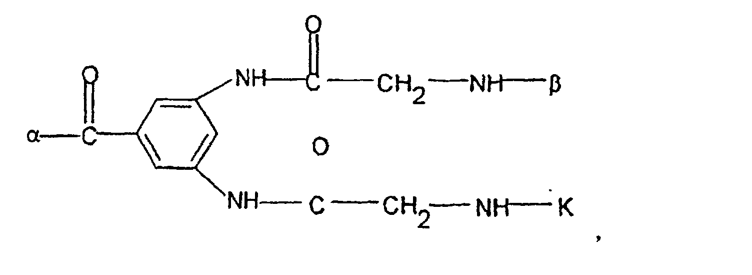

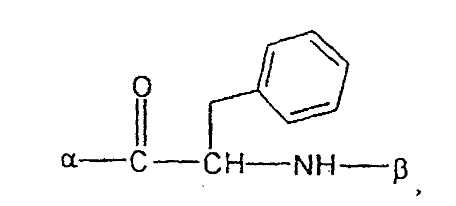

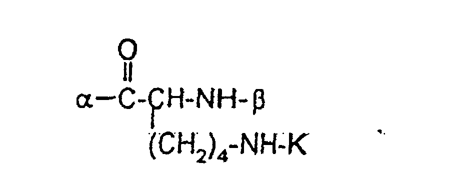

7. Compounds according to Claim 2, **characterized in that**
the C₁-C₂₀ alkylene chain that stands for U contains the groups:
-CH₂NHCO-, -NHCOCH₂O-, -NHCOCH₂OC₆H₄-, -N(CH₂CO₂H)-, -CH₂OCH₂-, -NHCOCH₂C₆H₄-, -NHCSNHC₆H₄-, -CH₂OC₆H₄-, -CH₂CH₂O-
and/or is substituted by groups -COOH and -CH₂COOH.

8. Compounds according to Claim 2,
**characterized in that**
U stands for a
-CH₂-, -CH₂CH₂-, -CH₂CH₂CH₂-, -C₆H₄-, -C₆H₁₀-, -CH₂C₆H₄-, -CH₂NHCOCH₂CH (CH₂CO₂H)-C₆H₄-, -CH₂NHCOCH₂OCH₂-, -CH₂NHCOCH₂C₆H₄- group.

9. Compounds according to Claim 1,
**characterized in that**
K has one of the following structures: 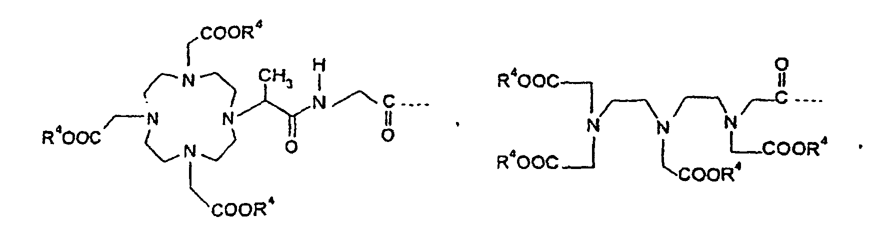 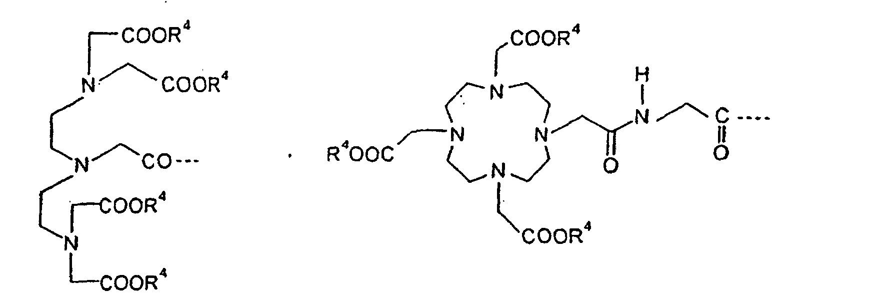 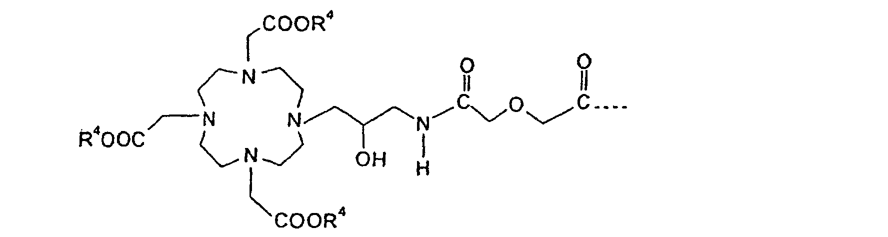 in which R⁴ has the meaning that is defined in Claim 2.

10. Compounds according to Claim 1,
**characterized in that**
q stands for number 1.

11. Process for the production of the compounds of general formula I,
**characterized in that** compounds of general formula I' 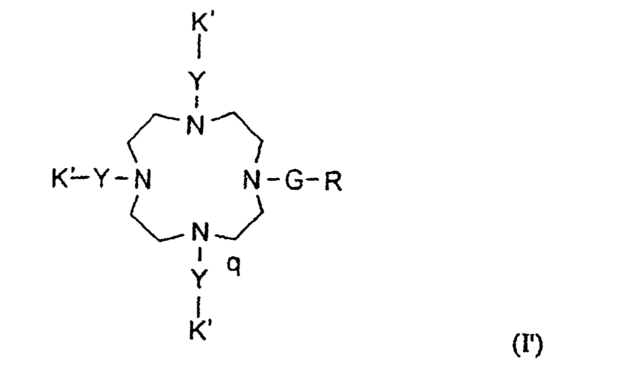 in which K' stands for K, with R⁴ meaning a hydrogen atom or a carboxy protective group,
are reacted, after the optionally present protective groups are cleaved off, in a way known in the art, with at least one metal oxide or metal salt of an element of atomic numbers 20-32, 37-39, 42-44, 49 or 57-83 and optionally then acid hydrogen atoms still present in the complex compounds that are thus obtained are substituted completely or partially by cations of inorganic and/or organic bases, amino acids or amino acid amides.

12. Process for the production of compounds of general formula I,
**characterized in that** a compound of general formula I" 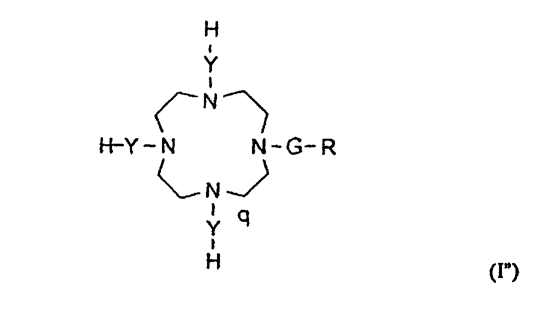 is reacted with a complex V' or VI', 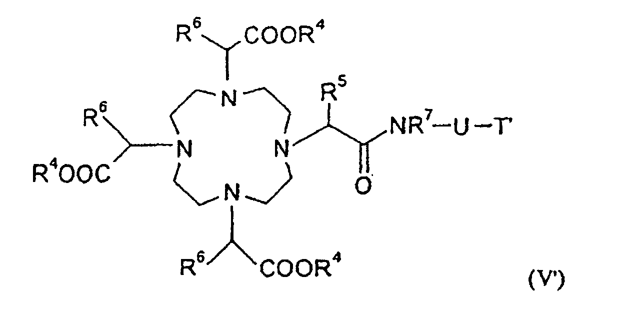 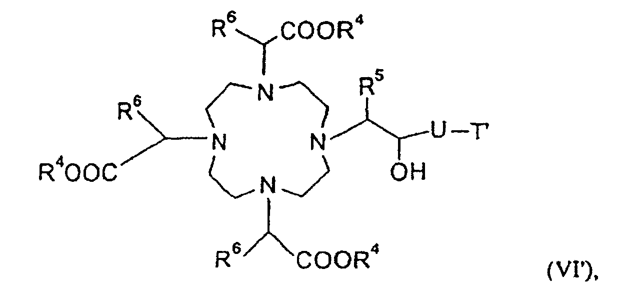 where T' stands for a -C*O, -COOH, -N=C=O or -N=C=S group and -C*O stands for an activated carboxyl group, and radicals R⁴ and R⁷ are defined as in Claim 2, provided that at least two (in the case of divalent metals) or three (in the case of trivalent metals) of substituents R⁴ stand for a metal ion equivalent of the above-mentioned elements and provided that optionally other carboxyl groups are present in the form of their salts with inorganic and/or organic bases, amino acids or amino acid amides.

13. Pharmaceutical agents that contain at least one physiologically compatible compound according to Claim 1, optionally with the additives that are commonly used in galenicals.

14. Use of at least one physiologically compatible compound according to Claim 1 or of a pharmaceutical agent according to Claim 13 as contrast media in ¹H-NMR diagnosis and ¹H-NMR spectroscopy.

15. Use of at least one physiologically compatible compound according to Claim 1 or of a pharmaceutical agent according to Claim 13 as contrast media in diagnostic radiology.

16. Use of at least one physiologically compatible compound according to Claim 1 or of a pharmaceutical agent according to Claim 13 as pharmaceutical agent for radiodiagnosis and radiotherapy.

17. Use according to Claim 14 or 15 as a diagnostic agent for the localization of an infarction or a necrosis.

18. Process for the production of pharmaceutical agents according to Claim 13,
**characterized in that** the compounds that are present in water or physiological salt solution, optionally with the additives that are commonly used in galenicals, are brought into a form that is suitable for enteral or parenteral administration.

## Revendications

1. Composés de formule générale I 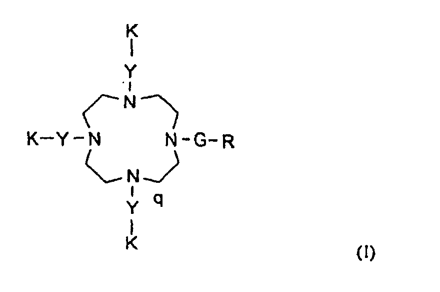 dans laquelle
q représente un nombre 1, 2 ou 3,
G représente une liaison directe, un groupe SO₂ ou CO,
K représente un complexe métallique ou ses sels de bases organiques et/ou inorganiques ou d'amino-acides ou d'amino-amides,
R représente une chaîne hydrocarbonée à C₄-C₃₀ non ramifiée ou ramifiée, qui est éventuellement substituée par 1 - 2 groupes amino, 1 - 2 groupes SO₂, 1 - 5 groupes OH, 1 - 5 groupes OR¹ dans lesquels R¹ représente un groupe alkyle en C₁-C₆, 1 groupe NH-K, 1 - 3 groupes carboxy, 1 - 2 noyaux aliphatiques ou 1 - 3 noyaux aromatiques, et qui renferme éventuellement 1 - 6 groupes amide, 1 - 2 atomes de soufre, 1 - 6 atomes d'oxygène, 1 - 2 noyaux aliphatiques ou 1 - 3 noyaux aromatiques, les noyaux aromatiques étant éventuellement substitués par 1 - 2 atomes de chlore, 1 - 2 groupes acides, 1 - 2 groupes OR¹ ou 1 - 2 groupes alkyle en C₁-C₆,
Y représente une liaison directe ou une chaîne de formules générales II ou III: 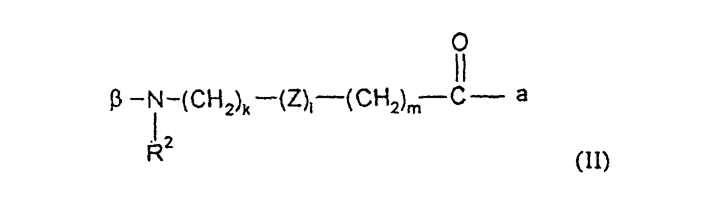 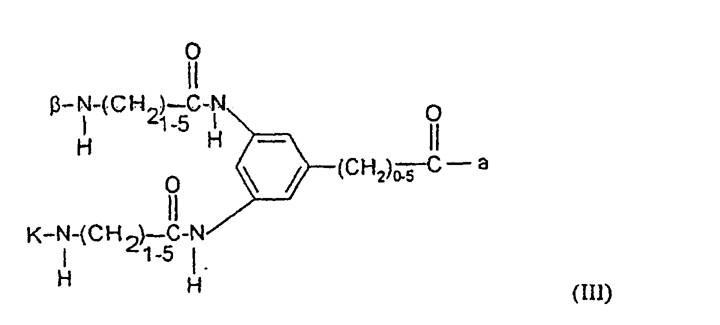 dans lesquelles
R² représente un atome d'hydrogène, un groupe phényle, un groupe benzyle ou un groupe alkyle en C₁-C₇, qui est éventuellement substitué par un groupe carboxy, méthoxy ou hydroxy,
Z représente un groupe polyglycol-éther ayant jusqu'à 5 motifs glycol ou une portion de molécule de formule générale IV
-CH(R³)- (IV),
dans laquelle R³ représente un radical alkyle en C₁-C₁₀, un acide carboxylique ayant de 1 à 7 atomes de carbone, un groupe phényle, un groupe benzyle ou un groupe -(CH₂)₁₋₅-NH-K,
α représente la liaison à l'atome d'azote de la chaîne du squelette, β représente la liaison au complexe métallique K, et dans lesquelles les variables k et m représentent des nombres naturels compris entre 0 et 10 et 1 vaut 0 ou 1.

2. Composés selon la revendication 1,
**caractérisés en ce que** K représente un complexe de formules générales V, VI, VII ou VIII, 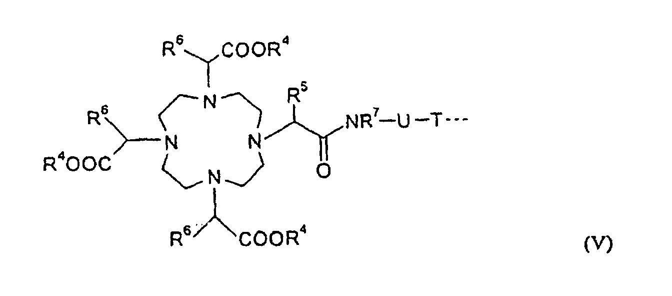 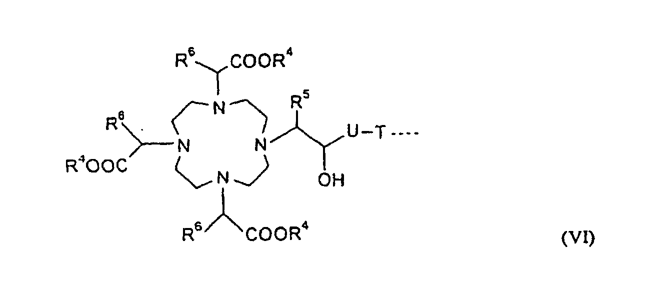 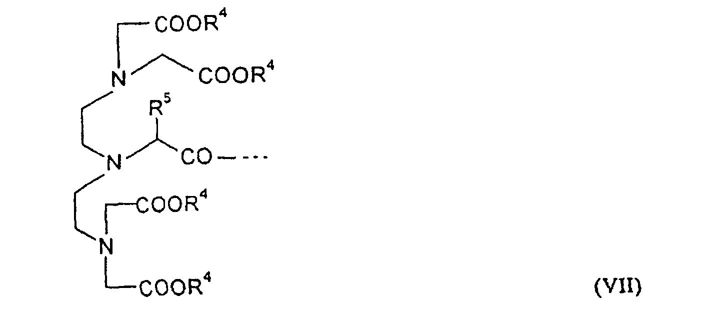 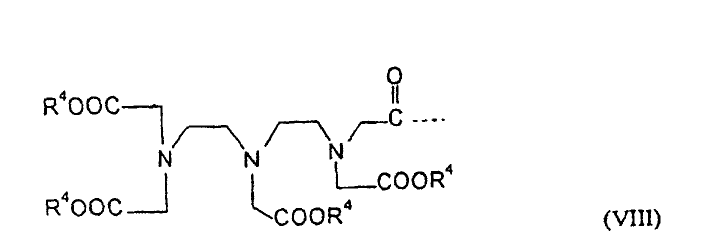 dans lesquelles
· R⁴ représente indépendamment un atome d'hydrogène ou un équivalent d'ion métallique des éléments de numéros atomiques 20 - 32, 37 - 39, 42 - 44, 49 ou 57 - 83, à condition qu'au moins deux des substituants R⁴ représentent un équivalent d'ions métalliques des éléments mentionnés,
· R⁵ représente un atome d'hydrogène ou une chaîne hydrocarbonée en C₁-C₃₀ linéaire, ramifiée, saturée ou insaturée, qui est éventuellement substituée par 1 - 5 groupes hydroxy, 1 - 3 groupes carboxy ou 1 groupe phényle et/ou est éventuellement interrompue par 1 - 10 atomes d'oxygène, 1 groupe phénylène ou 1 groupe phénylène-oxy,
· R⁶ représente un atome d'hydrogène, un radical alkyle en C₁-C₇ linéaire ou ramifié, un radical phényle ou benzyle,
· R⁷ représente un atome d'hydrogène, un groupe méthyle ou éthyle, qui est éventuellement substitué par un groupe hydroxy ou carboxy,
· U représente une chaîne hydrocarbonée en C₁-C₂₀ linéaire, ramifiée, saturée ou insaturée, renfermant éventuellement 1 - 5 groupes imino, 1 - 3 groupes phénylène, 1 - 3 groupes phénylène-oxy, 1 - 3 groupes phénylène-imino, 1 - 5 groupes amide, 1 - 2 groupes hydrazide, 1 - 5 groupes carbonyle, 1 - 5 groupes éthylène-oxy, 1 groupe urée, 1 groupe thiourée, 1 - 2 groupes carboxyalkylimino, 1 - 2 groupes ester, 1 - 10 atomes d'oxygène, 1 - 5 atomes de soufre et/ou 1 - 5 atomes d'azote et/ou éventuellement substituée par 1 - 5 groupes hydroxy, 1 - 2 groupes mercapto, 1 - 5 groupes oxo, 1 - 5 groupes thioxo, 1 - 3 groupes carboxy, 1 - 5 groupes carboxyalkyle, 1 - 5 groupes ester et/ou 1 - 3 groupes amino, les groupes phénylène éventuellement présents pouvant être substitués par 1 - 2 groupes carboxy, 1 - 2 groupes sulfone ou 1 - 2 groupes hydroxy,
· T représente un groupe -CO-β, -NHCO-β ou -NHCS-β, où β indique la position de liaison à Y.

3. Composés selon la revendication 1,
**caractérisés en ce que** R représente un groupe de formule générale IX, 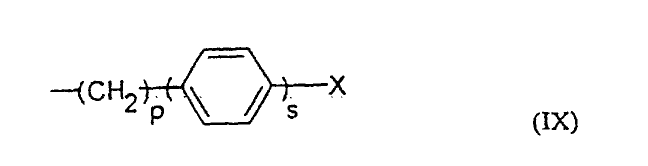 dans laquelle p représente les nombres 0 à 25 et s vaut 0, 1 ou 2 et p et s ne sont pas simultanément nuls,
et dans laquelle X représente un atome d'hydrogène, un groupe méthyle, un groupe carboxy, un groupe OH, un groupe OCH₃, un groupe CONH₂, un atome de chlore, une chaîne alkyle en C₁-C₁₀, un groupe O-CₙH₂ₙ₊₁, un groupe O-(CH₂)ₙ-COOH, un groupe -O-(CH₂CH₂)ᵣ-CₙH₂ₙ₊₁ ou un groupe NH-CO-CₙH₂ₙ₊₁, où n = 1 - 15 et r = 1 - 5.

4. Composés selon la revendication 1,
**caractérisés en ce que** R représente l'un des groupes suivants: 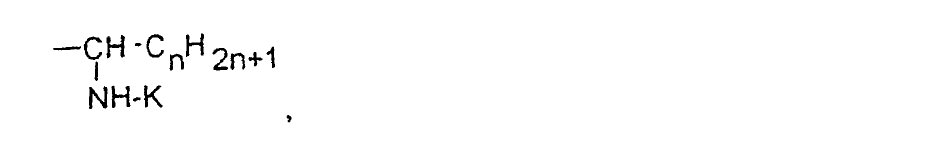 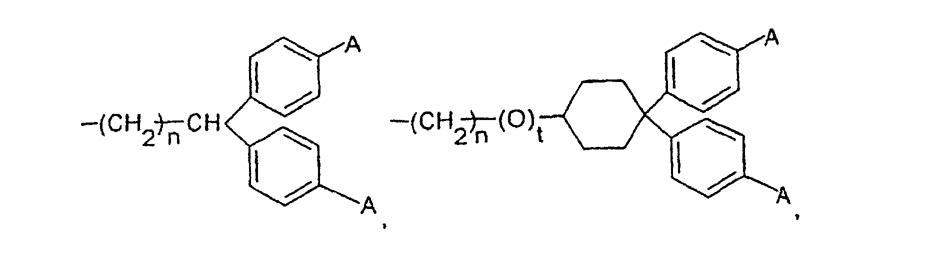 dans lesquels n = 1 - 15, t = 0 ou 1 et A représente un atome d'hydrogène, un atome de chlore ou un groupe OCH₃.

5. Composés selon la revendication 1,
**caractérisés en ce que** G et R présentent conjointement l'une des structures suivantes:
-CO-C₁₅H₃₁,-CO-C₁₄H₂₉, -CO-C₁₃H₂₇, -CO-C₁₀H₂₀-NH-CO-C₆H₁₃, -CO-C₆H₁₃,
-CO-CH₂-O-CH₂CH₂-O-C₁₀H₂₁, -CO-CH₂-O-C₁₃H₂₇, -C₁₅H₃₁, -C₁₄H₂₉,
-SO₂-C₁₃H₂₇, -SO₂-C₁₄H₂₉, -SO₂-C₁₅H₃₁,

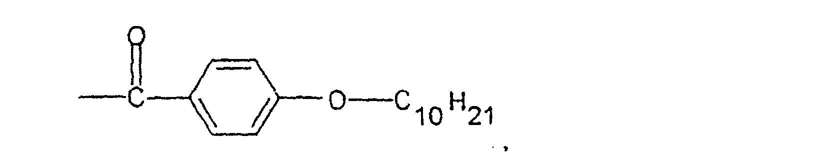

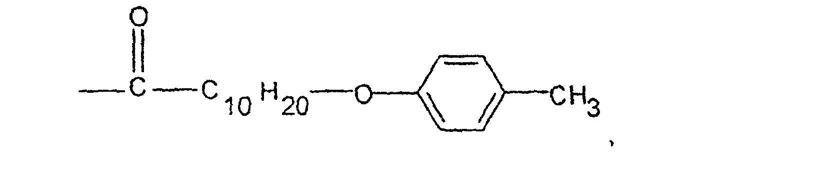

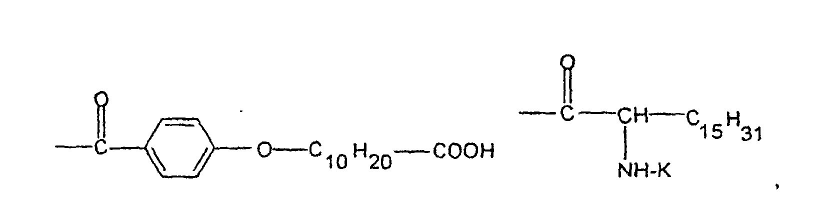

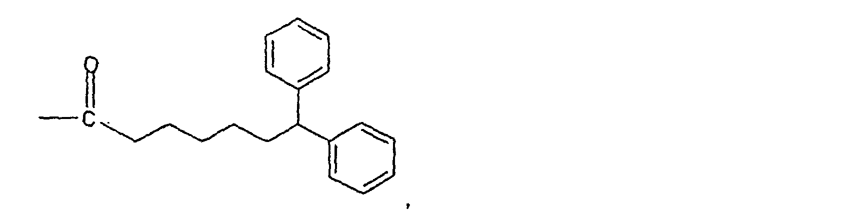

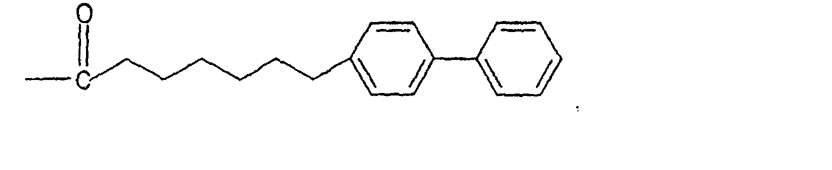

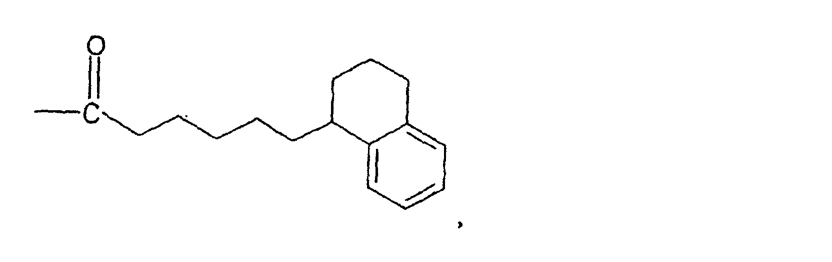

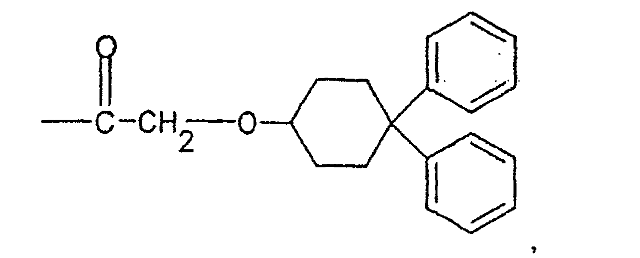

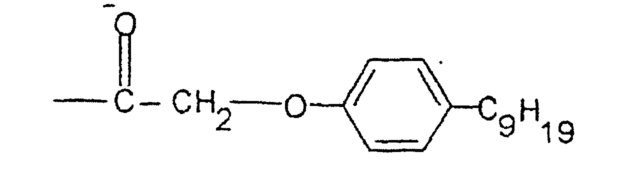

6. Composés selon la revendication 1,
**caractérisés en ce que** Y représente une portion de molécule présentant l'une des structures suivantes:

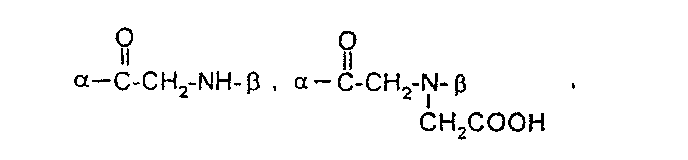

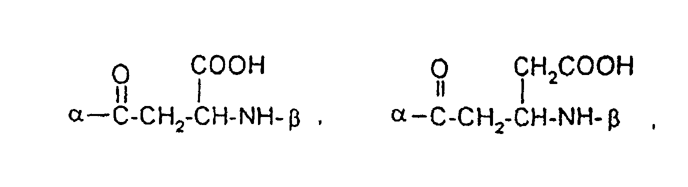

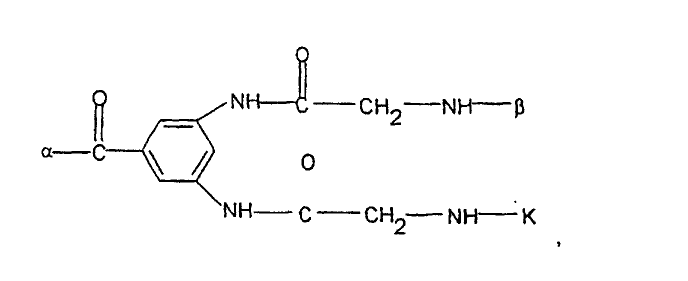

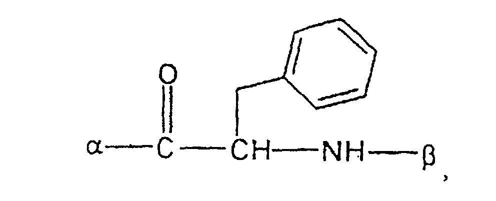

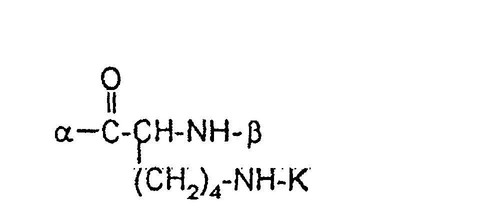

7. Composés selon la revendication 2,
**caractérisés en ce que** la chaîne alkylène en C₁-C₂₀ représentant U renferme les groupes
-CH₂NHCO-, -NHCOCH₂O-, -NHCOCH₂OC₆H₄-, -N(CH₂CO₂H)-,
-CH₂OCH₂-,
-NHCOCH₂C₆H₄-, -NHCSNHC₆H₄-, -CH₂OC₆H₄-, -CH₂CH₂O- et/ou est substituée par les groupes -COOH,
-CH₂COOH.

8. Composés selon la revendication 2,
**caractérisés en ce que** U représente un groupe
-CH₂-, -CH₂CH₂-, -CH₂CH₂CH₂-, -C₆H₄-, -C₆H₁₀-,
-CH₂C₆H₄-,
-CH₂NHCOCH₂CH(CH₂CO₂H)-C₆H₄-,
-CH₂NHCOCH₂OCH₂-,
-CH₂NHCOCH₂C₆H₄-.

9. Composés selon la revendication 1,
**caractérisés en ce que** K présente l'une des structures suivantes: 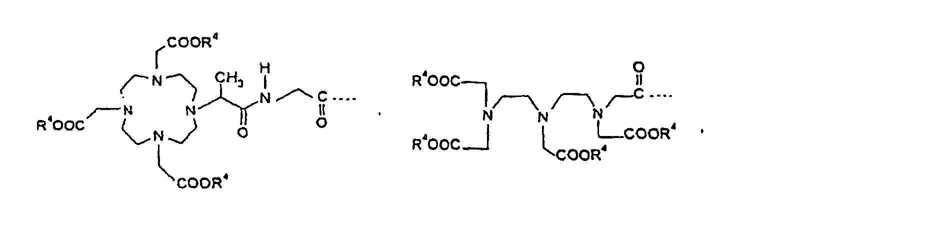 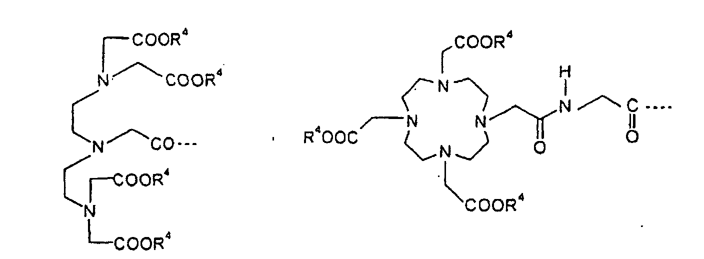 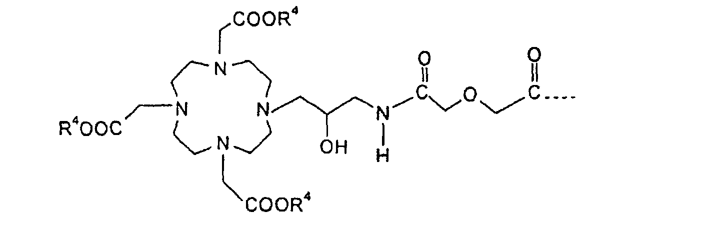 dans lesquelles R⁴ présente la signification indiquée dans la revendication 2.

10. Composés selon la revendication 1,
**caractérisés en ce que** q représente le nombre 1.

11. Procédé de préparation de composés de formule générale I,
**caractérisé en ce que** des composés de formule générale I' 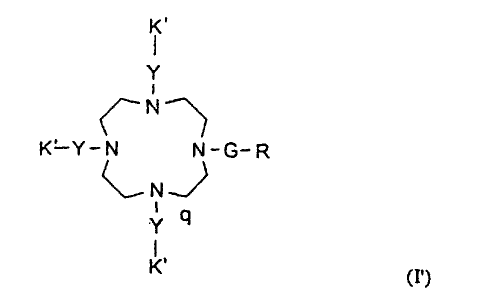 dans laquelle K' représente K où R⁴ représente un atome d'hydrogène ou un groupe protecteur de carboxy, sont mis à réagir, après élimination des groupes protecteurs éventuellement présents d'une façon connue en soi, avec au moins un oxyde métallique ou un sel métallique d'un élément de numéros atomiques 20 - 32, 37 - 39, 42 - 44, 49 ou 57 - 83 et ensuite, les atomes d'hydrogène acides encore présents dans les composés complexes ainsi obtenus sont éventuellement substitués, en totalité ou en partie, par des cations de bases inorganiques et/ou organiques, d'amino-acides ou d'amino-amides.

12. Procédé de préparation de composés de formule générale I,
**caractérisé en ce qu'**un composé de formule générale I" 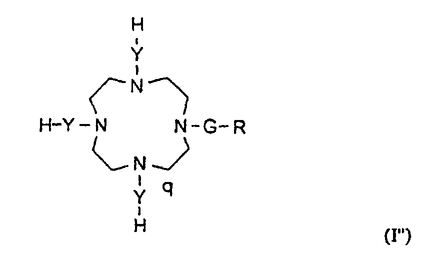 est mis à réagir avec un complexe V' ou VI', 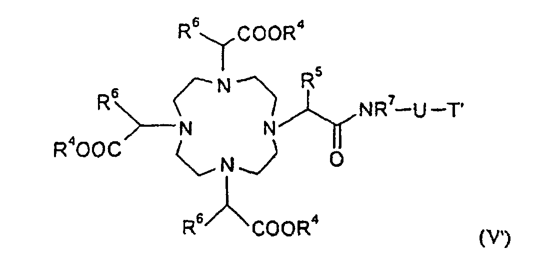 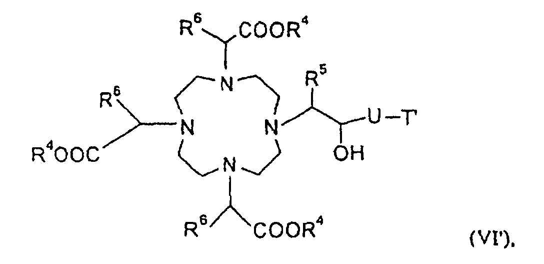 dans lesquels T' représente un groupe -C*O, -COOH, -N=C=O ou -N=C=S et -C*O représente un groupe carboxy activé, et les radicaux R⁴ et R⁷ sont tels que définis dans la revendication 2, à condition qu'au moins deux (dans le cas de métaux bivalents) ou trois (dans le cas de métaux trivalents) des substituants R⁴ représentent un équivalent d'ions métalliques des éléments susmentionnés et que, si on le souhaite, d'autres groupes carboxy soient présents sous la forme de leurs sels avec des bases inorganiques et/ou organiques, des aminoacides ou des amino-amides.

13. Composition pharmaceutique comprenant au moins un composé physiologiquement acceptable selon la revendication 1, éventuellement avec des additifs habituels en galénique.

14. Utilisation d'au moins un composé physiologiquement acceptable selon la revendication 1 ou d'une composition pharmaceutique selon la revendication 13, en tant que milieu de contraste en diagnostic et spectroscopie RMN ¹H.

15. Utilisation d'au moins un composé physiologiquement acceptable selon la revendication 1 ou d'une composition pharmaceutique selon la revendication 13, en tant que milieu de contraste en diagnostic par rayons X.

16. Utilisation d'au moins un composé physiologiquement acceptable selon la revendication 1 ou d'une composition pharmaceutique selon la revendication 13, en tant que composition pharmaceutique destinée au radiodiagnostic et à la radiothérapie.

17. Utilisation selon la revendication 14 ou 15, en tant qu'agent diagnostique destiné à la localisation d'un infarctus ou d'une nécrose.

18. Procédé de préparation des compositions pharmaceutiques selon la revendication 13, **caractérisé en ce que** les composés présents dans de l'eau ou une solution salée physiologique, éventuellement avec les additifs habituels en galénique, sont mis sous une forme appropriée pour l'administration par voie entérale ou parentérale.
